(19) Europäisches Patentamt
European Patent Office
Office européen des brevets

(11) EP 0 663 391 B1

(12) EUROPÄISCHE PATENTSCHRIFT

(45) Veröffentlichungstag und Bekanntmachung des Hinweises auf die Patenterteilung:
**09.04.1997 Patentblatt 1997/15**

(21) Anmeldenummer: **95100180.9**

(22) Anmeldetag: **09.01.1995**

(51) Int Cl.[6]: **C07C 305/06**, C07C 305/10, C07C 335/16, C07C 311/17, C07C 317/44, C07C 317/22, C07D 307/68, C07C 317/36, A61K 31/255

(54) **Schwefelsäureester von Zuckeralkoholen zur Behandlung von arteriosklerotischen Gefässwandveränderungen**

Sulphuric acid esters of sugar alcohols for the treatment of arteriosclerotic changes in the vascular walls

Esters d'acide sulfurique et des alcools de sucre pour le traitement des changements artériosclérotiques des parois vasculaires

(84) Benannte Vertragsstaaten:
**AT BE CH DE DK ES FR GB GR IE IT LI LU MC NL PT SE**

(30) Priorität: **14.01.1994 CH 114/94**
**07.11.1994 CH 3315/94**

(43) Veröffentlichungstag der Anmeldung:
**19.07.1995 Patentblatt 1995/29**

(73) Patentinhaber: **F. HOFFMANN-LA ROCHE AG**
**4002 Basel (CH)**

(72) Erfinder:
- **Chucholowski, Alexander**
  **D-79639 Grenzach-Wyhlen (DE)**
- **Fingerle, Jürgen**
  **D-79618 Rheinfelden (DE)**
- **Iberg, Niggi**
  **CH-4059 Basle (CH)**
- **Märki, Hans Peter**
  **CH-4059 Basle (CH)**
- **Müller, Rita**
  **CH-4051 Basle (CH)**
- **Pech, Michael**
  **D-79258 Hartheim (DE)**
- **Rouge, Marianne**
  **CH-4058 Basel (CH)**
- **Schmid, Gérard**
  **CH-4468 Kienberg (CH)**
- **Tschopp, Thomas**
  **CH-4107 Ettingen (CH)**
- **Wessel, Hans Peter**
  **D-79423 Heitersheim (DE)**

(74) Vertreter: **Bertschinger, Christoph, Dr. et al**
**F.Hoffmann-La Roche AG**
**Patent Department (PLP),124**
**Grenzacherstrasse**
**4070 Basel (CH)**

(56) Entgegenhaltungen:
**EP-A- 0 312 086**
**EP-A- 0 479 093**
**EP-A- 0 504 645**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 663 391 B1

Printed by Jouve, 75001 PARIS (FR)

## Beschreibung

Die vorliegende Erfindung betrifft neue Schwefelsäureester von Zuckeralkoholen und Zuckeralkohol-ähnlichen Verbindungen der allgemeinen Formel

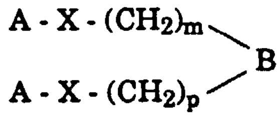

(I)

worin
A ein um die 1-Hydroxygruppe verminderter Rest eines Zuckeralkohols oder eines Derivats davon, oder der tris-(Hydroxymethyl)methylrest ist, wobei mindestens eine Hydroxygruppe des Restes A mit Schwefelsäure verestert ist;
X $-NR^1CO-$; -NHCONH-; -NHCSNH-; $-NHSO_2-$, $-NR^1-$ oder -O-;
m und p unabhängig voneinander 0 oder 1;
$R^1$ Wasserstoff, nieder-Alkyl oder Hydroxy-nieder-alkyl; und
B ein System von konjugierten Mehrfachbindungen ist;
und Salze davon.

Die Erfindung betrifft weiterhin pharmazeutische Präparate, enthaltend eine Verbindung der allgemeinen Formel I oder ein Salz davon; die Verwendung der Verbindungen der allgemeinen Formel I und deren Salze als Heilmittel, insbesondere zur Behandlung und/oder Prophylaxe von arteriosklerotischen Gefässwandveränderungen, z.B. zur Verhinderung von Restenose nach koronarer oder peripherer Angioplastie oder nach Bypass-Operationen; zur Herstellung von Heilmitteln für die genannten Indikationen; sowie ein Verfahren zur Herstellung der Verbindungen der allgemeinen Formel I und deren Salzen.

Vorzugsweise ist der um die 1-Hydroxygruppe verminderte Rest A eines Zuckeralkohols ein Rest $-(CHR^2)_nCH_2R^2$ und n eine ganze Zahl von 1-5; $R^2$ ist H, $-OSO_3H$ oder OZ; Z eine Schutzgruppe; und mindestens ein Rest ist $R^2$ $-OSO_3H$.

Beispiele von Zuckeralkoholen, von denen sich der Rest A ableitet, sind Hexitole, wie Glucitol, Galaktitol, Mannitol und Gulitol, und Pentitole, wie Arabinitol, Ribitol und Xylitol. Beispiele von Derivaten solcher Zucker sind einfach oder mehrfach desoxygenierte Zuckeralkohole, wie L-Rhamnitol. Diese Zuckeralkohole können in der D- oder L-Form oder als Razemate vorliegen, wobei die natürlich vorkommende Form bzw. die Form, die dem zugrundeliegenden, natürlich vorkommenden Zucker entspricht, bevorzugt ist.

Vorzugsweise sind die konjugierten Mehrfachbindungen konjugierte Doppelbindungen.

Beispiele von Systemen konjugierter Doppelbindungen sind Polyenkohlenwasserstoffreste mit 3-8 konjugierten Doppelbindungen, z.B. Octa-2,4,6-trien-2,7-diyl und 6,11-Dimethyl-hexadeca-2,4,6,8,10,12,14-heptaen-2,15-diyl; und aromatische Ringsysteme, insbesondere Phenylen, durch nieder-Alkyl substituiertes Phenylen, Naphthylen oder Fluorenylen, oder solche der Formeln (a) bis (s)

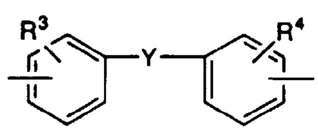

(a)

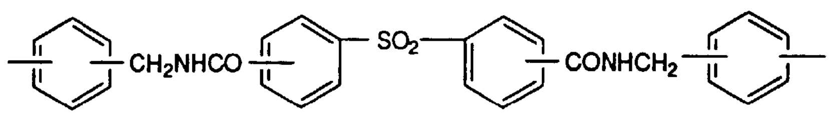

(b)

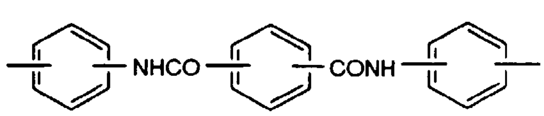

(c)

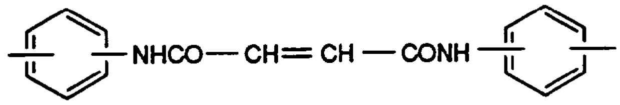

(d)

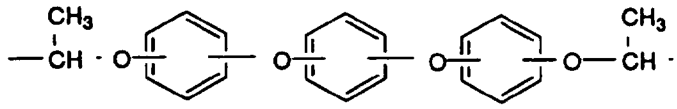

(e)

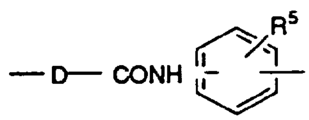

(f)

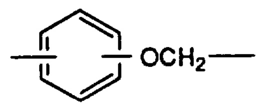

(g)

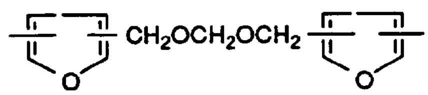

(h)

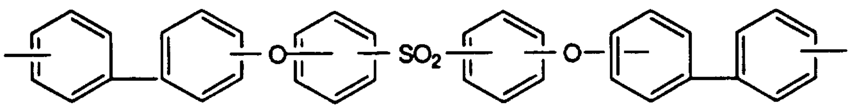

(i)

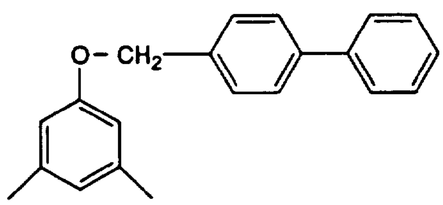

(j)

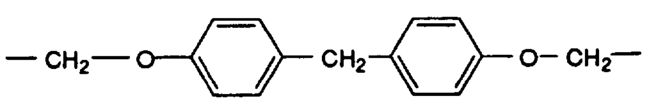

(k)

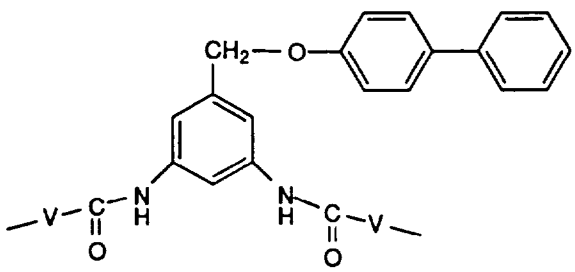

(l)

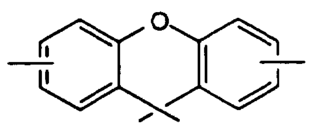

(m)

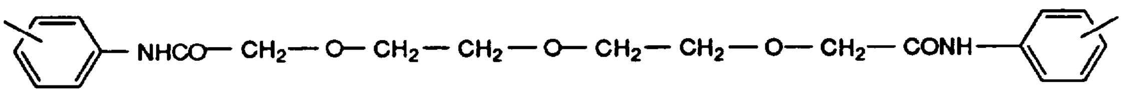

(n)

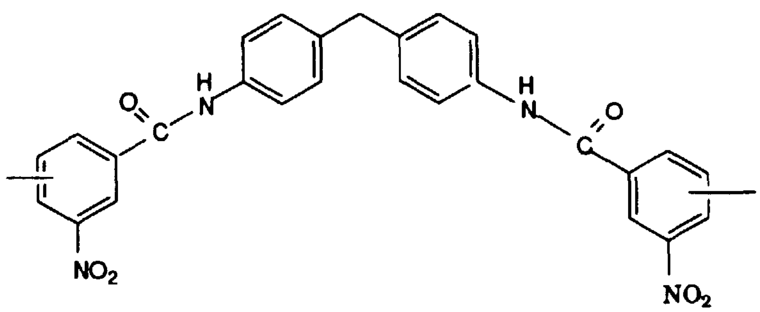

(o)

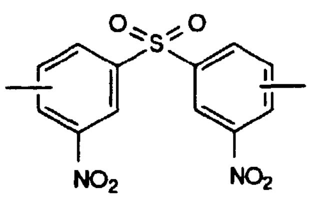

(p)

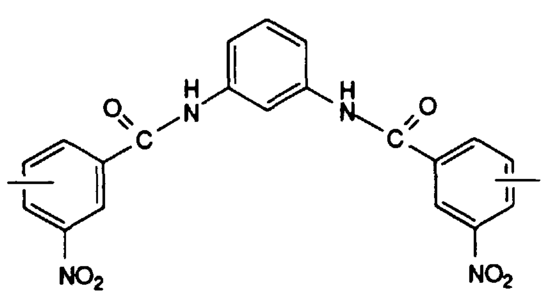

(q)

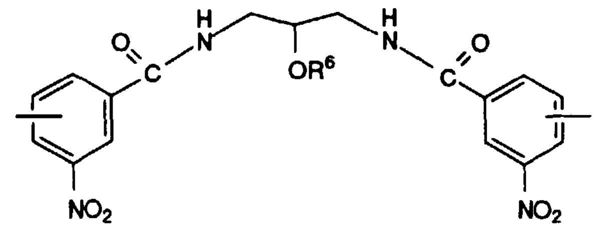

(r)

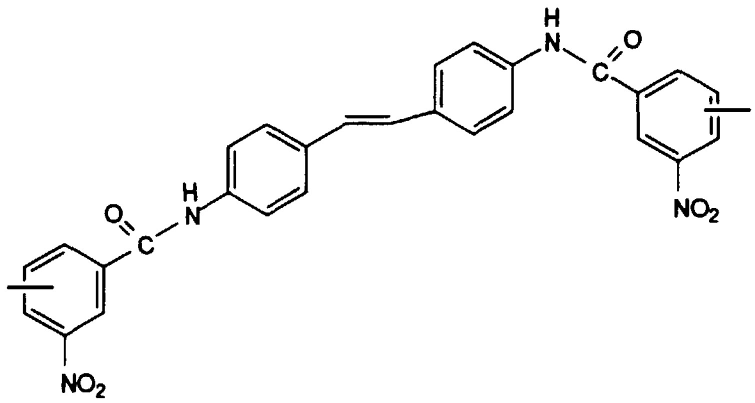

(s)

worin Y eine Kohlenstoff-Kohlenstoff-Bindung; -O-, -CO, $-CH_2-$, $-CH_2CH_2-$, -CH=CH-, -C≡C-, -CH(O)CH-,$-SO_2-$, $-C(CF_3)_2-$, $-CONR^1-$, -NHCONH-, -NHCOCONH-, Phenylen oder Phenylendioxy ist; $R^1$ Wasserstoff, nieder-Alkyl oder Hydroxy-nieder-Alkyl; $R^3$ und $R^4$ Wasserstoff, nieder-Alkyl, nieder-Alkoxy, Halogen oder $-SO_3H$; $R^5$ nieder-Alkoxy-nieder-alkoxy oder Phenoxy, oder durch Halogen substituiertes Phenoxy; $R^6$ Wasserstoff oder $SO_3H$ und D $-CH_2CH_2-$ oder -CH=CH- sind und worin V -CH=CH-, $-CH_2CH_2-$ oder eine Einfachbindung bedeutet.

Der Ausdruck "nieder" bezeichnet Gruppen mit 1-6 C-Atomen, wie Methyl, Aethyl, Propyl, Butyl bzw. Methoxy, Ethoxy, Propoxy und Butoxy. Halogen umfasst Fluor, Chlor, Brom und Jod, wovon Chlor bevorzugt ist. Phenylenreste sind vorzugsweise 1,3- und 1,4-Phenylenreste. Naphthylenreste sind vorzugsweise 1,4-, 1,5- und 2,6-Naphthylenreste. Ein bevorzugter Fluorenylenrest ist 2,7-Fluorenylen. In den Formeln (a) bis (i) stehen die von den Ringen ausgehenden Bindungen vorzugsweise in p-Stellung zueinander.

Beispiele von Salzen von Verbindungen der allgemeinen Formel I sind Alkalimetallsalze, wie Na- oder H-Salze, Ammoniumsalze und Salze von tertiären Aminen, wie Triethylamin, oder Pyridinium- oder Imidazoliumsalze, oder quaternäre Ammoniumsalze, wie Dodecyl-trimethylammonium-, Ethylpyridinium- und Benzethoniumsalze; sowie Erdalkalimetallsalze, wie Ca- oder Mg-Salze.

Beispiele für repräsentative Vertreter, worin das aromatische Ringsystem ein Rest der Formel (a) ist, sind:

(E)-Stilben-4,4'-dicarbonsäure-bis-(2,3,4,5,6-penta-O-sulfo-D-glucit-1-ylamid) Decanatriumsalz,

(Z)-Stilben-4,4'-dicarbonsäure-bis-(2,3,4,5,6-penta-O-sulfo-D-glucit-1-ylamid) Decanatriumsalz,

4,4'-Sulfonyl-dibenzoesäure-bis-(2,3,4,5,6-penta-O-sulfo-D-glucit-1-ylamid) Decanatriumsalz,

4,4'-Ethylen-dibenzoesäure-bis-(2,3,4,5,6-penta-O-sulfo-D-glucit-1-ylamid) Decanatriumsalz,

4,4'-Carbonyl-dibenzoesäure-bis-(2,3,4,5,6-penta-O-sulfo-D-glucit-1-ylamid) Decanatriumsalz,

4,4'-Methylen-dibenzoesäure-bis-(2,3,4,5,6-penta-O-sulfo-D-glucit-1-ylamid) Decanatriumsalz,

4,4'-Oxy-dibenzoesäure-bis-(2,3,4,5,6-penta-O-sulfo-D-glucit-1-ylamid) Decanatriumsalz,

4,4'-(Hexafluoro-propan-2,2-diyl)-dibenzoesäure-bis-(2,3,4,5,6-penta-O-sulfo-D-glucit-1-ylamid) Decanatriumsalz,

Biphenyl-4,4'-dicarbonsäure-bis-(2,3,4,5,6-penta-O-sulfo-D-glucit-1-ylamid) Decanatriumsalz,

3,3'-Bis-(2,3,4,5,6-penta-O-sulfo-D-glucit-1-yl)-1,1'-(3,3'-dichloro-4,4'-methylen-diphenyl)-diharnstoff Decanatriumsalz,

3,3'-Bis-(2,3,4,5,6-penta-O-sulfo-D-glucit-1-yl)-1,1'-(3,3'-dimethyl-biphenyl-4,4'-diyl)-diharnstoff Decanatriumsalz,

3,3'-Bis-(2,3,4,5,6-penta-O-sulfo-D-glucit-1-yl)-1,1'-(3,3'dimethoxy-biphenyl-4,4'-diyl)-diharnstoff Decanatriumsalz,

3,3'-Bis-(2,3,4,5,6-penta-O-sulfo-D-glucit-1-yl)-1,1'-(3,3'-dichlor-biphenyl-4,4'-diyl)-diharnstoff Decanatriumsalz,

1,1'-(4,4'-Methylen-diphenyl)-3,3'-bis-(2,3,4,5,6-penta-O-sulfo-D-glucit-1-yl)-diharnstoff Decanatriumsalz,

(E)-4,4'-Bis-[3-(2,3,4,5,6-penta-O-sulfo-D-glucit-1-yl)-thioureido]-stilben-2,2'-disulfonsäure Dodecanatriumsalz,
Biphenyl-4,4'-disulfonsäure-bis-(2,3,4,5,6-penta-O-sulfo-D-glucit-1-ylamid) Decanatriumsalz,
4,4'-Oxy-dibenzolsulfonsäure-bis-(2,3,4,5,6-penta-O-sulfo-D-glucit-1-ylamid) Decanatriumsalz,
1,3-Bis-[4-(2,3,4,5-tetra-O-sulfo-D-arabinit-1-yloxy)-phenyl]-harnstoff Octanatriumsalz,
N'-Methyl-N-(2,3,4,5,6-penta-O-sulfo-D-glucit-1-yl)-N'-[4-(2,3,4,5,6-penta-O-sulfo-D-glucit-1-ylcarbamoyl)-phenyl]-terephthalamid Decanatriumsalz,
N-(2,3,4,5,6-Penta-O-sulfo-D-glucit-1-yl)-N'-[4-(2,3,4,5,6-penta-O-sulfo-D-glucit-1-ylcarbamoyl)-phenyl]-terephthalamid Decanatriumsalz,
N,N'-Bis-[4-[3-(2,3,4,5,6-penta-O-sulfo-D-glucit-1-yl)-ureido]-phenyl]-oxalamid Decanatriumsalz,
Z-Stilben-4,4'-dicarbonsäure-bis-(2-hydroxysulfonyloxy-1,1-bis-hydroxysulfonyloxymethyl-ethylamid) Hexanatriumsalz,
4,4'-Ethinylendibenzoesäure-bis-(2,3,4,5,6-penta-O-sulfo-D-glucit-1-ylamid) Decanatriumsalz,
2,2',3,3',4,4',5,5'-Octa-O-sulfo-1,1'-O-(4,4'-ethyndiyl-diphenyl)-di-D-arabinitol Octanatriumsalz,
2,2',3,3',4,4',5,5'-Octa-O-sulfo-1,1'-biphenyl-4,4'-diyl-di-D-arabinitol Octanatriumsalz,
(Z)-2,2',3,3',4,4',5,5'-Octa-O-sulfo-1,1'-O-(4,4'-ethylen-di-phenylen)-di-D-arabinitol Octanatriumsalz,
2,2',3,3',4,4',5,5'-Octa-O-sulfo-1,1'-O-(4,4'ethan-1,2-diyl-diphenylen)-di-D-arabinitol Octanatriumsalz,
1,3-Bis-[4-(2,3,4,5,6-penta-O-sulfo-D-galactit-6-yloxy)-phenyl]-harnstoff Decanatriumsalz,
cis-4,4'-Oxiran-2,3-diyl-dibenzoesäure-bis-(2,3,4,5,6-penta-O-sulfo-D-glucit-1-ylamid) Decanatrium-salz,
(E)-Stilben-3,4'-dicarbonsäure-bis-(2,3,4,5,6-penta-O-sulfo-D-glucit-1-ylamid) Decanatriumsalz,
(E)-Stilben-3,3'-dicarbonsäure-bis-(2,3,4,5,6-penta-O-sulfo-D-glucit-1-ylamid) Decanatriumsalz,
(E)-Stilben-2,4'-dicarbonsäure-bis-(2,3,4,5,6-penta-O-sulfo-D-glucit-1-ylamid) Decanatriumsalz,
(E)-Stilben-2,3'-dicarbonsäure-bis-(2,3,4,5,6-penta-O-sulfo-D-glucit-1-ylamid) Decanatriumsalz,
(Z)-Stilben-2,2'-dicarbonsäure-bis-(2,3,4,5,6-penta-O-sulfo-D-glucit-1-ylamid) Decanatriumsalz,
(Z)-Stilben-3,4'-dicarbonsäure-bis-(2,3,4,5,6-penta-O-sulfo-D-glucit-1-ylamid) Decanatriumsalz,
(Z)-Stilben-3,3'-dicarbonsäure-bis-(2,3,4,5,6-penta-O-sulfo-D-glucit-1-ylamid) Decanatriumsalz,
(Z)-Stilben-2,3'-dicarbonsäure-bis-(2,3,4,5,6-penta-O-sulfo-D-glucit-1-ylamid) Decanatriumsalz,
3,4'-Ethylendibenzoesäure-bis-(2,3,4,5,6-penta-O-sulfo-D-glucit-1-ylamid) Decanatriumsalz,
2,4'-Ethylendibenzoesäure-bis-(2,3,4,5,6-penta-O-sulfo-D-glucit-1-ylamid) Decanatriumsalz,
2,3'-Ethylendibenzoesäure-bis-(2,3,4,5,6-penta-O-sulfo-D-glucit-1-ylamid) Decanatriumsalz,
2,2'-Ethylendibenzoesäure-bis-(2,3,4,5,6-penta-O-sulfo-D-glucit-1-ylamid) Decanatriumsalz,
3,3'-Ethylendibenzoesäure-bis-(2,3,4,5,6-penta-O-sulfo-D-glucit-1-ylamid) Decanatriumsalz,
(E)-2-Chlorstilben-4,4'-dicarbonsäure-bis-(2,3,4,5,6-penta-O-sulfo-D-glucit-1-ylamid) Decanatriumsalz,
(Z)-2-Chlorstilben-4,4'-dicarbonsäure-bis-(2,3,4,5,6-penta-O-sulfo-D-glucit-1-ylamid) Decanatriumsalz,
(E)-2-Bromstilben-4,4'-dicarbonsäure-bis-(2,3,4,5,6-penta-O-sulfo-D-glucit-1-ylamid) Decanatriumsalz,
(Z)-2-Bromstilben-4,4'-dicarbonsäure-bis-(2,3,4,5,6-penta-O-sulfo-D-glucit-1-ylamid) Decanatriumsalz,
(Z)-Stilben-4,4'-dicarbonsäure bis-(2,3,4,5,6-penta-O-sulfo-D-mannit-1-ylamid) Decanatriumsalz,
(Z)-Stilben-4,4'-dicarbonsäure bis-(2,3,4,5,6-penta-O-sulfo-D-galactit-1-ylamid) Decanatriumsalz,
(Z)-Stilben-4,4'-dicarbonsäure bis-(2,3,4,5,6-penta-O-sulfo-D-arabinit-1-ylamid) Octanatriumsalz,
(Z)-Stilben-4,4'-dicarbonsäure bis-(2,3,4,5,6-penta-O-sulfo-L-rhamnit-1-ylamid) Octanatriumsalz.

Beispiele von repräsentativen Vertretern, worin das aromatische Ringsystem Naphthylen oder Fluorenylen ist, sind:

Naphthalin-2,6-dicarbonsäure-bis-(2,3,4,5,6-penta-O-sulfo-D-glucit-1-ylamid) Decanatriumsalz,
Naphtalin-1,5-disulfonsäure-bis-(2,3,4,5,6-penta-O-sulfo-D-glucit-1-ylamid) Decanatriumsalz,
Naphthalin-1,4-dicarbonsäure-bis-(2,3,4,5,6-penta-O-sulfo-D-glucit-1-ylamid) Decanatriumsalz,
9-Oxo-9H-fluoren-2,7-dicarbonsäure-bis-(2,3,4,5,6-penta-O-sulfo-D-glucit-1-ylamid) Decanatriumsalz,
9-Oxo-9H-fluoren-2,7-dicarbonsäure bis-[methyl-(2,3,4,5,6-penta-O-sulfo-D-glucit-1-yl)-amid] Decanatriumsalz.

Beispiele von repräsentativen Vertretern, worin das aromatische Ringsystem Phenylen oder durch nieder-Alkyl substituiertes Phenylen ist, sind:

3,3'-Bis-(2,3,4,5,6-penta-O-sulfo-D-glucit-1-yl)-1,1'-(benzol-1,4-diyl)-dithioharnstoff Decanatriumsalz,
3,3'-Bis-(2,3,4,5,6-penta-O-sulfo-D-glucit-1-yl)-1,1'-(benzol-1,4-diyl)-dithioharnstoff Decanatriumsalz,
N,N'-Bis-(2,3,4,5,6-penta-O-sulfo-D-glucit-1-yl)-terephthalamid Decanatriumsalz,
N,N'-Bis-(2,3,4,5,6-penta-O-sulfo-D-glucit-1-yl)-isophthalamid Decanatriumsalz,
3,3'-Bis-(2,3,4,5,6-penta-O-sulfo-D-glucit-1-yl)-1,1'-(toluol-2,6-diyl)-diharnstoff Decanatriumsalz,
3,3'-Bis-(2,3,4,5,6-penta-O-sulfo-D-glucit-1-yl)-1,1'-(benzol-1,3-diyl-dimethylen)-diharnstoff Decanatriumsalz,
N,N'-Dimethyl-N,N'-bis-(2,3,4,5,6-penta-O-sulfo-D-glucit-1-yl)-terephtalamid Decanatriumsalz,
3,3'-Dimethyl-3,3'-bis-(2,3,4,5,6-penta-O-sulfo-D-glucit-1-yl)-1,1'-(toluol-2,6-diyl)-diharnstoff Decanatriumsalz,
3,3'-Bis-(2,3,4,5,6-penta-O-sulfo-D-mannit-1-yl)-1,1'-(toluen-2,6-diyl)-diharnstoff Decanatriumsalz,
3,3'-Bis-(2,3,4,5,6-penta-O-sulfo-D-galactit-1-yl)-1,1'-(toluen-2,6-diyl)-diharnstoff Decanatriumsalz,

3,3'-Bis-(2,3,4,5,6-penta-O-sulfo-D-arabinit-1-yl)-1,1'-(toluen-2,6-diyl)-diharnstoff Octanatriumsalz,

3,3'-Bis-(2,3,4,5,6-penta-O-sulfo-L-rhamnit-1-yl)-1,1'-(toluen-2,6-diyl)-diharnstoff Octanatriumsalz.

Beispiele von repräsentativen Vertretern, worin das aromatische Ringsystem ein Rest der Formel (b)-(s), sind:

4,4'-Sulfonyl-dibenzoesäure-bis-[4-(2,3,4,5,6-penta-O-sulfo-D-glucit-1-ylcarbamoyl)-benzylamid] Decanatriumsalz, Formel b)

N,N'-Bis-[4-[3-(2,3,4,5,6-penta-O-sulfo-D-glucit-1-yl)-ureido]-phenyl]-isophthalamid Decanatriumsalz, Formel c)

N,N'-Bis-[4-(2,3,4,5-tetra-O-sulfo-D-arabinit-1-yloxy)-phenyl]-isophthalamid Octanatriumsalz, Formel c)

N,N'-Bis-[4-[3-(2,3,4,5,6-penta-O-sulfo-D-glucit-1-yl)-ureido]-phenyl]-isophthalamid Decanatriumsalz, Formel c)

N,N'-Bis-[4-(2,3,4,5-tetra-O-sulfo-D-arabinit-1-yloxy)-phenyl]-terephthalamid Octanatriumsalz, Formel c)

N,N'-Bis-[4-[3-(2,3,4,5,6-penta-O-sulfo-D-glucit-1-yl)-ureido]-phenyl]-fumaramid Decanatriumsalz, Formel d)

2-[4-[4-[4-[1-(2,3,4,5,6-Penta-O-sulfo-D-glucit-1-ylcarbamoyl)-ethoxy]-phenoxy]-phenoxy]-phenoxy]-propionsäure-2,3,4,5,6-penta-O-sulfo-D-glucit-1-ylamid Decanatriumsalz, Formel e)

(Z)-But-2-endicarbonsäure 1-[4-(4-chloro-phenoxy)-3-(2,3,4,5,6-penta-O-sulfo-D-glucit-1-ylcarbamoyl)-phenyl]-amid 4-(2,3,4,5,6-penta-O-sulfo-D-glucit-1-yl)-amid Decanatriumsalz, Formel f)

N1-[4-(4-chloro-phenoxy)-3-(2,3,4,5,6-penta-O-sulfo-D-glucit-1-ylcarbamoyl)-phenyl]-N4-(2,3,4,5,6-penta-O-sulfo-D-glucit-1-yl)-bernsteinsäureamid Decanatriumsalz, Formel f)

(Z)-But-2-endicarbonsäure 1-(2,3,4,5,6-penta-O-sulfo-D-glucit-1-yl)-amid 4-[4-(2-methoxy-ethoxy)-3-(2,3,4,5,6-penta-O-sulfo-D-glucit-1-ylcarbamoyl)-phenyl]-amid Decanatriumsalz, Formel f)

N1-[4-(2-methoxy-ethoxy)-3-(2,3,4,5,6-penta-O-sulfo-D-glucit-1-ylcarbamoyl)-phenyl]-N4-(2,3,4,5,6-penta-O-sulfo-D-glucit-1-yl)-bernsteinsäureamid Decanatriumsalz, Formel f)

N-(2,3,4,5,6-Penta-O-sulfo-D-glucit-1-yl)-4-(2,3,4,5,6-penta-O-Sulfo-D-glucit-1-ylcarbamoylmethoxy)-benzamid Decanatriumsalz, Formel g)

5,5'-(Methylene-bis-oxymethylen)-di-furan-2-carbonsäure bis-(2,3,4,5,6-penta-O-sulfo-D-glucit-1-yl-amid) Decanatriumsalz, Formel h)

4'-[4-[4-[4'-(2,3,4,5,6-Penta-O-sulfo-D-glucit-1-ylcarbamoyl)-biphenyl-4-yloxy]-phenylsulfonyl]-phenoxy]-biphenyl-4-carbonsäure 2,3,4,5,6-penta-O-sulfo-D-glucit-1-ylamid Decanatriumsalz, Formel i)

5-Biphenyl-4-ylmethoxy-N,N'-bis-(2,3,4,5,6-penta-O-sulfo-D-glucit-1-yl)-isophthalamid Decanatriumsalz, Formel j)

N-(2,3,4,5,6-Penta-O-sulfo-D-glucit-1-yl)-2-[4-[4-(2,3,4,5,6-penta-O-sulfo-D-glucit-1-ylcarbamoylmethoxy)-benzyl]-phenoxy]-acetamid Decanatriumsalz, Formel k)

(Z)-Butendisäure (Z)-[3-biphenyl-4-yloxymethyl-5-[3-(2,3,4,5,6-penta-O-sulfo-D-glucit-1-ylcarbamoyl)-acryloylamino]-phenylamid]-(2,3,4,5,6,-penta-O-sulfo-D-glucit-1-yl)-amid Decanatriumsalz, Formel l)

N1-[3-Biphenyl-4-yloxymethyl-5-[3-(2,3,4,5,6-penta-O-sulfo-D-glucit-1-ylcarbamoyl)-propionylamino]-phenyl]-N4-(2,3,4,5,6-penta-O-sulfo-D-glucit-1-yl)-bernsteinsäureamid Decanatriumsalz, Formel l)

3,3'-Bis-(2,3,4,5,6-penta-O-sulfo-D-glucit-1-yl)-1,1'-(5-biphenyl-4-yloxymethyl-benzol-1,3-diyl)-diharnstoff Decanatriumsalz, Formel l)

2,2',3,3',4,4',5,5'-Octa-O-sulfo-1,1'-O-(9,9-dimethyl-9H-xanthen-3,6-diyl)-di-D-arabinitol Octanatriumsalz, Formel m)

3,6,9-Trioxaundecandisäure bis-[4-(2,3,4,5-tetra-O-sulfo-D-arabinit-1-yloxy)phenylamid] Octanatriumsalz, Formel n)

4,4'-Bis-(2,3,4,5,6-penta-O-sulfo-D-glucit-1-ylamino)-3,3'-dinitro-N.N'-(4,4'-methylen-diphenyl)-dibenzamid Decanatriumsalz, Formel o)

N,N'-Bis-(2,3,4,5,6-penta-O-sulfo-D-glucit-1-yl)-2,2'-dinitro-4,4'-sulfonyldianilin Decanatriumsalz, Formel p)

4,4'-Bis-(2,3,4,5,6-penta-O-sulfo-D-glucit-1-ylamino)-3,3'-dinitro-N,N'-benzen-1,3-diyl-dibenzamid Decanatriumsalz, Formel q)

4,4'-Bis-(2,3,4,5,6-penta-O-sulfo-D-glucit-1-ylamino)-3,3'-dinitro-N,N'-(2-hydroxysulfonyloxy-propan-1,3-diyl)-dibenzamid Undekanatriumsalz, Formel r)

(E)-4,4'-Bis-(2,3,4,5,6-penta-O-sulfo-D-glucit-1-ylamino)-3,3'-dinitro-N,N'-stilben-4,4'-diyl-dibenzamid Decanatriumsalz, Formel s).

Beispiele von repräsentativen Verbindungen, worin das System konjugierter Mehrfachbindungen B ein Polyen-Kohlenwasserstoff ist, sind:

(2E,4E,6E,8E,10E,12E,14E)-2,6,11,15-Tetramethyl-hexadeca-2,4,6,8,10,12,14-heptaendicarbonsäure bis-(2,3,4,5,6-penta-O-sulfo-D-glucit-1-ylamid) Decanatriumsalz,

(2E,4E,6E)-2,7-Dimethyl-octa-2,4,6-triendicarbonsaure-bis-(2,3,4,5,6-penta-O-sulfo-D-glucit-1-ylamid) Decanatriumsalz.

Ein System konjugierter Dreifachbindungen ist beispielsweise ein Polyin-Kohlenwasserstoffrest mit 2 konjugierten Dreifachbindungen; eine repräsentative derartige Verbindung ist beispielsweise 2,2',3,3',4,4',5,5'-Octa-O-sulfo-1,1'-O-hexa-2,4-diyn-1,6-diyl-di-D-arabinitol Octanatriumsalz.

Die Verbindungen der Formel I können dadurch hergestellt werden, dass man eine der allgemeinen Formel I entsprechende, nicht sulfatierte Verbindung mit einem Sulfatierungsmittel umsetzt.

Die erfindungsgemässe Sulfatierung kann mittels Methoden, die für die Sulfatierung von Hydroxygruppen an sich bekannt sind, vorgenommen werden. Beispiele von Sulfatierungsmitteln sind $SO_3$ - Komplexe wie $SO_3$·Pyridin, $SO_3$·Trimethylamin, $SO_3$·Dioxan und $SO_3$·Dimethylformamid. Weitere Beispiele von Sulfatierungsmitteln sind Chlorsulfonsäure, Gemische von Chlorsulfonsäure und Schwefelsäure; und Piperidin-N-Sulfat.

Die Umsetzung erfolgt zweckmässig in einem geeigneten Lösungsmittel, insbesondere einem polaren Lösungsmittel, z.B. Dimethylformamid, Dimethylsulfoxid oder Hexamethylphosphortriamid. Die Reaktion kann bei Raumtemperatur oder bei erhöhter Temperatur, z.B. bei 20-70 °C, durch-geführt werden, wobei durch Variation von Reaktionsdauer und -temperatur der Sulfatierungsgrad beeinflusst werden kann. Der jeweils erreichte Sulfatierungsgrad kann durch HPLC abgeschätzt werden. In einer bevorzugten Ausführungsform werden durch geeignete Wahl von Reaktionsdauer und -temperatur alle oder praktisch alle freien Hydroxygruppen sulfatiert. Die Aufarbeitung des Reaktiongemisches bzw. die Isolierung des Reaktionprodukts der Formel I kann nach an sich bekannten Methoden erfolgen, z. B. durch Gelfiltration oder Ultrafiltration. Zweckmässig wird das Reaktionsgemisch vor der Aufarbeitung mit einer zur Salzbildung mit den Sulfonsäuregruppen in der Verbindung der Formel I befähigten, hinreichend basischen Verbindung, z.B. mit einem Alkalimetallazetat, wie Natriumazetat, versetzt und die Verbindung der Formel I in Salzform, z.B. als Natriumsalz, isoliert.

Die Ausgangsstoffe für das erfindungsgemässe Verfahren, d.h. die den Verbindungen der Formel I entsprechenden, nicht sulfatierten Verbindungen können wie in den nachstehenden Beispielen beschrieben oder in Analogie dazu hergestellt werden. Die Ausgangsverbindungen für Substanzen der Formel I können allgemein wie folgt dargestellt werden:

Ist X = $-NR^1CO$, kann ein geeignetes Glycamin, d. h. ein 1-Amino-1-desoxy-derivat eines Zuckers, ein entsprechendes $R^1$-substituiertes Glycamin oder Tris-(hydroxymethyl)-methylamin mit einer geeigneten Carbonsäure umgesetzt werden. Hierzu wird die Carbonsäure mit in der Peptidchemie bekannten Methoden aktiviert, wie z. B. als gemischtes Anhydrid oder als Aktivester, in gewissen Fällen kann ein einfacher Ester oder ein Lacton zur Kopplung eingesetzt werden.

$R^1$-substituierte Glycamine können direkt umgesetzt werden oder aber Glycamine können gekoppelt und anschliessend mit $R^1$ derivatisiert werden.

Harnstoff- oder Thioharnstoffderivate (X = -NHCONH- oder -NHCSNH-) können z. B. direkt durch Umsetzung eines Isocyanates oder Isothiocyanates mit einem geeigneten Glycamin, einem entsprechenden substituierten Glycamin oder Tris-(hydroxymethyl)-methylamin gewonnen werden.

Sulfonamide (X = $-NHSO_2-$) sind im Prinzip zugänglich durch Reaktion einer aktivierten Sulfonylverbindung $-SO_2Z$ (z. B. eines Sulfonylchlorids) mit einem geeigneten Glycamin, einem entsprechenden substituierten Glycamin oder Tris-(hydroxymethyl)-methylamin.

Analoge Ether (X = O) sind erhältlich durch Umsetzung eines Glycitols mit einem Phenol oder einem benzylischen oder allylischen Alkohol. Nach im Prinzip bekannten Methoden der Etherbildung kann eine der Hydroxylgruppen aktiviert werden (wie z. B. nach Mitsunobu), oder es kann ein aktiviertes Hydroxylequivalent zur Umsetzung gebracht werden (wie z. B. ein Allyl- oder Benzylhalogenid). Aminoverbindungen (X = $NR^1$) sind nach an sich bekannten Methoden erhältlich, so z.B. durch Umsetzung eines Aminoglycitols mit einem aktivierten Aromaten.

Die erfindungsgemässen Verbindungen hemmen die Migration und Proliferation von glatten Muskelzellen der Gefässwand. Sie können also zur Behandlung und/oder Prophylaxe von arteriosklerotischen Gefässwandveränderungen, insbesondere zur Verhinderung von Restenose nach koronarer oder peripherer Angioplastie oder nach Bypass-Operationen, verwendet werden. Im Prinzip können diese Substanzen zur Behandlung und/oder Prophylaxe von allen Erkrankungen, bei denen Migration oder Proliferation von glatten Muskelzellen eine Rolle spielen, eingesetzt werden.

Im Gegensatz zu Heparin haben diese Verbindungen aber keine $AT_{III}$-Aktivität (Antithrombin III) und somit keine inhibierende Wirkung auf die Gerinnungsfaktoren IIa und Xa. Demzufolge ist ihre blutgerinnungshemmende Aktivität sehr viel geringer als die von Heparin, und damit ist das Blutungsrisiko bei der Therapie mit diesen Substanzen minimal.

Da Heparin-bindende Proteine bei verschiedenen Krankheiten eine wichtige Rolle spielen, können Heparin-ähnliche Substanzen wie die in diesem Patent erwähnten Verbindungen zusätzlich auch zur Behandlung dieser Krankheiten eingesetzt werden: z.B. wird die Aufnahme von verschiedenen Viren (Herpes, HIV) durch solche Substanzen inhibiert, die arterielle Thrombose (vWF, Plättchenadhäsion) wird durch solche Substanzen gehemmt, die Aktivierung des Komplementsystem (z.B. bei Reperfusion) kann gemindert werden, und verschiedene Wachstumsfaktoren oder Cytokine (z.B. bFGF in Tumoren) können inhibiert werden.

Die pharmakologischen Aktivitäten der erfindungsgemässen Verbindungen können in den nachstehend beschriebenen Versuchsanordnungen gezeigt werden:

Antiproliferative Aktivität

Die antiproliferative Aktivität einer Substanz wird als $r_i$-Wert ausgedrückt, der einen Vergleichswert zu der entsprechenden Aktivität von Heparin darstellt und der in Zellkulturen wie folgt ermittelt wurde: Glatte Muskelzellen der Ratte wurden mit einer Dichte von $8x10^3$ Zellen/Loch auf Zellkulturplatten ausgebracht (Medium: DMEM mit 10% FCS. Kultivation bei 37°C und 5% $CO_2$). Nach 4 Stunden wurde die Zahl der adhärierten Zellen bestimmt und die zu testenden Substanzen (100 µg/ml, gelöst in $H_2O$) zugegeben. Als Kontrolle dienten a) Zellen, denen nichts zugegeben wurde, und b) Heparin (100 µg/ml). Anschliessend wurden die Zellen für 48 h inkubiert, und danach wurde wieder die Zellzahl bestimmt.

Aus diesen Werten wurde die Inhibition **i** des Zellwachstums, d.h. die Erniedrigung der Wachstumsrate der Zellen in Prozenten im Vergleich zur Kontrolle ausgerechnet.

$$i = 100 - \frac{\mu \text{ Substanz}}{\mu \text{ Kontrolle}} \cdot 100$$

wobei die Wachstumsrate µ errechnet wurde als

$$\mu = \frac{\Delta \ln Z}{\Delta t_{[d]}} = \ln \frac{Z_{(t_2)}}{Z_{(t_1)}} * \frac{1}{\Delta t_{[d]}} \; [d^{-1}]$$

wobei Z die Anzahl der Zellen ist.

Schliesslich wurde $r_i$ - die relative inhibitorische Aktivität - welche the Aktivität von einer Substanz (bei 100 µg/ml) im Vergleich zur Aktivität von Heparin bei gleicher Konzentration im gleichen Experiment ausdrückt, errechnet:

$$r_i = \frac{i_{Substanz}}{i_{Heparin}}$$

Blutgerinnungshemmung

Die blutgerinnungshemmende Wirkung wurde wie folgt ermittelt:

Hemmung von Thrombin oder Faktor Xa im Chromogen Substrat Test (Teien et al., Thrombosis Research 10.399-410(1977)): Die Bestimmung erfolgte im Cobas-Bio Centrifugal Automatic Spectrophotometer. Die verwendete Pufferlösung bestand aus 50 mM Tris-Puffer, 180 mM NaCl, 7.5 mM EDTA-$Na_2$, 1% PEG 6000 und 0.02% Tween-80, pH 8.4. Die Testlösung bestand aus 50 µl Puffer, 30 µl Antithrombin III (1U/ml, Kabi-Diagnostica) und 20 µl Plasma, das verschiedene Konzentrationen der Versuchsverbindungen enthielt. Im Analyseautomaten wurden 30 µl Probenlösung und 20 µl Wasser mit 180 µl Thrombin (1U/ml, Thrombin Reagent Roche Basel) in die Testküvette gegeben. Nach 240 Sekunden Inkubation bei 37 °C wurden 60 µl S-2238 (H-D-Phe-Pip-Arg-NH.pNA, Kabi Diagnostica, Möndal, Schweden, 0.75 mM in Wasser) und 20 µl Wasser zugesetzt. Die Freisetzung von pNA (p-Nitro-anilin) wurde während 60 Sekunden bei 405 nm in 10-Sekunden-Intervallen im Vergleich zu Wasser als Blindwert verfolgt. Die Hemmwirkung wird als $IC_{50}$, d.h. als Konzentration [µg/ml] angegeben, bei der die amidolytische Aktivität von Thrombin im Vergleich zum Plasma-Kontrollwert um 50% vermindert wird.

In gleicher Weise wurde die Hemmung von Faktor Xa gemessen, wobei eine Lösung von Faktor Xa (2.8 nkat/ml und 2 mM S-2222 (Bz-CO-Ile-Glu-Arg-NH.pNA, Kabi Diagnostica) in Wasser anstelle von Thrombin bzw. S-2238 verwendet wurde.

In der nachstehenden Tabelle sind die in den vorstehend beschriebenen Versuchsanordnungen mit einer repräsentativen Anzahl Verbindungen der Formel I erhaltenen Aktivitätsdaten angeführt:

| **Beispiel** | **Antiproliferative Aktivität ri** | **Antikoagulative Aktivität $IC_{50}$ [µg/ml]** | |
|---|---|---|---|
| | | **Thrombin** | **Faktor Xa** |
| 1 | 0,6 | >1000 | >1000 |
| 3 | 1,5 | >1000 | >1000 |
| 4 | 0,3 | >1000 | >1000 |
| 6 | 1,5 | >1000 | >1000 |
| 10 | 1,5 | >1000 | >1000 |

(fortgesetzt)

| Beispiel | Antiproliferative Aktivität ri | Antikoagulative Aktivität $IC_{50}$ [µg/ml] | |
|---|---|---|---|
| | | Thrombin | Faktor Xa |
| 11 | 1,0 | >1000 | >1000 |
| 12 | 0,9 | >1000 | >1000 |
| 14 | 0,6 | >1000 | >1000 |
| 16 | 0,7 | >1000 | >1000 |
| 26 | 1,5 | >1000 | >1000 |
| 33 | 0,8 | >1000 | >1000 |
| 34 | 0,4 | >1000 | >1000 |
| 37 | 0,7 | >1000 | >1000 |
| 56 | 2,2 | >1000 | >1000 |
| 65 | 0,8 | >1000 | >1000 |
| 67 | 1,0 | >1000 | >1000 |
| 68 | 1,1 | >1000 | >1000 |
| 85 | 2,1 | >1000 | >1000 |
| 93 | 1,0 | >1000 | >1000 |
| Heparin | 1,0 | 1,9 | 2,7 |

Die Versuchsresultate zeigen, dass die erfindungsgemässen Verbindungen eine antiproliferative Wirkung aufweisen, die derjenigen von Heparin entspricht (oder nahekommt) oder grösser ist, im Gegensatz zu Heparin jedoch keine oder eine viel geringere anti-Koagulationswirkung zeigt.

Die Arzneimittel auf der Basis der erfindungsgemässen Verbindungen können enteral, z.B. oral in Form von Tabletten, Lacktabletten, Dragées, Hart- und Weichgelatinekapseln, Lösungen, Emulsionen oder Suspensionen, oder rektal, z.B. in Form von Suppositorien, verabreicht werden. Die Verabreichung erfolgt jedoch vorzugsweise parenteral, z.B. in Form von Injektionslösungen.

Zur Herstellung von Tabletten, Lacktabletten, Dragées und Hartgelatinekapseln kann der Wirkstoff mit pharmazeutisch inerten, anorganischen oder organischen Excipientien vermischt werden. Als solche Excipientien kann man für Tabletten, Dragées und Hartgelatinekapseln z.B. Lactose, Maisstärke oder Derivate davon, Talk, Stearinsäure oder deren Salze, verwenden. Für Weichgelatinekapseln eignen sich als Excipientien z.B. vegetabile Oele, Wachse, Fette, halbfeste und flüssige Polyole; je nach Beschaffenheit des Wirkstoffes sind jedoch bei Weichgelatinekapseln überhaupt keine Excipientien erforderlich. Zur Herstellung von Lösungen und Sirupen eignen sich als Excipientien z.B. Wasser, Polyole, Saccharose, Invertzucker und Glukose, für Injektionslösungen eignen sich z.B. Wasser, Alkohole, Polyole, Glycerin und vegetabile Oele, und für Suppositorien eignen sich z.B. natürliche oder gehärtete Oele, Wachse, Fette und halbflüssige oder flüssige Polyole.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Bei enteraler Verabreichung kann die Resorption des Wirkstoffs mit Hilfe von Liposomen erhöht werden.

Die Dosierung des Wirkstoffes kann innerhalb weiter Grenzen variieren und ist natürlich in jedem einzelnen Fall den individuellen Gegebenheiten anzupassen. Im allgemeinen dürfte bei parenteraler Verabreichung eine Dosis von etwa 0,1 bis 100 mg/kg, vorzugsweise von etwa 1,5 bis 15 mg/kg, pro Tag für den Erwachsenen angemessen sein, wobei aber die soeben angegebene obere Grenze auch überschritten werden kann, wenn sich dies als angezeigt erweisen sollte.

Die Erfindung wird durch die nachstehenden Beispiele weiter erläutert.

Beispiel 1

A. Eine Suspension von 4,33 g Naphthalin-2,6-dicarbonsäure in 5 ml Wasser und 20 ml Aceton wurde bei 0 - 5 °C über 15 Minuten mit 6,2 ml Triethylamin in 50 ml Aceton versetzt. Bei -5 - 0 °C wurden dann 4,8 g Chlorameisensäureethylester in 30 ml Aceton zugegeben. Eine Lösung von 8,33 g D-Glucamin (1-Amino-1-desoxy-glucitol) in 50 ml Wasser wurde während 20 Minuten bei 0 - 5 °C zugetropft. Nach 20 Stunden Rühren bei Raumtemperatur wurde für 2 Stunden auf 50 °C erwärmt. Das Reaktionsgemisch wurde eingedampft, mit Acetanhydrid in Pyridin 17 Stunden bei Raumtemperatur acetyliert und wiederum eingedampft. Der Rückstand wurde extrahierend mit

Eiswasser und Methylenchlorid aufgearbeitet. Die organische Phase wurde mit Natriumhydrogencarbonatlö-sung und Wasser gewaschen, über Magnesiumsulfat getrocknet und eingedampft. Der Rückstand wurde über Kieselgel chromatographiert. Die Produktfraktionen wurden in Methylenchlorid aufgenommen und zur Kristallisation mit Methanol versetzt. Die farblosen Kristalle wurden abgenutscht und mit Methanol und kaltem Ether gewaschen. Eine Suspension von 5.0 g dieses Produktes in 75 ml Dioxan und 50 ml Methanol wurde mit 3 ml einer frisch bereiteten Natriummethylatlösung (2 % Na in Methanol) versetzt und 5 Stunden bei Raumtemperatur und anschliessend 20 Minuten unter Eiskühlung gerührt. Der Niederschlag von Naphthalin-2,6-dicarbonsäure-bis-D-glucit-1-ylamid wurde abgenutscht, mit Methanol/Dioxan 1:1 gewaschen und getrocknet, $[\alpha]_D^{20}$ -14° (*c* 0,5; Dimethylsulfoxid), MS: *m/z* 543,4 ($[M+H]^+$).

B. Eine Suspension von 1,1 g Naphthalin-2,6-dicarbonsäure-bis-D-glucit-1-ylamid und 5,6 g Schwefeltrioxidtrimethylaminkomplex in 20 ml absolutem Dimethylformamid wurde 22 Stunden bei 70 - 75 °C gerührt. Nach Abkühlen wurde mit 6,56 g Natriumacetat in 50 ml Wasser versetzt und eingedampft. Der Rückstand wurde in Wasser aufgenommen und der pH-Wert mit verdünnter Natronlauge auf 7 - 8 eingestellt. Die Lösung wurde eingedampft und in destilliertem Wasser per Gelfiltration (Sephadex® LH 20) gereinigt. Die Produktfraktionen wurden lyophilisiert und ergaben Naphthalin-2,6-dicarbonsäure-bis-(2,3,4,5,6-penta-O-sulfo-D-glucit-1-ylamid) Decanatriumsalz, $[\alpha]_D^{20}$ -7,4° (*c* 0,5; Wasser), MS: *m/z* 1562,0 (rekonstruiertes M).

<u>Beispiel 2</u>

A. Eine Suspension von 4,03 g E-Stilben-4,4'-dicarbonsäure in 37,5 ml Acetonitril und 37,5 ml Tetrahydrofuran wurde bei -10 °C mit 4,6 ml Triethylamin und 4,3 ml Chlorameisensäureisobutylester versetzt und bei -4 - 0 °C 50 Minuten unter Argon gerührt. Eine Lösung von 6,79 g D-Glucamin in 20 ml Wasser und 10 ml Acetonitril wurde zugetropft. Nach 3 Stunden bei Raumtemperatur und 16 Stunden bei 50 °C wurde eingedampft und der Rückstand mit Acetanhydrid in Pyridin acetyliert. Aufarbeitung, Trennung und Deacetylierung wie in 1.A. beschrieben ergaben (E)-Stilben-4,4'-dicarbonsäure-bis-D-glucit-1-ylamid, $[\alpha]_D^{20}$ -2,6° (*c* 0,5; Dimethylsulfoxid), MS: *m/z* 595,4 ($[M+H]^+$).

B. Eine Suspension von 0,59 g (E)-Stilben-4,4'-dicarbonsäure-bis-D-glucit-1-ylamid und 2,78 g Schwefeltrioxidtrimethylaminkomplex in 20 ml absolutem Dimethylformamid wurde 22 Stunden bei 70 - 75 °C gerührt. Nach Abkühlen wurde die obere Phase abdekantiert. Der Rückstand wurde mit 1,64 g Natriumacetat in 20 ml Wasser versetzt und eingedampft. Der Rückstand wurde in Wasser aufgenommen, erneut eingedampft und in destilliertem Wasser per Gelfiltration (Sephadex® LH 20) gereinigt. Die Produktfraktionen wurden lyophilisiert und ergaben (E)-Stilben-4,4'-dicarbonsäure-bis-(2,3,4,5,6-penta-O-sulfo-D-glucit-1-ylamid) Decanatriumsalz, $[\alpha]_D^{20}$ -5,6° (*c* 0,5; Wasser), MS: *m/z* 1614,5 (rekonstruiertes M).

<u>Beispiel 3</u>

A. Eine Suspension von 1,34 g (Z)-Stilben-4,4'-dicarbonsäure in 12,5 ml Acetonitril, 12,5 ml Tetrahydrofuran und 12,5 ml Dimethylformamid wurde bei -10 °C mit 2,0 ml Triethylamin und 1,44 ml Chlorameisensäureisobutylester in 5 ml Tetrahydrofuran versetzt und bei -15 - -20 °C 20 Minuten unter Argon gerührt. Dann wurde 2,27 g D-Glucamin zugegeben. Nach 2 Stunden bei Raumtemperatur und 18 Stunden bei 45 °C wurde eingedampft und der Rückstand mit Acetanhydrid in Pyridin acetyliert. Aufarbeitung, Trennung und Deacetylierung wie in 1.A. beschrieben ergaben (Z)-Stilben-4,4'-dicarbonsäure-bis-D-glucit-1-ylamid, $[\alpha]_D^{20}$ -3,8° (*c* 0,5; Dimethylsulfoxid), MS: *m/z* 595,4 ($[M+H]^+$).

B. Sulfatierung von (Z)-Stilben-4,4'-dicarbonsäure-bis-D-glucit-1-ylamid wie unter 2.B. beschrieben ergab (Z)-Stilben-4,4'-dicarbonsäure-bis-(2,3,4,5,6-penta-O-sulfo-D-glucit-1-ylamid) Decanatriumsalz, $[\alpha]_D^{20}$ -3,5° (*c* 0,4; Wasser), MS: *m/z* 1615,5 (rekonstruiertes M).

<u>Beispiel 4</u>

Eine Lösung von 3,34 g 4,4'-Sulfonyl-bis-(benzoesäuremethylester) in 100 ml absolutem Methanol wurde mit 7,24 g D-Glucamin versetzt und 4 Tage unter Rückfluss erwärmt. Nach Abkühlung wurde das Reaktionsgut abgenutscht und getrocknet. Acetylierung, Chromatographie und Deacetylierung analog Beispiel 1.A. ergaben 4,4'-Sulfonyl-dibenzoesäure-bis-D-glucit-1-ylamid, $[\alpha]_D^{20}$ -7,4° (*c* 0,5; Dimethylsulfoxid), MS: *m/z* 633,4 ($[M+H]^+$).

B. Sulfatierung von 4,4'-Sulfonyl-dibenzoesäure-bis-D-glucit-1-ylamid wie unter 1.B. beschrieben ergab 4,4'-Sulfonyl-dibenzoesäure-bis-(2,3,4,5,6-penta-O-sulfo-D-glucit-1-ylamid) Decanatriumsalz, $[\alpha]_D^{20}$ -3,5° (*c* 0,4; Wasser),

MS: *m/z* 1652,5 (rekonstruiertes M).

Beispiel 5

A. Eine Lösung von 1,472 g 4,4'-(Phenylen-1,4-dioxy)-diphenol in 10 ml Dimethylformamid wurde langsam und unter Feuchtigkeitsausschluss bei 0 - 5 °C zu einer Aufschlämmung von 0,48 g Natriumhydriddispersion in 10 ml Dimethylformamid zugetropft und anschliessend 1 Stunde bei Raumtemperatur gerührt. Hierauf wurden 2,0 g 2-Brompropionsäureethylester gelöst in 10 ml Dimethylformamid innert 5 Minuten zugetropft und das Reaktionsgemisch während 16 Stunden bei 100 °C gerührt. Danach wurde das Reaktionsgemisch auf 150 ml Eiswasser gegossen, mit Methylenchlorid extrahiert, die Methylenchloridphase über Magnesiumsulfat getrocknet und eingeengt.

Das so erhaltene ölige Rohprodukt wurde in einem Gemisch von 50 ml Methanol und 10 ml 4N Kaliumhydroxydlösung gelöst und 4 Stunden unter Rückfluss erwärmt. Nach dem Abkühlen wurde der grösste Teil des Methanols unter vermindertem Druck abdestilliert, dann wurde mit verdünnter wässriger Salzsäure angesäuert und der dabei gebildete Niederschlag abfiltriert. So wurde 2-[4-[4-[4-(1-Carboxy-ethoxy)-phenoxy]-phenoxy]-phenoxy]-propionsäure, ein Gemisch der racemischen und meso-Formen, als weisse Kristalle mit Smp. 135-136 °C erhalten.

B. Eine Suspension von 0,877 g 2-[4-[4-[4-(1-Carboxy-ethoxy)-phenoxy]-phenoxy]-phenoxy]-propionsäure in 10 ml Acetonitril wurde unter Feuchtigkeitsausschluss bei 0 - 5 °C mit 0,5 mi 4-Methylmorpholin versetzt und während 10 Minuten intensiv gerührt. Hierauf wurden 0,704 g 2-Chlor-4,6-dimethoxy-1,3,5-triazin fest hinzugefügt und weitere 2 Stunden bei 0 - 5°C gerührt. Anschliessend wurden 0,725 g D-Glucamin gefolgt von 10 ml Dimethylformamid zugesetzt und weitere 18 Stunden bei Raumtemperatur gerührt. Hierauf wurde das Reaktionsgemisch mit Wasser verdünnt und mehrmals unter vermindertem Druck bei 40 - 50 °C eingedampft. Das so erhaltene Rohprodukt wurde hierauf an LiChroprep RP-18 Kieselgel mit Wasser und anschliessend mit Wasser mit ansteigendem Anteil an Methanol chromatographiert. Nach dem Eindampfen der relevanten Fraktionen und Trocknen bei 50 °C im Vakuum wurde 2-[4-[4-[4-(1-D-Glucit-1-xylcarbamoyl-ethoxy)-phenoxy]-phenoxy]-phenoxy]-propionsäure-D-glucit-1-ylamid, ein Gemisch von 3 Diastereomeren, als weisses Pulver erhalten, MS: *m/z* 765,4 ($[M+H]^+$).

C. Das oben erhaltene 2-[4-[4-[4-(1-D-Glucit-1-ylcarbamoyl-ethoxy]-phenoxy]-phenoxy]-phenoxy]-propionsäure-D-glucit-1-ylamid wurde analog Beispiel 1.B. zu 2-[4-[4-[4-[1-(2,3,4,5,6-Penta-O-sulo-D-glucit-1-ylcarbamoyl)-ethoxy]-phenoxy]-phenoxy]-phenoxy]-propionsäure-2,3,4,5,6-penta-O-sulfo-D-glucit-1-ylamid Decanatriumsalz (Gemisch von 3 Diastereomeren), $[\alpha]_D^{20}$ - 5,7° (c= 0,6; Wasser), MS: *m/z* 1785,5 (rekonstruiertes M), umgesetzt.

Beispiel 6

A. Eine Suspension von 0,660 g 8,8'-Diapo-ψ,ψ-carotin-8,8'-dicarbonsäure in 10 ml Acetonitril wurde unter Feuchtigkeitsausschluss bei 0 - 5 °C mit 0,5 ml 4-Methylmorpholin versetzt und während 10 Minuten intensiv gerührt. Hierauf wurden 0.705 g 2-Chlor-4,6-dimethoxy-1,3,5-triazin fest hinzugefügt. Es wurde weitere 2 Stunden bei 0 - 5 °C gerührt. Anschliessend wurden 0,73 g D-Glucamin gefolgt von 10 ml Dimethylformamid zugesetzt und weitere 18 Stunden bei Raumtemperatur gerührt. Hierauf wurde das Reaktionsgemisch mit Wasser verdünnt und mehrmals unter vermindertem Druck bei 40 - 50°C eingedampft. Nun wurde mit 40 ml Wasser und 3 ml Triäthylamin versetzt, kurz aufgekocht und über einen 0,8 μ Cellulosefilter filtriert. Der Filterrückstand wurde mit Wasser gewaschen und bei 60°C im Vakuum getrocknet. So wurde (2E,4E,6E,8E,10E,12E,14E)-2,6,11,15-Tetramethyl-hexadeca-2,4,6,8,10,12,14-heptaendicarbonsäure-bis-D-glucit-1-ylamid als weisses Pulver erhalten, MS: *m/z* 655.6 ($[M+H]^+$).

B. Das oben erhaltene (2E,4E,6E,8E,10E,12E,14E)-2,6,11,15-Tetramethyl-hexadeca-2,4,6,8,10,12,14-heptaendicarbonsäure-bis-D-glucit-1-ylamid wurde analog Beispiel 1.B. zu (2E,4E,6E,8E,10E,12E,14E)-2,6,11,15-Tetramethyl-hexadeca-2,4,6,8,10,12,14-heptaendicarbonsäure bis-(2,3,4,5,6-penta-O-sulfo-D-glucit-1-ylamid) Decanatriumsalz, $[\alpha]_D^{20}$ -4,3° (*c* 1,0; Wasser), umgesetzt.

Beispiel 7

A. Einer Suspension von 27,0 g D-Glucamin in 500 mi trockenem Dimethylformamid wurde bei 5 - 10°C während einer Stunde portionsweise 24,9 g 4-Nitro-phenylisocyanat zugegeben. Nach 3 Stunden Rühren bei Raumtemperatur wurde mit 800 ml Methanol versetzt. Der entstandene Niederschlag wurde wurde abgenutscht, mit Methanol und Ether gewaschen und getrocknet, dann in 350 ml Wasser aufgeschlämmt, abgenutscht, mit Wasser gewaschen und getrocknet. Es wurde 1-D-Glucit-1-yl-3-(4-nitro-phenyl)-harnstoff erhalten, $[\alpha]_D^{20}$ -17,4° (*c* 0,5; Dimethylsulfoxid), MS : *m/z* 346,2 $(M+H]^+)$.

B. Eine Lösung von 18 g 1-D-Glucit-1-yl-3-(4-nitro-phenyl)-harnstoff in 250 ml Pyridin wurde mit 125 ml Acetanhydrid versetzt, 6 Stunden bei Raumtemperatur gerührt und bei vermindertem Druck eingeengt. Der Rückstand wurde in Essigester aufgenommen und mit Eiswasser, 2 N wässriger Schwefelsäure, Wasser, gesättigter wässriger Natriumhydrogencarbonatlösung und gesättigter Natriumchloridlösung gewaschen, über Magnesiumsulfat getrocknet und eingedampft. Dieses Rohprodukt wurde in 300 ml Ethanol und 160 ml 1N Chlorwasserstoffsäure gelöst und über 2,9 g 10 % Palladium auf Aktivkohle bei Raumtemperatur und 1,1 Bar hydriert. Nach 4 Stunden wurde das Reaktionsgemisch über Filtrierhilfe abgenutscht und mit Ethanol/Wasser gewaschen. Das Filtrat wurde eingeengt, in Essigester aufgenommen und mit gesättigter Hydrogencarbonatlösung und gesättigter Natriumchloridlösung gewaschen. Die organische Phase wurde über Magnesiumsulfat getrocknet und eingeengt. Der Rückstand wurde über Kieselgel chromatographiert, und Produktfraktionen wurden aus Methylenchlorid/Ether/Hexan kristallisiert. Es resultierte 1-(4-Amino-phenyl)-3-(2,3,4,5,6-penta-O-acetyl-D-glucit-1-yl)-harnstoff, $[\alpha]_D^{20}$ +13,8° (*c* 0,5; Chloroform), MS : *m/z* 526,3 $([M+H]^+)$.

C. Eine Lösung von 2,1 g 1-(4-Aminophenyl)-3-(2,3,4,5,6-penta-O-acetyl-D-glucit-1-yl)-harnstoff in 10 ml Tetrahydrofuran wurde bei 5 - 10°C mit 1,6 ml 4-Methylmorpholin und 410 mg Isophthalsäuredichlorid versetzt. Nach 3 Stunden Rühren bei Raumtemperatur wurde auf Eiswasser gegossen und mit Essigester extrahiert. Die organischen Phasen wurden mit gesättigter Natriumchloridlösung und Wasser gewaschen, über Magnesiumsulfat getrocknet und eingeengt. Der Rückstand wurde auf Kieselgel chromatographiert. Produktfraktionen wurden eingedampft und deacetyliert wie unter Beispiel 1.A. beschrieben. Es resultierte N,N'-Bis-[4-[3-(D-glucit-1-yl)-ureido]-phenyl]-isophthalamid, $[\alpha]_D^{20}$ -10,0° (*c* 0,2; Dimethylsulfoxid), MS: *m/z* 760,5 $(M^+)$.

D. Sulfatierung von N,N'-Bis-[4-[3-(D-glucit-1-yl)-ureido]-phenyl]-isophthalamid wie unter 2.B. beschrieben ergab N,N'-Bis-[4-[3-(2,3,4,5,6-penta-O-sulfo-D-glucit-1-yl)-ureido]-phenyl]-isophthalamid Decanatriumsalz, $[\alpha]_D^{20}$ +1,2° (*c* 0,5; Wasser), MS: *m/z* 1781,0 (rekonstruiertes M).

Beispiel 8

A. Eine Lösung von 1,74 g Toluol-2,4-diyl-diisocyanat in 50 ml Dimethylformamid wurde bei 0°C mit 3,8 g D-Glucamin versetzt und 5 Stunden bei Raumtemperatur gerührt. Das Lösungsmittel wurde am Hochvakuum abgedampft. Der Rückstand wurde mit 100 ml Methanol versetzt, 30 Minuten gerührt und abgenutscht. Das Nutschgut wurde mit Methanol und Ether gewaschen, getrocknet und ergab 3,3'-Di-D-glucit-1-yl-1,1'-(toluol-2,4-diyl)-diharnstoff, $[\alpha]_D^{20}$ -12,4° (*c* 0,5; Dimethylsulfoxid), MS: *m/z* 537,3 $(M^+)$.

B. Sulfatierung von 3,3'-Di-D-Glucit-1-yl-1,1'-(toluol-2,4-diyl)-diharnstoff wie unter 2.B. beschrieben ergab 3,3'-Bis-(2,3,4,5,6-penta-O-sulfo-D-glucit-1-yl)-1,1'-(toluol-2,4-diyl)-diharnstoff Decanatriumsalz, $[\alpha]_D^{20}$ -1,4°(*c* 0,5; Wasser), MS: *m/z* 1556,5 (rekonstruiertes M).

Beispiel 9

A. Eine Lösung von 0,96 g 1,4-Phenylen-diisothiocyanat in 30 ml Dimethylformamid wurde bei 0°C mit 1,9 g D-Glucamin versetzt und 3 Stunden bei Raumtemperatur gerührt. Das Lösungsmittel wurde am Hochvakuum abgedampft. Der Rückstand wurde mit 50 ml Methanol versetzt, bis zum Entstehen einer einheitlichen Suspension gerührt und abgenutscht. Das Nutschgut wurde mit Methanol und Ether gewaschen, getrocknet und ergab 3,3'-Di-D-Glucit-1-yl-1,1'-(benzol-1,4-diyl)-dithioharnstoff, $[\alpha]_D^{20}$ - 17,8°(*c* 0,5; Dimethylsulfoxid), MS: *m/z* 555,3 $(M^+)$.

B. Sulfatierung von 3,3'-Di-D-Glucit-1-yl-1,1'-(benzol-1,4-diyl)-dithioharnstoff wie unter 2.B. beschrieben ergab 3,3'-Bis-(2,3,4,5,6-penta-O-sulfo-D-glucit-1-yl)-1,1'-(benzol-1,4-diyl)-dithioharnstoff Decanatriumsalz, $[\alpha]_D^{20}$ +4,2° (*c* 0,5; Wasser), MS: *m/z* 1574,5 (rekonstruiertes M).

Beispiel 10

Eine Lösung von 0,2 g (E)-Stilben-4,4'-dicarbonsäure-bis-(2,3,4,5,6-penta-O-sulfo-D-glucit-1-ylamid) Decanatriumsalz (vgl. Beispiel 2) in 15 ml Wasser wurde in Gegenwart von 10 % Palladium auf Kohle während 3 Stunden bei Raumtemperatur hydriert. Der Katalysator wurde abfiltriert und das Filtrat eingeengt. Der Rückstand wurde über eine Ionenaustauschersäule gereinigt und ergab 4,4'-Ethylen-dibenzoesäure-bis-(2,3,4,5,6-penta-O-sulfo-D-glucit-1-ylamid) Decanatriumsalz, $[\alpha]_D^{20}$ -4,8°(*c* 0,4; Wasser), MS: *m/z* 1616,5 (rekonstruiertes M).

Beispiel 11

A. Eine Lösung von 14,5 g D-Glucamin in 120 ml Wasser wurde mit einer Lösung von 11,4 g Naphtalin-1,5-disulfonylchlorid in 300 ml Ethylacetat überschichtet. Zu der heftig gerührten Mischung wurden während 4 Stunden 80 ml einer 1,0 N Natriumhydroxidlösung zugetropft. Nach 20 Stunden Rühren bei Raumtemperatur wurde die wässrige Phase separiert, einmal mit 100 ml Ethylacetat gewaschen und am Rotationsverdampfer bei 50°C eingeengt. Der viskose Rückstand wurde dreimal mit je 50 ml Toluol azeotrop eingedampft, anschliessend in einer Lösung von 300 ml Pyridin und 100 ml Acetanhydrid aufgenommen und 8 h zum Rückfluss erhitzt. Das Reaktionsgemisch wurde wiederum zur Trockne eingeengt und zwischen Wasser und Ethylacetat verteilt. Die organische Phase wurde mit Wasser und Natriumhydrogencarbonatlösung gewaschen, über Magnesiumsulfat getrocknet und einrotiert. Der Rückstand wurde an Kieselgel in Hexan/Ethylacetat als Eluent chromatographiert. 5.6 g des gereinigten Produktes wurden in 100 ml Methanol gelöst und mit 2 mg Natrium versetzt. Nach 16 h Rühren wurde vom unlöslichen Niederschlag abfiltriert. Dieser wurde bei 50°C im Hochvakuum getrocknet und ergab Naphtalin-1,5-disulfonsäure-bis-D-glucit-1-ylamid, MS: *m/z* 615 ($[M+H]^+$); 637 ($[M+Na]^+$).

B. Eine Lösung von 2.78 g Naphtalin-1,5-disulfonsäure-bis-D-glucit-1-ylamid und 8,8 g Schwefeltrioxidtrimethylaminkomplex in 75 ml absolutem Dimethylformamid wurden 48 h bei 80°C gerührt. Die Reaktionslösung wurde bei 50 - 60°C eingedampft und der Rückstand in wenig Wasser gelöst. 6.5 g Natriumacetat wurde hinzugefügt und anschliessend erneut zur Trockne eingedampft. Das Abdampfen von je 5 ml Wasser wurde dreimal wiederholt. Gelfiltration des in Wasser gelösten Rohproduktes an Sephadex® SP 25 und Sephadex® LH 20 lieferte Naphtalin-1,5-disulfon-säure-bis-(2,3,4,5,6-penta-O-sulfo-D-glucit-1-ylamid) Decanatriumsalz, MS: *m/z* 1635 (rekonstruiertes M).

Beispiel 12

A. Zu 11,6 g 2,3:4,5-Di-O-isopropyliden-D-arabinitol, 7,23 g 4-Nitrophenol und 16,4 g Triphenylphosphin in 450 mi absolutem Tetrahydrofuran wurde unter Argon und Rühren eine Lösung von 8,94 ml Diäthylazodicarboxylat in 50 ml Tetrahydrofuran bei maximal 10°C zugetropft. Der Ansatz rührte während 18 Stunden bei Raumtemperatur. Nach dem Abdestillieren des Lösungsmittels im Wasserstrahlvakuum wurde der Rückstand an Kieselgel mit Hexan/Methylenchlorid chromatographiert. Man erhielt so 2,3:4,5-Di-O-isopropyliden-1-O-(4-nitrophenyl)-D-arabinitol, welches aus Hexan umkristallisiert wurde. Smp.: 63-65°C.

B. 9,29 g 2,3:4,5-Di-O-isopropyliden-1-O-(4-nitrophenyl)-D-arabinitol wurden durch katalytische Hydrierung mit 2,1 g Pd-Kohle (10%) in Methanol reduziert. Nach dem Abfiltrieren des Katalysators und Abdestillieren des Lösungsmittels im Wasserstrahlvakuum wurde 1-O-(4-Amino-phenyl)-2,3:4,5-di-Oisopropyliden-D-arabinitol erhalten, MS: *m/z* 323 ($M^+$).

C. 1,36 g 1-O-(4-Amino-phenyl)-2,3:4,5-di-O-isopropyliden-D-arabinitol wurden in 30 ml Methylenchlorid mit 0,42 g Isophthaloyldichlorid unter Zusatz von 1,38 ml Triethylamin bei 5°C unter Argon umgesetzt; der Ansatz rührte bei Raumtemperatur während 60 Stunden. Nach dem Abdestillieren des Lösungsmittels im Wasserstrahlvakuum wurde das Rohprodukt aus Methylenchlorid/Hexan umkristallisiert. Man erhielt N,N'-Bis-[4-(2,3:4,5-di-O-isopropyliden-D-arabinit-1-yloxy)-phenyl]-isophthalamid, MS: *m/z* 777,4 ($[M+H]^+$).

D. 1,30 g N,N'-Bis-[4-(2,3:4,5-di-O-isopropyliden-D-arabinit-1-yloxy)-phenyl]-isophthalamid wurden in 20 ml Dioxan mit 1,4 ml Trifluoressigsäure unter Zusatz von 2,8 ml dest. Wasser während 3 Stunden unter Rückfluss gerührt; es wurden 2 x 50 ml Toluol zugesetzt und jeweils im Wasserstrahlvakuum eingeengt. Der so erhaltene Rückstand wurde während 4 Stunden im Hochvakuum bei Raumtemperatur über Phosphorpentoxid getrocknet. Man erhielt N,N'-Bis-(4-D-arabinit-1-yloxy-phenyl)-isophthalamid, welches direkt in die nächste Stufe eingesetzt wurde.

E. 0,7 g N,N'-Bis-[4-D-arabinit-1-yloxy-phenyl)-isophthalamid wurden in Analogie zu Beispiel 1.B. mit Schewfeltrioxidtrimethylaminkomplex umgesetzt. Dabei wurde N,N'-Bis-[4-(2,3,4,5-tetra-O-sulfo-D-arabinit-1-yloxy)-phenyl]-isophthalamid Octanatriumsalz erhalten, MS: *m/z* 1433 (rekonstruiertes M).

Beispiel 13

A. Umsetzung von Terephthalsäure und D-Glucamin wie unter 1.A. beschrieben ergab N,N'-Bis-D-glucit-1-yl-terephthalamid, $[\alpha]^{20}_{D}$ -10,0° (*c* 0,5; Dimethylsulfoxid), MS: *m/z* 491,7 ($[M-H]^-$).

B. Sulfatierung von N,N'-Bis-D-glucit-1-yl-terephthalamid wie unter 1.B. beschrieben ergab N,N'-Bis-(2,3,4,5,6-penta-O-sulfo-D-glucit-1-yl)-terephthalamid Decanatriumsalz, $[\alpha]_D^{20}$ -9,0°(*c* 0,5; Wasser).

Beispiel 14

A. Umsetzung von Isophthalsäure und D-Glucamin wie unter 1.A. beschrieben ergab N,N'-Bis-D-glucit-1-yl-isophthalamid, $[\alpha]_D^{20}$ -12,5°(*c* 0,4; Dimethylsulfoxid), MS: *m/z* 493,4 ($[M+H]^+$).

B. Sulfatierung von N,N'-Bis-D-glucit-1-yl-isophthalamid wie unter 2.B. beschrieben ergab N,N'-Bis-(2,3,4,5,6-penta-O-sulfo-D-glucit-1-yl)-isophthalamid Decanatriumsalz, $[\alpha]_D^{20}$ -9,5°(*c* 0,4; Wasser), MS: *m/z* 1512,5 (rekonstruiertes M).

Beispiel 15

A. Umsetzung von Benzophenon-4,4'-dicarbonsäure und D-Glucamin wie unter 1.A. beschrieben ergab 4,4'-Carbonyl-dibenzoesäure-bis-D-glucit-1-ylamid, $[\alpha]_D^{20}$ -7,0°(*c* 0,5; Dimethylsulfoxid), MS: *m/z* 597,4 ($[M+H]^+$).

B. Sulfatierung von 4,4'-Carbonyl-dibenzoesäure-bis-D-glucit-1-ylamid wie unter 1.B. beschrieben ergab 4,4'-Carbonyl-dibenzoesäure-bis-(2,3,4,5,6-penta-O-sulfo-D-glucit-1-ylamid) Decanatriumsalz, $[\alpha]_D^{20}$ -7,8° (*c* 0,5; Wasser).

Beispiel 16

A. Umsetzung von Diphenylmethan-4,4'-dicarbonsäure und D-Glucamin wie unter 1.A. beschrieben ergab 4,4'-Methylen-dibenzoesäure-bis-D-glucit-1-ylamid, $[\alpha]_D^{20}$ -6,4° (*c* 0,5; Dimethylsulfoxid), MS: *m/z* 583,4 ($[M+H]^+$).

B. Sulfatierung von 4,4'-Methylen-dibenzoesäure-bis-D-glucit-1-ylamid wie unter 1.B. beschrieben ergab 4,4'-Methylen-dibenzoesäure-bis-(2,3,4,5,6-penta-O-sulfo-D-glucit-1-ylamid) Decanatriumsalz, $[\alpha]_D^{20}$ -6,2° (*c* 0,5; Wasser), MS: *m/z* 1602,5 (rekonstruiertes M).

Beispiel 17

A. Umsetzung von 4,4'-Oxy-dibenzoesäure und D-Glucamin wie unter 1.A. beschrieben ergab 4,4'-Oxy-dibenzoesäure-bis-D-glucit-1-ylamid, $[\alpha]_D^{20}$ - 5,8°(*c* 0,5; Dimethylsulfoxid), MS: *m/z* 585,2 ($[M+H]^+$).

B. Sulfatierung von 4,4'-Oxy-dibenzoesäure-bis-D-glucit-1-ylamid wie unter 2.B. beschrieben ergab 4,4'-Oxy-dibenzoesäure-bis-(2,3,4,5,6-penta-O-sulfo-D-glucit-1-ylamid) Decanatriumsalz, $[\alpha]_D^{20}$ -5,2°(*c* 0,5; Wasser), MS: *m/z* 1604,5 (rekonstruiertes M).

Beispiel 18

A. Umsetzung von 4,4'-(Hexafluoro-propan-2,2-diyl)-dibenzoesäure und D-Glucamin wie unter 1.A. beschrieben ergab 4,4'-(Hexafluoro-propan-2,2-diyl)-dibenzoesäure-bis-D-glucit-1-ylamid, $[\alpha]_D^{20}$ -5,6°(*c* 0,5; Dimethylsulfoxid), MS: *m/z* 719,4 ($[M+H]^+$).

B. Sulfatierung von 4,4'-(Hexafluoro-propan-2,2-diyl)-dibenzoesäure-bis-D-glucit-1-ylamid wie unter 1.B. beschrieben ergab 4,4'-(Hexafluoro-propan-2,2-diyl)-dibenzoesäure-bis-(2,3,4,5,6-penta-O-sulfo-D-glucit-1-ylamid) Decanatriumsalz, $[\alpha]_D^{20}$ -4,6°(*c* 0,5; Wasser), MS: *m/z* 1738,5 (rekonstruiertes M).

Beispiel 19

A. Umsetzung von Biphenyl-4,4'-dicarbonsäure und D-Glucamin wie unter 1.A. beschrieben ergab Biphenyl-4,4'-dicarbonsäure-bis-D-glucit-1-ylamid, $[\alpha]_D^{20}$ -4,6°(*c* 0,2; Dimethylsulfoxid), MS: *m/z* 569,2 ($[M+H]^+$).

B. Sulfatierung von Biphenyl-4,4'-dicarbonsäure-bis-D-glucit-1-ylamid wie unter 2.B. beschrieben ergab Biphenyl-4,4'-dicarbonsäure-bis-(2,3,4,5,6-penta-O-sulfo-D-glucit-1-ylamid) Decanatriumsalz, $[\alpha]_D^{20}$ -6,4°(*c* 0,5; Wasser), MS: *m/z* 1588,5 (rekonstruiertes M).

Beispiel 20

A. Umsetzung von Naphthalin-1,4-dicarbonsäure und D-Glucamin wie unter 1.A. beschrieben ergab Naphthalin-1,4-dicarbonsäure-bis-D-glucit-1-ylamid, $[\alpha]_D^{20}$ -8,6°(*c* 0,5; Dimethylsulfoxid), MS: *m/z* 963,4 ($[M+H]^+$).

B. Sulfatierung von Naphthalin-1,4-dicarbonsäure-bis-D-glucit-1-ylamid wie unter 2.B. beschrieben ergab Naphthalin-1,4-dicarbonsäure-bis-(2,3,4,5,6-penta-O-sulfo-D-glucit-1-ylamid) Decanatriumsalz, $[\alpha]_D^{20}$ -14,6°(*c* 0,5; Wasser), MS: *m/z* 1562,5 (rekonstruiertes M).

Beispiel 21

A. Umsetzung von (4-Carboxy-phenoxy)-essigsäure und D-Glucamin wie unter 1.A. beschrieben ergab N-D-Glucit-1-yl-4-(D-glucit-1-ylcarbamoyl-methoxy)-benzamid, $[\alpha]_D^{20}$ -10,8°(*c* 0,5; Dimethylsulfoxid), MS: *m/z* 523,4 ($[M+H]^+$).

B. Sulfatierung von N-D-Glucit-1-yl-4-(D-glucit-1-ylcarbamoylmethoxy)-benzamid wie unter 2.B. beschrieben ergab N-(2,3,4,5,6-Penta-O-sulfo-D-glucit-1-yl)-4-(2,3,4,5,6-penta-O-sulfo-D-glucit-1-ylcarbamoylmethoxy)-benzamid Decanatriumsalz, $[\alpha]_D^{20}$ -14,6°(*c* 0,5; Wasser), MS: *m/z* 1562,5 (rekonstruiertes M).

Beispiel 22

A. Umsetzung von 9-Oxo-9H-fluoren-2,7-dicarbonsäure und D-Glucamin wie unter 3.A. beschrieben ergab 9-Oxo-9H-fluoren-2,7-dicarbonsäure-bis-D-glucit-1-ylamid, $[\alpha]_D^{20}$ -2,5°(*c* 0,2; Dimethylsulfoxid), MS: *m/z* 595,4 ($[M+H]^+$).

B. Sulfatierung von 9-Oxo-9H-fluoren-2,7-dicarbonsäure-bis-D-glucit-1-ylamid wie unter 2.B. beschrieben ergab 9-Oxo-9H-fluoren-2,7-dicarbonsäure-bis-(2,3,4,5,6-penta-O-sulfo-D-glucit-1-ylamid) Decanatriumsalz, $[\alpha]_D^{20}$ -7,4° (*c* 0,5; Wasser), MS: *m/z* 1614,5 (rekonstruiertes M).

Beispiel 23

A. Umsetzung von 1,1';4',1''-Terphenyl-4,4''-dicarbonsäure und D-Glucamin wie unter 3.A. beschrieben ergab 1,1'; 4',1''-Terphenyl-4,4''-dicarbonsäure-bis-D-glucit-1-ylamid, MS: *m/z* 645,6 ($[M+H]^+$).

B. Sulfatierung von 1,1';4,1''-Terphenyl-4,4''-dicarbonsäure-bis-D-glucit-1-ylamid wie unter 2.B. beschrieben ergab 1,1';4',1''-Terphenyl-4,4''-dicarbon-säure-bis-(2,3,4,5,6-penta-O-sulfo-D-glucit-1-ylamid) Decanatriumsalz, $[\alpha]_D^{20}$ -5,6° (*c* 0,5; Wasser), MS: *m/z* 1665,0 (rekonstruiertes M).

Beispiel 24

A. Analog Beispiel 6.A. wurde aus (2E,4E,6E)-2,7-Dimethyl-octa-2,4,6-triendicarbonsäure und D-Glucamin (2E, 4E,6E)-2,7-Dimethyl-octa-2,4,6-triendicarbonsäure-bis-D-glucit-1-ylamid als weisses Pulver erhalten, MS: *m/z* 523,4 ($[M+H]^+$).

B. Das oben erhaltene (2E,4E,6E)-2,7-Dimethyl-octa-2,4,6-triendicarbonsäure-bis-D-glucit-1-ylamid wurde analog Beispiel 1.B. zu (2E,4E,6E)-2,7-Dimethyl-octa-2,4,6-triendicarbonsäure-bis-(2,3,4,5,6-penta-O-sulfo-D-glucit-1-ylamid) Decanatriumsalz, $[\alpha]_D^{20}$ - 6,3° (*c* 0,7; Wasser), umgesetzt.

Beispiel 25

A. 1,51 g 4-(Aminomethyl)-benzoesäure und 2,76 g Kaliumcarbonat wurden in 40 ml Wasser durch Erwärmen gelöst. Hierauf wurden 1,72 g 4,4'-Sulfonyl-dibenzoylchlorid aufgeschlämmt in 40 ml Toluol hinzugefügt. Das Reaktionsgemisch wurde bei Raumtemperatur während 18 Stunden intensiv gerührt und anschliessend unter vermindertem Druck zur Trockne eingedampft, in Wasser aufgekocht und durch einen 0.8 μ Cellulosefilter filtriert. Der Filterrückstand wurde hierauf in verdünnter Salzsäure erneut aufgekocht und noch einmal über einen 0.8 μ Cellulosefilter filtriert. Dabei wurde 4,4'-Sulfonyl-dibenzoesäure-bis-(4-carbonsäure-benzylamid) als farblose Kristalle, MS: *m/z* 571,3 ($[M-H]^-$), erhalten.

B. Analog Beispiel 6.A. wurde aus 4,4'-Sulfonyl-dibenzoesäure-bis-(4-carbonsäure-benzylamid) und D-Glucamin 4,4'-Sulfonyl-dibenzoesäure-bis-(4-D-glucit-1-ylcarbamoyl-benzylamid) als weisses Pulver erhalten, $[\alpha]_D^{20}$ -4,0°(*c*

0,4; Dimethylsulfoxid), MS: *m/z* 899,4 ($[M+H]^+$).

C. Das oben erhaltene 4,4'-Sulfonyl-dibenzoesäure-bis-(4-D-glucit-1-ylcarbamoyl-benzylamid) wurde analog Beispiel 1.B. zu 4,4'-Sulfonyl-dibenzoesäure-bis-[4-(2,3,4,5,6-penta-O-sulfo-D-glucit-1-ylcarbamoyl)-benzylamid] Decanatriumsalz , $[\alpha]_D^{20}$ - 3,6° (*c* 0,5; Wasser), MS: *m/z* 1920,0 (rekonstruiertes M), umgesetzt.

Beispiel 26

A. Umsetzung von Toluol-2,6-diyl-diisocyanat und D-Glucamin wie unter 8.A. beschrieben ergab 3,3'-Di-D-glucit-1-yl-1,1'-(toluol-2,6-diyl)-diharnstoff, $[\alpha]_D^{20}$ -8,4° (*c* 0,5; Dimethylsulfoxid), MS: *m/z* 537,5 ($[M+H]^+$).

B. Sulfatierung von 3,3'-Di-D-glucit-1-yl-1,1'-(toluol-2,6-diyl)-diharnstoff wie unter 2.B. beschrieben ergab 3,3'-Bis-(2,3,4,5,6-penta-O-sulfo-D-glucit-1-yl)-1,1'-(toluol-2,6-diyl)-diharnstoff Decanatriumsalz, $[\alpha]_D^{20}$ -1,6° (*c* 0,5; Wasser), MS: *m/z* 1556,5 (rekonstruiertes M).

Beispiel 27

A. Umsetzung von (Benzol-1,3-diyl-dimethylen)-diisocyanat und D-Glucamin wie unter 8.A. beschrieben ergab 3,3'-Di-D-glucit-1-yl-1,1'-(benzol-1,3-diyl-dimethylen)-diharnstoff, $[\alpha]_D^{20}$ -4,0° (*c* 0,5; Dimethylsulfoxid), MS: *m/z* 551,6 ($[M+H]^+$).

B. Sulfatierung von 3,3'-Di-D-glucit-1-yl-1,1'-(benzol-1,3-diyl-dimethylen)-diharnstoff wie unter 2.B. beschrieben ergab 3,3'-Bis-(2,3,4,5,6-penta-O-sulfo-D-glucit-1-yl)-1,1'-(benzol-1,3-diyl-dimethylen)-diharnstoff Decanatriumsalz, $[\alpha]_D^{20}$ -1,6° (*c* 0,5; Wasser), MS: *m/z* 1556,5 (rekonstruiertes M).

Beispiel 28

A. Umsetzung von 2,2'-Dichloro-4,4'-methylen-bis-phenylisocyanat und D-Glucamin wie unter 8.A. beschrieben ergab 3,3'-D-Glucit-1-yl-1,1'-(3,3'-dichloro-4,4'-methylen-diphenyl)-diharnstoff, $[\alpha]_D^{20}$ -7,5° (*c* 0,5; Dimethylsulfoxid), MS: *m/z* 681,4 ($[M+H]^+$).

B. Sulfatierung von 3,3'-D-Glucit-1-yl-1,1'-(3,3'-dichloro-4,4'-methylen-diphenyl)-diharnstoff wie unter 2.B. beschrieben ergab 3,3'-Bis-(2,3,4,5,6-penta-O-sulfo-D-glucit-1-yl)-1,1'-(3,3'-dichloro-4,4'-methylen-diphenyl)-diharnstoff Decanatriumsalz, $[\alpha]_D^{20}$ +0,80 (*c* 0,5; Wasser), MS: *m/z* 1702,0 (rekonstruiertes M).

Beispiel 29

A. Umsetzung von 4,4'-(3,3'-Dimethyl-biphenyl-diyl)-diisocyanat und D-Glucamin wie unter 8.A. beschrieben ergab 3,3'-D-Glucit-1-yl-1,1'-(3,3'-dimethyl-biphenyl-4,4'-diyl)-diharnstoff, $[\alpha]_D^{20}$ -15,5° (*c* 02; Dimethylsulfoxid), MS: *m/z* 627,3 ($[M+H]^+$).

B. Sulfatierung von 3,3'-D-Glucit-1-yl-1,1'-(3,3'-dimethyl-biphenyl-4,4'-diyl)-diharnstoff wie unter 2.B. beschrieben ergab 3,3'-Bis-(2,3,4,5,6-penta-O-sulfo-D-glucit-1-yl)-1,1'-(3,3'-dimethyl-biphenyl-4,4'-diyl)-diharnstoff Decanatriumsalz, $[\alpha]_D^{20}$ +1,0° (*c* 0,5; Wasser), MS: *m/z* 1647,0 (rekonstruiertes M).

Beispiel 30

A. Umsetzung von (3,3'-Dimethoxy-biphenyl-4,4'-diyl)-diisocyanat und D-Glucamin wie unter 8.A. beschrieben ergab 3,3'-D-Glucit-1-yl-1,1'-(3,3'-dimethoxy-biphenyl-4,4'-diyl)-diharnstoff, $[\alpha]_D^{20}$ -10,0° (*c* 0,2; Dimethylsulfoxid), MS: *m/z* 659,3 ($[M+H]^+$).

B. Sulfatierung von 3,3'-D-Glucit-1-yl-1,1'-(3,3'-dimethoxy-biphenyl-4,4'-diyl)-diharnstoff wie unter 2.B. beschrieben ergab 3,3'-Bis-(2,3,4,5,6-penta-O-sulfo-D-glucit-1-yl)-1,1'-(3,3'-dimethoxy-biphenyl-4,4'-diyl)-diharnstoff Decanatriumsalz, $[\alpha]_D^{20}$ +1,6° (*c* 0,5; Wasser).

Beispiel 31

A. Umsetzung von 4,4'-(3,3'-Dichlor-biphenyl-diyl)-diisocyanat und D-Glucamin wie unter 8.A. beschrieben ergab

3,3'-D-Glucit-1-yl-1,1'-(3,3'-dichlor-biphenyl-4,4'-diyl)-diharnstoff, MS: *m/z* 667,4 ([M+H]$^+$).

B. Sulfatierung von 3,3'-D-Glucit-1-yl-1,1'-(3,3'-dichlor-biphenyl-4,4'-diyl)-diharnstoff wie unter 2.B. beschrieben ergab 3,3'-Bis-(2,3,4,5,6-penta-O-sulfo-D-glucit-1-yl)-1,1'-(3,3'-dichlor-biphenyl-4,4'-diyl)-diharnstoff Decanatrium-salz, $[\alpha]_D^{20}$ +4,6° (*c* 0,5; Wasser).

Beispiel 32

A. Umsetzung von 4,4'-Diphenylmethan-diisocyanat und D-Glucamin wie unter 8.A. beschrieben ergab 3,3'-Di-D-glucit-1-yl-1,1'-(4,4'-methylen-diphenyl)-diharnstoff, $[\alpha]_D^{20}$ -13,2° (*c* 0,2; Dimethylsulfoxid), MS: *m/z* 613,4 ([M+H]$^+$).

B. Sulfatierung von 3,3'-Di-D-glucit-1-yl-1,1'-(4,4'-methylen-diphenyl)-diharnstoff wie unter 2.B. beschrieben ergab 1,1'-(4,4'-Methylen-diphenyl)-3,3'-bis-(2,3,4,5,6-penta-O-sulfo-D-glucit-1-yl)-diharnstoff Decanatriumsalz, $[\alpha]_D^{20}$ -0,8° (*c* 0,5; Wasser).

Beispiel 33

A. Umsetzung von 1-(4-Aminophenyl)-3-(2,3,4,5-penta-O-acetyl-D-glucit-1-yl)-harnstoff und Terephthaloyldichlorid wie unter 7.C. beschrieben ergab N,N'-Bis-[4-[3-(D-glucit-1-yl)-ureido]-phenyl]-terephthalamid.

B. Sulfatierung von N,N'-Bis-[4-[3-(D-glucit-1-yl)-ureido]-phenyl]-isophthalamid wie unter 2.B. beschrieben ergab N,N'-Bis-[4-[3-(2,3,4,5,6-penta-O-sulfo-D-glucit-1-yl)-ureido]-phenyl]-isophthalamid Decanatriumsalz, $[\alpha]_D^{20}$ 0,0° (*c* 0,2; Wasser), MS: *m/z* 1781,0 (rekonstruiertes M).

Beispiel 34

A. Umsetzung von 1-(4-Aminophenyl)-3-(2,3,4,5,6-penta-O-acetyl-D-glucit-1-yl)-harnstoff und Fumarsäuredichlorid wie unter 7.C. beschrieben ergab N,N'-Bis-[4-[3-(D-glucit-1-yl)-ureido]-phenyl]-fumaramid, $[\alpha]_D^{20}$ -12,5°(*c* 0,2; Dimethylsulfoxid), MS: *m/z* 711,4 ([M+H]$^+$).

B. Sulfatierung von N,N'-Bis-[4-[3-(D-glucit-1-yl)-ureido]-phenyl]-fumaramid wie unter 2.B. beschrieben ergab N, N'-Bis-[4-[3-(2,3,4,5,6-penta-O-sulfo-D-glucit-1-yl)-ureido]-phenyl]-fumaramid Decanatriumsalz, $[\alpha]_D^{20}$ +1,0° (*c* 0,5; Wasser), MS: *m/z* 1731,0 (rekonstruiertes M).

Beispiel 35

A. Umsetzung von 4,4'-Diisocyanato-E-stilben-2,2'-disulfonsäure Dinatriumsalz und D-Glucamin wie unter 9.A. beschrieben ergab (E)-4,4'-Bis-(3-D-glucit-1-yl-thioureido)-stilben-2,2'-disulfonsäure Dinatriumsalz, $[\alpha]_D^{20}$ -16,4°(*c* 0,2; Dimethylsalfoxid), MS: *m/z* 860,0 (M$^+$).

B. Sulfatierung von (E)-4,4'-Bis-(3-D-glucit-1-yl-thiuoureido)-stilben-2,2'-disulfonsäure Dinatriumsalz wie unter 2.B. beschrieben ergab (E)-4,4'-Bis-[3-(2,3,4,5,6-penta-O-sulfo-D-glucit-1-yl)-thioureido]-stilben-2,2'-disulfonsäure Dodecanatriumsalz, $[\alpha]_D^{20}$ +2,0°(*c* 0,5; Wasser), MS: *m/z* 1880,5 (rekonstruiertes M).

Beispiel 36

A. Zu einer Lösung von 1,92 g N-Methyl-D-glucamin und 1,36 g Kaliumcarbonat in 15 ml Wasser wurde 1 g Terephthalsäuredichlorid, aufgeschlämmt in 15 ml Toluol, gegeben. Das Reaktionsgemisch wurde 20 Stunden bei Raumtemperatur intensiv gerührt, anschliessend unter vermindertem Druck eingeengt und dann an LiChroprep RP- 18 Kieselgel mit Wasser und anschliessend mit Wasser mit ansteigendem Anteil an Methanol chromatographiert. Einengen der relevanten Fraktionen und Trocknen am Vakuum ergab N,N'-Di-glucit-1-yl-N,N'-dimethyl-terephthalamid als farblose Kristalle, $[\alpha]_D^{20}$ -7,4° (*c* 0,5; Dimethylsulfoxid), MS: *m/z* 521,2 ([M+H]$^+$).

B. Das oben erhaltene N,N'-Di-glucit-1-yl-N,N'-dimethyl-terephthalamid wurde analog Beispiel 1.B. zu N,N'-Dimethyl-N,N'-bis-(2,3,4,5,6-penta-O-sulfo-D-glucit-1-yl)-terephtalamid Decanatriumsalz , $[\alpha]_D^{20}$ -14,2° (*c* 0,5; Wasser), MS: *m/z* 1541,0 (rekonstruiertes M), umgesetzt.

Beispiel 37

A. Analog Bsp. 5.B. wurde aus 9-Oxo-9H-fluoren-2,7-dicarbonsäure und N-Methyl-D-glucamin 9-Oxo-9H-fluoren-2,7-dicarbonsäure bis-[(D-glucit-1-yl)-methyl-amid] als gelbliches Pulver erhalten, $[\alpha]_D^{20}$ -2° (*c* 0,4; Dimethylsulfoxid), MS 623,4 ($[M+H]^+$).

B. Das oben erhaltene 9-Oxo-9H-fluoren-2,7-dicarbonsäure bis-[(D-glucit-1-yl)-methyl-amid] wurde analog Beispiel 1.B. zu 9-Oxo-9H-fluoren-2,7-dicarbonsäure bis-[methyl-(2,3,4,5,6-penta-O-sulfo-D-glucit-1-yl)-amid] Decanatriumsalz , $[\alpha]_D^{20}$ +2,2° (*c* 0,5; Wasser), umgesetzt.

Beispiel 38

A. Umsetzung von Toluol-2,6-diyl-diisocyanat und N-Methyl-D-glucamin wie unter 8.A. beschrieben ergab 3,3'-Di-D-glucit-1-yl-3,3'-dimethyl-1,1'-(toluol-2,6-diyl)-diharnstoff, $[\alpha]_D^{20}$ +35,5° (*c* 0,2; Dimethylsulfoxid), MS: *m/z* 565,4 ($[M+H]^+$).

B. Sulfatierung von 3,3'-Di-D-glucit-1-yl-3,3'-dimethyl-1,1'-(toluol-2,6-diyl)-diharnstoff wie unter 2.B. beschrieben ergab 3,3'-Dimethyl-3,3'-bis-(2,3,4,5,6-penta-O-sulfo-D-glucit-1-yl)-1,1'-(toluol-2,6-diyl)-diharnstoff Decanatriumsalz, $[\alpha]_D^{20}$ -1,6° (*c* 0,5; Wasser), MS: *m/z* 1584,5 (rekonstruiertes M).

Beispiel 39

A. Eine Lösung von 7,25 g D-Glucamin in 22 ml 1,0 N Natriumhydroxid wurde mit einer Lösung von 3,66 g Biphenyl-4,4'-disulfonylchlorid in 100 ml Tetrahydrofran versetzt. Nach 56 Stunden Rühren bei Raumtemperatur wurde die wässrige Phase separiert und am Rotationsverdampfer bei 50°C eingeengt. Der Rückstand wurde dreimal mit je 50 ml Toluol azeotrop eingedampft, anschliessend mit Acetanhydrid in Pyridin acyliert. Die Aufarbeitung und Reinigung erfolgte wie unter 11.A. beschrieben und lieferte Biphenyl-4,4'-disulfonsäure-bis-D-glucit-1-ylamid, MS: *m/z* 640 ($[M-H]^-$).

B. Sulfatierung von Biphenyl-4,4'-disulfonsäure-bis-D-glucit-1-ylamid wie unter 11.B. beschrieben lieferte Biphenyl-4,4'-disulfonsäure-bis-(2,3,4,5,6-penta-O-sulfo-D-glucit-1-ylamid) Decanatriumsalz, MS: *m/z* 1661 (rekonstruiertes M).

Beispiel 40

A. Umsetzung von 4,4'-Oxy-bis-(benzolsulfonylchlorid) mit D-Glucamin wie unter 11.A. beschrieben ergab 4,4'-Oxy-dibenzolsulfonsäure-bis-D-glucit-1-ylamid, MS: *m/z* 657 ($[M+H]^+$).

B. Sulfatierung von 4,4'-Oxy-dibenzolsulfonsäure-bis-D-glucit-1-ylamid wie unter 11.B. beschrieben lieferte 4,4'-Oxy-dibenzolsulfonsäure-bis-(2,3,4,5,6-penta-O-sulfo-D-glucit-1-ylamid) Decanatriumsalz, $[\alpha]_D^{20}$ +4,4°(*c* 1,0; Wasser).

Beispiel 41

A. 0,68 g 1-O-(4-Amino-phenyl)-2,3:4,5-di-O-isopropyliden-D-arabinitol wurden in 15 ml Methylenchlorid mit 0,21 g Terephthaloyldichlorid unter Zusatz von 0,69 ml Triethylamin bei 5°C unter Argon umgesetzt; der Ansatz rührte bei Raumtemperatur während 60 Stunden. Nach dem Abdestillieren des Lösungsmittels im Wasserstrahlvakuum wurde das Rohprodukt an Kieselgel mit Methylenchlorid/ Methanol chromatographiert. Man erhielt N,N'-Bis-[4-(2,3:4,5-di-O-isopropyliden-D-arabinit-1-yloxy)-phenyl]-terephthalamid, MS: *m/z* 778,4 $(M+H)^+$.

B. 0,77 g N,N'-Bis-[4-(2,3:4,5-di-O-isopropyliden-D-arabinit-1-yloxy)-phenyl]-terephthalamid wurden in 15 ml Dioxan mit 1,0 ml Trifluoressigsäure unter Zusatz von 2 ml $H_2O$ dest. während 18 Stunden bei 50°C gerührt; es wurden 2 x 50 ml Toluol zugesetzt, jeweils im Wasserstrahlvakuum eingeengt und der Rückstand im Hochvakuum bei 50°C über Phosphorpentoxid während 3 Stunden getrocknet. Man erhielt N,N'-Bis-(4-D-arabinit-1yloxy-phenyl)-terephthalamid, welches direkt in die nächste Stufe eingesetzt wurde.

C. 0,3 g N,N'-Bis-(4-D-arabinit-1yloxy-phenyl)-terephthalamid wurden in Analogie zu Beispiel 1.B. mit Schwefeltrioxidtrimethylaminkomplex umgesetzt. Dabei wurde N,N'-Bis-[4-(2,3,4,5-tetra-O-sulfo-D-arabinit-1-yloxy)-phenyl]-terephthalamid Octanatriumsalz erhalten, MS: *m/z* 1432 (rekonstruiertes M).

Beispiel 42

A. 0,65 g 1-O-(4-Amino-phenyl)-2,3:4,5-di-O-isopropyliden-D-arabinitol und 0,16 g N,N-Carbonyl-diimidazol wurden in 15 ml Tetrahydrofuran unter Argon während 16 Stunden bei Raumtemperatur gerührt. Das Reaktionsgemisch wurde eingedampft; der Rückstand wurde mit Eiswasser versetzt, mit Ether extrahiert, die vereinigten Etherphasen mit verd. Salzsäure und Wasser gewaschen, über Magnesiumsulfat getrocknet, filtriert und eingedampft. Man erhielt 1,3-Bis-[4-(2,3:4,5-di-O-isopropyliden-D-arabinit-1-yloxy)-phenyl]-harnstoff, welcher aus Methylenchlorid/n-Hexan umkristallisiert wurde, MS: *m/ z* 673,4 ($[M+H]^+$).

B. 0,40 g 1,3-Bis-[4-(2,3:4,5-di-O-isopropyliden-D-arabinit-1-yloxy)-phenyl]-harnstoff wurden in Analogie zu Beispiel 12.D. mit Trifluoressigsäure (50%ig) in 15 ml Dioxan während 4 Stunden unter Rückfluss gerührt und analog aufgearbeitet. Man erhielt 1,3-Bis-(4-D-arabinit-1yloxy-phenyl)-harnstoff, welcher direkt in die nächste Stufe eingesetzt wurde.

C. 0,37 g 1,3-Bis-(4-D-arabinit-1yloxy-phenyl)-harnstoff wurden in Analogie zu Beispiel 1.B. mit Schwefeltrioxid-trimethylaminkomplex umgesetzt. Dabei wurde 1,3-Bis-[4-(2,3,4,5-tetra-O-sulfo-D-arabinit-1-yloxy)-phenyl]-harnstoff Octanatriumsalz erhalten, MS: *m/z* 1328 (rekonstruiertes M).

Beispiel 43

A. 0,995 g Terephthalsäure-monomethylesterchlorid und 0,756 g 4-(Amino-methyl)-benzoesäure methylester wurden in 25 ml Aceton gelöst, mit 0,691 g wasserfreiem Kaliumcarbonat versetzt. Das Reaktionsgemisch wurde während 30 Stunden bei Raumtemperatur gerührt. Hierauf wurde das Reaktionsgemisch eingedampft, der Rückstand mit Wasser verstetzt und die so gebildeten Kristalle abfiltriert. Dabei wurde N-(4-Methoxycarbonyl-phenyl)-N-methyl-terephthalmonoamidsäure-methylester in Form farbloser Kristalle erhalten, MS: *m/z* 327 ($M^+$).

B. 0,99 g N-(4-Methoxycarbonyl-phenyl)-N-methyl-terephthalmono-amidsäure-methylester wurden in 10 ml Acetonitril gelöst und mit 10 ml 1 N Natronlauge versetzt und das Reaktionsgemisch 16 Stunden bei Raumtemperatur gerührt. Anschliessend wurde mit 1 N Salzsäure angesäuert und der dabei gebildete Niederschlag abfiltriert. Die so erhaltene N-(4-Carboxyphenyl)-N-methyl-terephthalmonoamidsäure wurde direkt in die nächste Stufe eingesetzt.

C. Analog Beispiel 5.B. wurde aus N-(4-Carboxyphenyl)-N-methyl-terephthalmonoamidsäure und D-Glucamin N-(D-Glucit-1-yl)-N'-(4-D-glucit- 1-ylcarbamoyl-phenyl)-N'-methyl-terephthalamid als weisses Pulver erhalten, MS: *m/z* 626,4 ($[M+H]^+$).

D. Das oben erhaltene N-(D-Glucit-1-yl)-N'-(4-D-glucit-1-ylcarbamoyl-phenyl)-N'-methyl-terephthalamid wurde analog Beispiel 1.B. zu N'-Methyl-N-(2,3,4,5,6-penta-O-sulfo-D-glucit-1-yl)-N'-[4-(2,3,4,5,6-penta-O-sulfo-D-glucit-1-ylcarbamoyl)-phenyl]-terephthalamid Decanatriumsalz, $[\alpha]_D^{20}$ -5,7°, (*c* 0,6; Wasser), MS: *m/z* 1645,5 (rekonstruiertes M), umgesetzt.

Beispiel 44

A. Analog Beispiel 43.A.und 43.B. wurde aus Terephthalsäure-monomethylesterchlorid und 4-Aminobenzoesäure-methylester N-(4-Carboxy-phenyl)-terephthalmonoamidsäure als farbloses Pulver erhalten, MS: *m/z* 284,1 ($[M-H]^-$).

B. Analog Beispiel 6.A. wurde aus N-(4-Carboxy-phenyl)-terephthalmonoamidsäure und D-Glucamin N-D-Glucit-1-yl-N'-(4-D-glucit-1-ylcarbamoyl-phenyl)-terephthalamid als weisses Pulver erhalten, MS: *m/z* 612,4 ($[M+H]^+$).

C. Das oben erhaltene N-D-Glucit-1-yl-N'-(4-D-glucit-1-ylcarbamoyl-phenyl)-terephthalamid wurde analog Beispiel 1.B. zu N-(2,3,4,5,6-Penta-O-sulfo-D-glucit-1-yl)-N'-[4-(2,3,4,5,6-penta-O-sulfo-D-glucit-1-ylcarbamoyl)-phenyl]-terephthalamid Decanatriumsalz, $[\alpha]_D^{20}$ -6,1°, (*c* 0,7; Wasser), MS: *m/z* 1632,0 (rekonstruiertes M), umgesetzt.

Beispiel 45

A. Das (Z)-But-2-endicarbonsäure 3-carboxy-4-(4-chloro-phenoxy)-phenylamid wurde von der MAYBRIDGE CHEMICAL COMPANY LTD. erworben. Es kann auf folgende Weise hergestellt werden:

A.1. 2-(4-Chlorophenoxy)-5-nitro-benzoesäuremethylester wird durch Verwendung von Palladium/Kohle als Katalysator mit Hilfe von Wasserstoff in einem Lösungsmittel wie Methanol zu 5-Amino-2-(4-chlorophenoxy)-benzoesäuremethylester reduziert. Diese Reduktionsstufe kann auch durch Verwendung von Zinntetrachlorid in wässeriger Salzsäure oder durch Verwendung von Natriumsulfid in Methanol/Wasser bei jeweils erhöhter Temperatur durchgeführt werden entweder mit dem oben erwähnten Ester oder der freien 2-(4-Chlorophenoxy)-5-nitro benzoesäure zu 5-Amino-2-(4-chlorophenoxy)-benzoesäure , welche dann anschliessend durch Erwärmen in methanolischer Salzsäure in 5-Amino-2-(4-chlorophenoxy)- benzoesäure methylester überführt werden kann.

A.2. Die Umsetzung von 5-Amino-2-(4-chlorophenoxy)-benzoesäure methylester mit Maleinsäureanhydrid in einem Lösungsmittel wie Methylenchlorid oder Dimethylformamid in Gegenwart von Pyridin oder 4-Dimethylaminopyridin ergibt (Z)-But-2-endicarbonsäure 3-carbomethoxy-4-(4-chloro-phenoxy)-phenylamid, welches sich durch Verseifung mit wässeriger Natronlauge oder Kalilauge in Wasser und einem zweiten Lösungsmittel wie Methanol, Ethanol oder Acetonitril in (Z)-But-2-endicarbonsäure-3-carboxy-4-(4-chloro-phenoxy)-phenylamid überführen lässt, Schmelzpunkt: 224-226°C.

B. Analog Beispiel 5.B. wurde aus (Z)-But-2-endicarbonsäure-3-carboxy-4-(4-chloro-phenoxy)-phenylamid und D-Glucamin (Z)-But-2-endicarbonsäure 1-[4-(4-chloro-phenoxy)-3-D-glucit-1-ylcarbamoyl-phenyl]amid 4-D-glucit-1-ylamid als weisses Pulver erhalten, MS: *m/z* 688,4 ($[M+H]^+$).

C. Das oben erhaltene (Z)-But-2-endicarbonsäure 1-[4-(4-chloro-phenoxy)-3-D-glucit-1-ylcarbamoyl-phenyl]amid 4-D-glucit-1-ylamid wurde analog Beispiel 1.B. zu (Z)-But-2-endicarbonsäure 1-[4-(4-chloro-phenoxy)-3-(2,3,4,5,6-penta-O-sulfo-D-glucit-1-ylcarbamoyl)-phenyl]-amid 4-(2,3,4,5,6-penta-O-sulfo-D-glucit-1-yl)-amid Decanatriumsalz, $[\alpha]_D^{20}$ -4°, (*c* 0,5; Wasser), MS: *m/z* 1708,0 (rekonstruiertes M), umgesetzt.

Beispiel 46

A. Das 3'-Carboxy-4'-(4-chloro-phenoxy)-bernsteinsäuremonoanilid wurde von der MAYBRIDGE CHEMICAL COMPANY LTD. erworben. Es kann wie in Beispiel 45.A. beschrieben hergestellt werden, wobei Maleinsäureanhydrid durch Bernsteinsäureanhydrid ersetzt wird. So erhält man N-[3-Carboxy-4-(4-chloro-phenoxy)-phenyl]-bernsteinsäuremonoamid, Schmelzpunkt: 226-228°C.

B. Analog Beispiel 5.B. wurde aus N-[3-Carboxy-4-(4-chloro-phenoxy)-phenyl]-bernsteinsäuremonoamid und D-Glucamin N1-[4-(4-Chloro-phenoxy)-3-D-glucit-1-ylcarbamoyl-phenyl]-N4-D-glucit-1-yl-bernsteinsäureamid als weisses Pulver erhalten, MS: *m/z* 690,2 ($[M+H]^+$).

C. Das oben erhaltene N1-[4-(4-Chloro-phenoxy)-3-D-glucit-1-ylcarbamoyl-phenyl]-N4-D-glucit-1-yl-bernsteinsäureamid wurde analog Beispiel 1.B. zu N1-[4-(4-chloro-phenoxy)-3-(2,3,4,5,6-penta-O-sulfo-D-glucit-1-ylcarbamoyl)-phenyl]-N4-(2,3,4,5,6-penta-O-sulfo-D-glucit-1-yl)-bernsteinsäureamid Decanatriumsalz, $[\alpha]_D^{20}$ -4,6°, (*c* 0,5; Wasser), MS: *m/z* 1710,5 (rekonstruiertes M), umgesetzt.

Beispiel 47

A. Das (Z)-But-2-endicarbonsäure 3-carboxy-4-(2-methoxyethoxy)-phenylamid wurde von der MAYBRIDGE CHEMICAL COMPANY LTD. erworben. Es kann auf folgende Weise hergestellt werden:

A.1. 2-Hydroxy-5-nitro-benzoesäure methylester wird in Gegenwart einer Base wie Kaliumcarbonat, Natriumhydrid oder Natriummethylat in einem Lösungsmittel wie Methanol oder Dimethylformamid bei erhöhter Temperatur mit 1-Bromo-2-methoxyethan oder 2-Methoxyethyl-4-toluol-sulfonat zu 2-(2-Methoxyethoxy)-5-nitro-benzoesäure methylester umgesetzt. Dieser wird anschliessend wie in den Beispielen 45.A.1. und 45.A.2. beschrieben in (Z)-But-2-endicarbonsäure 3-carboxy-4-(2-methoxy-ethoxy)-phenylamid, Schmelzpunkt: 202-205°C, überführt.

B. Analog Beispiel 5.B. wurde aus (Z)-But-2-endicarbonsäure 3-carboxy-4-(2-methoxy-ethoxy)-phenylamid und D-Glucamin (Z)-But-2-endicarbonsäure 1-D-glucit-1-ylamid-4-[3-D-glucit-1-ylcarbamoyl-4-(2-methoxy-ethoxy)-phenyl]-amid als weisses Pulver erhalten, Elementaranalyse berechnet für $C_{26}H_{41}N_3O_{15}x1.1H_2O$: C=47.65%, H = 6.64%, N = 6.41%; gefunden: C = 47,47%, H = 6.65%, N = 6.46%.

C. Das oben erhaltene (Z)-But-2-endicarbonsäure 1-D-glucit-1-ylamid-4-[3-D-glucit-1-ylcarbamoyl-4-(2-methoxy-ethoxy)-phenyl]-amid wurde analog Beispiel 1.B. zu (Z)-But-2-endicarbonsäure 1-(2,3,4,5,6-penta-O-sulfo-D-glucit-1-yl)-amid 4-[4-(2-methoxy-ethoxy)-3-(2,3,4,5,6-penta-O-sulf-D-glucit-1-ylcarbamoyl)-phenyl]-amid Decanatriumsalz, $[\alpha]_D^{20}$ -2,7°, (*c* 0,6; Wasser), MS: *m/z* 1655,5 (rekonstruiertes M), umgesetzt.

Beispiel 48

A. Eine Lösung von 1,0 g (Z)-But-2-endicarbonsäure 1-D-glucit-1-ylamid-4-[3-D-glucit-1-ylcarbamoyl-4-(2-methoxy-ethoxy)-phenyl]-amid in 8 ml Wasser wurde mit 100 mg Palladium auf Kohle Katalysator (10%) versetzt und während 2 Stunden in einer Wasserstoffatmosphäre hydriert. Hierauf wurde der Katalysator abfiltriert und das Filtrat lyophilisiert. So erhielt man N1-D-Glucit-1-yl-N4-[3-D-glucit-1-ylcarbamoyl-4-(2-methoxy-ethoxy)-phenyl]-bernsteinsäureamid als gräuliches Pulver, MS: *m/z* 638,4 ($[M+H]^+$).

B. Das oben erhaltene N1-D-Glucit-1-yl-N4-[3-D-glucit-1-ylcarbamoyl-4-(2-methoxy-ethoxy)-phenyl]-bernsteinsäureamid wurde analog Beispiel 1.B. zu N1-[4-(2-methoxy-ethoxy)-3-(2,3,4,5,6-penta-O-sulfo-D-glucit-1-ylcarbamoyl)-phenyl]-N4-(2,3,4,5,6-penta-O-sulfo-D-glucit-1-yl)-bernsteinsäureamid Decanatriumsalz, $[\alpha]_D^{20}$ -2,4°, (*c* 0,5; Wasser), MS: *m/z* 1657,5 (rekonstruiertes M), umgesetzt.

Beispiel 49

A. 1,07 g 4-(4-Hydroxy-phenyl)-benzoesäure, 2,8 g wasserfreies Kaliumcarbonat, 0,65 g Bis (4-fluorophenyl)sulfon und 70 mg Dicyclohexano-18-krone-6 wurden in 18 ml 1,3-Dimethyl-2-imidazolidinon suspendiert und während 6 Stunden bei 150°C und weiteren 3 Stunden bei 175°C unter Argon gerührt. Hierauf wurde das Reaktionsgemisch auf Ethanol gegossen, die ausgefallenen Kristalle abfiltriert und mit Ethanol/Ether gewaschen. Das so erhaltene Rohprodukt wurde hierauf an LiChroprep RP-18 Kieselgel mit Wasser und anschliessend mit Wasser mit ansteigendem Anteil an Methanol chromatographiert. Nach dem Eindampfen der relevanten Fraktionen, kurzem Aufkochen in 1 N Salzsäure und Filtration über einen 0,8 µm Cellulosefilter wurde 4'-[4-[4-(4'-Carboxy-biphenyl-4-yloxy)-phenylsulfonyl]-phenoxy]-biphenyl-4-carbonsäure als farbloses Pulver erhalten, MS: *m/z* 641,2 ($[M-H]^-$).

B. Analog Beispiel 6.A. wurde aus 4'-[4-[4-(4'-Carboxy-biphenyl-4-yloxy)-phenylsulfonyl]-phenoxy]-biphenyl-4-carbonsäure und D-Glucamin 4'-[4-[4-[4'-(D-glucit-1-ylcarbamoyl)-biphenyl-4-yloxy]-phenylsulfonyl]-phenoxy]-biphenyl-4-carbonsäure D-glucit-1-ylamid erhalten, welches direkt in die nächste Stufe eingesetzt wurde.

C. Das oben erhaltene 4'-[4-[4-[4'-(D-glucit-1-ylcarbamoyl)-biphenyl-4-yloxy]-phenylsulfonyl]-phenoxy]-biphenyl-4-carbonsäure D-glucit-1-ylamid wurde analog Beispiel 1.B. zu 4'-[4-[4-[4'-(2,3,4,5,6-Penta-O-sulfo-D-glucit-1-ylcarbamoyl)-biphenyl-4-yloxy]-phenylsulfonyl]-phenoxy]-biphenyl-4-carbonsäure 2,3,4,5,6-penta-O-sulfo-D-glucit-1-ylamid Decanatriumsalz, $[\alpha]_D^{20}$ -4,3°,(*c* 0,6; Wasser), MS: *m/z* 1989,0 (rekonstruiertes M), umgesetzt.

Beispiel 50

A. Analog Beispiel 5.B. wurde aus 5,5'-(Methylen-bis-oxymethylen)-di-furan-2-carbonsäure und D-Glucamin 5,5'-(Methylen-bis-oxymethylen)-di-furan-2-carbonsäure-bis-D-glucit-1-ylamid als weisses Pulver erhalten,MS: *m/z* 623,4 ($[M+H]^+$).

B. Das oben erhaltene 5,5'-(Methylen-bis-oxymethylen)-di-furan-2-carbonsäure-bis-D-glucit-1-ylamid wurde analog Beispiel 1.B. zu 5,5'-(Methylene-bis-oxymethylen)-di-furan-2-carbonsäure bis-(2,3,4,5,6-penta-O-sulfo-D-glucit-1-yl-amid) Decanatriumsalz , $[\alpha]_D^{20}$ -2,2°(*c* 0,6; Wasser), MS: *m/z* 1642,5 (rekonstruiertes M), umgesetzt.

Beispiel 51

A. Eine Lösung von 3,15 g 1-(4-Amino-phenyl)-3-(2,3,4,5,6-penta-O-acetyl-D-glucit-1-yl)-harnstoff (vgl. Bsp. 7.B.) und 0,63 g Oxalsäurediimidazolid in 30 ml Acetonitril wurde 2 Stunden bei Raumtemperatur gerührt und dann eingedampft. Der Rückstand wurde mit Methylenchlorid/Isopropanol an Kieselgel chromatographiert und ergab N,N'-Bis-[4-[3-(penta-O-acetyl-D-glucit-1-yl)-ureido]-phenyl]-oxalamid, $[\alpha]_D^{20}$ -21,2°(*c* 0,5; Chloroform), MS: *m/z* 1105,7 ($[M+H]^+$).

B. Eine Lösung von oben erhaltenem N,N'-Bis-[4-[3-(penta-O-acetyl-D-glucit-1-yl)-ureido]-phenyl]-oxalamid in 10 ml Dimethoxyethan und 25 ml Methanol wurde in Gegenwart von 1 ml 2%iger methanolischer Natriummethylatlösung 4 Stunden bei Raumtemperatur gelöst. Der entstandene Niederschlag wurde abgenutscht, mit Methanol und Ether nachgewaschen und bei 60°C getrocknet. Man erhielt N,N'-Bis-[4-[3-(D-glucit-1-yl)ureido]-phenyl]-oxalamid, $[\alpha]_D^{20}$ -15,4°(*c* 0,5; Dimethylsulfoxid), MS: *m/z* 685,2 ($[M+H]^+$).

C. Sulfatierung von N,N'-Bis-[4-[3-(D-glucit-1-yl)-ureido]-phenyl]-oxalamid wie unter 1.B. beschrieben lieferte N, N'-Bis-[4-[3-(2,3,4,5,6-penta-O-sulfo-D-glucit-1-yl)-ureido]-phenyl]-oxalamid Decanatriumsalz, $[\alpha]_D^{20}$ +3,6° (c 0,5; Wasser), MS: *m/z* 1705,5 (rekonstruiertes M).

Beispiel 52

A. Umsetzung von Z-Stilben-4,4'-dicarbonsäure und Tris-(hydroxymethyl)-aminomethan wie unter 2.A. beschrieben ergab Z-Stilben-4,4'-dicarbonsäure-bis-(2-hydroxy-1,1-bis-hydroxymethyl-ethylamid), MS: *m/z* 475,4 ($[M+H]^+$).

B. Sulfatierung von Z-Stilben-4,4'-dicarbonsäure-bis-(2-hydroxy-1,1-bis-hydroxymethyl-ethylamid) wie unter 1B beschrieben lieferte Z-Stilben-4,4'-dicarbonsäure-bis-(2-hydroxysulfonyloxy-1,1-bis-hydroxysulfonyloxymethyl-ethylamid) Hexanatriumsalz, MS: *m/z* 1086,8 (rekonstruiertes M).

Beispiel 53

A. 18,2 g 5-Hydroxy-isophthalsäure wurden in 300 ml Methanol unter Zusatz von 5 ml Schwefelsäure (96%) während 18 Stunden bei Rückfluss gerührt. Nach dem Abkühlen auf 5°C wurde mit gesättigter Natriumbicarbonatlösung auf pH 8 gestellt, dann das Methanol im Wasserstrahlvakuum abdestilliert. Der heterogene Rückstand wurde mit Methylenchlorid erschöpfend extrahiert; die organische Phase wurde über Magnesiumsulfat getrocknet, filtriert und eingeengt. Man erhielt 5-Hydroxy-isophthalsäure-dimethylester, welcher aus Methylenchlorid/n-Hexan umkristallisiert wurde, Schmelzpunkt 159-161 °C.

B. Zu einer Lösung von 0,690 g 5-Hydroxy-isophthalsäuredimethylester, 0,582 g Biphenyl-4-yl-methanol und 1,182 g Triphenylphosphin in 40 ml Tetrahydrofuran wurde bei -10°C 1,043 g Di-tert.-butyl-azodicarboxylat fest hinzugefügt. Das Reaktionsgemisch wurde 1 Stunde bei 0 °C und weitere 16 Stunden bei Raumtemperatur unter Argon gerührt. Anschliessend wurde das Reaktionsgemisch filtriert, der Filterrückstand mit Methylenchlorid gewaschen und das Filtrat eingedampft. Der so erhaltene Rückstand wurde mit Methylenchlorid an Kieselgel chromatographiert. Dabei erhielt man 5-Biphenyl-4-ylmethoxy-isophthalsäuredimethylester, Smp. 118-119 °C.

C. 1,03 g 5-Biphenyl-4-ylmethoxy-isophthalsäuredimethylester wurden in 12 ml 1 N Natronlauge und 6 ml Acetonitril während 16 Stunden bei Raumtemperatur gerührt. Das Reaktionsgemisch wurde unter vermindertem Druck eingeengt, mit Wasser versetzt, mit verdünnter $KHSO_4$-Lösung angesäuert und filtriert. Dabei erhielt man 5-Biphenyl-4-ylmethoxy-isophthalsäure, MS: *m/z* 347,3 ($[M-H]^-$).

D. Analog Beispiel 6.A. wurde aus 5-Biphenyl-4-ylmethoxy-isophthalsäure und D-Glukamin 5-Biphenyl-4-ylmethoxy-N,N'-di-D-glucit-1-yl-isophthal-amid als weisses Pulver erhalten, MS: *m/z* 675,4 ($[M+H]^+$).

E. Das oben erhaltene 5-Biphenyl-4-ylmethoxy-N,N'-di-D-glucit-1-yl-isophthalamid wurde analog Beispiel 1.B. zu 5-Biphenyl-4-ylmethoxy-N,N'-bis-(2,3,4,5,6-penta-O-sulfo-D-glucit-1-yl)-isophthalamid Decanatriumsalz, $[\alpha]_D^{20}$ -6,5° (*c* 0.8, Wasser), umgesetzt.

Beispiel 54

A. Umsetzung von 4,4'-Ethinylendibenzoesäure (Tetrahedron Lett. 32,3117 (1967)) und D-Glucamin wie unter Beispiel 3.A. beschrieben ergab 4,4'-Ethinylendibenzoesäure-bis-D-glucit-1-ylamid, $[\alpha]_D^{20}$ -3,2° (*c* 0,5; DMSO), MS: *m/z* 593,5 ($[M+H]^+$).

B . Sulfatierung von 4,4'-Ethinylendibenzoesäure-bis-D-glucit-1-ylamid wie unter 2.B. beschrieben ergab 4,4'-Ethinylendibenzoesäure-bis-(2,3,4,5,6-penta-O-sulfo-D-glucit-1-ylamid) Decanatriumsalz, $[\alpha]_D^{20}$ -5,6° (*c* 0,5; Wasser), MS: *m/z* 1613,0 (rekonstruiertes M).

Beispiel 55

A. Eine Lösung von 0,4 g Bis-(4-hydroxyphenyl)-methan in 8 ml Acetonitril wurde unter Argon mit 0,3 g gepulvertem Kaliumcarbonat und 0,4 g tert.-Butylbromacetat versetzt und 2,5 Stunden bei Raumtemperatur gerührt. Anschliessend wurden nochmals 0,13 g tert.-Butylbromacetat hinzugefügt und während 70 Stunden bei Raumtemperatur gerührt. Hierauf wurde das Reaktionsgemisch eingeengt und der Rückstand mit Methylenchlorid/Ether (95:5) an Kieselgel chromatographiert, dabei wurde 4,4'-Methylen-bis-phenoxyessigsäure-di-tert.-butylester als farbloser Festkörper erhalten, MS: *m/z* 428 ($[M]^+$).

B. Eine Lösung von 1,0 g 4,4'-Methylen-bis-phenoxyessigsäuredi-tert.-butylester in 18 ml Dioxan wurde mit 3 ml Trifluoressigsäure und 3 ml Wasser versetzt und während 8 Stunden bei 110°C gerührt. Anschliessend wurde das Reaktionsgemisch eingeengt und mit Wasser versetzt. Die ausgefallenen Kristalle wurden abfiltriert, dabei wurde 4,4'-Methylen-bis-phenoxyessigsäure als farbloser Festkörper erhalten, MS: *m/z* 316 ($[M]^+$).

C. Analog Beispiel 6.A. wurde aus 4,4'-Methylen-bis-phenoxyessigsäure und D-Glucamin N-D-Glucit-1-yl-2-[4-(4-D-glucit-1-ylcarbamoylmethoxy-benzyl)-phenoxy]-acetamid als farbloser Festkörper erhalten, MS: *m/z* 665,3 ($[M+Na]^+$).

E. Das oben erhaltene N-D-Glucit-1-yl-2-[4-(4-D-glucit-1-yl-carbamoyl-methoxy-benzyl)-phenoxy]-acetamid wurde analog Beispiel 1.B. zu N-(2,3,4,5,6-Penta-O-sulfo-D-glucit-1-yl)-2-[4-[4-(2,3,4,5,6-penta-O-sulfo-D-glucit-1-ylcarbamoylmethoxy)-benzyl]-phenoxy]-acetamid Decanatriumsalz, $[\alpha]_D^{20}$ +5,4° (*c* 0.5, Wasser), umgesetzt; MS: *m/z* 1663,0 (rekonstruiertes M).

Beispiel 56

A. Eine Suspension von 0,98 g (Z)-3-[3-Biphenyl-4-yloxymethyl-5-[(Z)-3-carboxy-acryloylamino]-phenylcarbamoyl]-acrylsäure in 10 ml Dimethylformamid wurde unter Feuchtigkeitsausschluss bei 0 - 5°C mit 0,5 ml 4-Methylmorpholin versetzt und während 10 Minuten intensiv gerührt. Hierauf wurden 0,75 g 2-Chlor-4,6-dimethoxy-1,3,5-triazin fest hinzugefügt, und das Reaktionsgemisch wurde weitere 2 Stunden bei 0 - 5°C gerührt. Nach Zugabe von 0,75 g D-Glukamin wurde weitere 18 Stunden bei Raumtemperatur gerührt. Hierauf wurde das Reaktionsgemisch mehrmals mit Wasser verdünnt und unter vermindertem Druck bei 40 - 50°C eingedampft. Das so erhaltene Rohprodukt wurde hierauf an LiChroprep RP-18 Kieselgel mit Wasser und anschliessend mit Wasser mit ansteigendem Anteil an Methanol chromatographiert. Nach dem Eindampfen der relevanten Fraktionen und Trocknen bei 50°C im Vakuum wurde (Z)-Butendisäure (Z)-[3-biphenyl-4-yloxymethyl-5-(3-D-glucit-1-ylcarbamoyl-acryloylamino)-phenylamid]-D-glucit-1-ylamid als bräunliches Pulver erhalten, MS: *m/z* 835,2 ($[M+Na]^+$).

B. Das oben erhaltene (Z)-Butendisäure (Z)-[3-biphenyl-4-yloxymethyl-5-(3-D-glucit-1-ylcarbamoyl-acryloylamino)-phenylamid]-D-glucit-1-ylamid wurde analog Beispiel 1.B. zu (Z)-Butendisäure (Z)-[3-biphenyl-4-yloxymethyl-5-[3-(2,3,4,5,6-penta-O-sulfo-D-glucit-1-ylcarbamoyl)-acryloylamino]-phenylamid]-(2,3,4,5,6-penta-O-sulfo-D-glucit-1-yl)-amid Decanatriumsalz, $[\alpha]_D^{20}$ -3,4° (*c* 0.5, Wasser), umgesetzt; MS: *m/z* 1834,0 (rekonstruiertes M).

Beispiel 57

A. 0,581 mg 5-(Biphenyl-4-yloxymethyl)-benzol-1,3-diamin, 0,4 g Maleinsäureanhydrid, 40 mg 4-Dimethylamino-pyridin und 0,6 ml Triäthylamin wurden in 18 ml Methylenchlorid während 18 Std. unter Argon gerührt. Hierauf wurde das Reaktionsgemisch eingeengt. Der Rückstand wurde mit Wasser versetzt und mit Essigester extrahiert. Aus der wässerigen Phase wurde durch Zugabe von 1 N Salzsäure das Produkt ausgefällt. Nach Abfiltrieren und Trocknen wurde (Z)-3-[3-Biphenyl-4-yloxymethyl-5-[(Z)-3-carboxy-acryloylamino]-phenylcarbamoyl]-acrylsäure als bräunlicher Festkörper MS: *m/z* 485,3 ($[M-H]^-$).

B. Eine Suspension von 0,94 g (Z)-3-[3-Biphenyl-4-yloxymethyl-5-[(Z)-3-carboxy-acryloylamino]-phenylcarbamoyl]-acrylsäure in 10 ml Dimethylformamid wurde unter Feuchtigkeitsausschluss bei 0 - 5°C mit 0,45 ml 4-Methylmorpholin versetzt und während 10 Minuten intensiv gerührt. Hierauf wurden 0,702 g 2-Chlor-4,6-dimethoxy-1,3,5-triazin fest hinzugefügt und weitere 2 Stunden bei 0 - 5°C gerührt. Nach Zugabe von 0,727 g D-Glukamin wurde weitere 18 Stunden bei Raumtemperatur gerührt. Hierauf wurde das Reaktionsgemisch mehrmals mit Wasser verdünnt und unter vermindertem Druck bei 40 - 50°C eingedampft, mit 40 ml Pyridin und 50 ml Acetanhydrid versetzt und weitere 18 Std. gerührt. Dann wurde das Reaktionsgemisch erneut eingeengt und mit Eiswasser und 1 N Salzsäure versetzt. Der so erhaltene Niederschlag wurde abfiltriert und mit Methylenchlorid/ Isopropanol an

Kieselgel chromatographiert. Dabei resultierte (Z)-Butendicarbonsäure-(Z)-1-[3-biphenyl-4-yloxymethyl-5-[3-(2,3,4,5,6-penta-O-acetyl-D-glucit-1-ylcarbamoyl)-acryloylamino]-phenylamid] 4-(2,3,4,5,6-penta-O-acetyl-D-glucit-1-ylamid) als bräunlicher Festkörper, MS: *m/z* 1250,6 ($[M+NH_4]^+$).

C. 0,188 g (Z)-Butendicarbonsäure-(Z)-1-[3-biphenyl-4-yloxymethyl-5-[3-(2,3,4,5,6-penta-O-acetyl-D-glucit-1-ylcarbamoyl)-acryloylamino]-phenylamid]-4-(2,3,4,5,6-penta-O-acetyl-D-glucit-1-ylamid) wurden in 15 ml Aethylacetat unter Zusatz von 30 mg Palladium auf Kohle (10%) in einer Wasserstoffatmosphäre erschöpfend hydriert. Anschliessend wurde das Reaktionsgemisch über Dicalit abfiltriert und eingeengt. Dabei wurde N1-(2,3,4,5,6-penta-O-acetyl-D-glucit-1-yl)-N4-[5-biphenyl-4-yloxymethyl-3-[3-(2,3,4,5,6-penta-O-acetyl-D-glucit-1-ylcarbamoyl)-propionylamino]- phenyl]-bernsteinsäureamid als farbloser Festkörper erhalten; MS: *m/z* 1259,7 ($[M+Na]^+$).

D. Eine Lösung von 0,17 g N1-(2,3,4,5,6-Penta-O-acetyl-D-glucit-1-yl)-N4-[5-biphenyl-4-yloxymethyl-3-[3-(2,3,4,5,6-penta-O-acetyl-D-glucit-1-yl-carbamoyl)-propionylamino]-phenyl]-bernsteinsäureamid in 10 ml Methanol wurde mit 10 mg wasserfreiem Natriumcarbonat versetzt und über Nacht bei Raumtemperatur gerührt. Der dabei gebildete Niederschlag wurde abfiltriert und mit Methanol gewaschen. So wurde N1-[5-Biphenyl-4-yloxymethyl-3-(3-D-glucit-1-ylcarbamoyl-propionylamino)-phenyl]-N4-D-glucit-1-yl-bernsteinsäureamid als bräunlicher Festkörper erhalten; MS: *m/z* 839,6 ($[M+Na]^+$).

E. Das oben erhaltene N1-[5-Biphenyl-4-yloxymethyl-3-(3-D-glucit-1-ylcarbamoyl-propionylamino)-phenyl]-N4-D-glucit-1-yl-bernsteinsäureamid wurde analog Beispiel 1.B. zu N1-[3-Biphenyl-4-yloxymethyl-5-[3-(2,3,4,5,6-penta-O-sulfo-D-glucit-1-ylcarbamoyl)-propionylamino]-phenyl]-N4-(2,3,4,5,6-penta-O-sulfo-D-glucit-1-yl)-bernsteinsäureamid Decanatriumsalz umgesetzt, $[\alpha]_D^{20}$ -4,4° (*c* 0.5, Wasser), MS: *m/z* 1837,5 (rekonstruiertes M).

Beispiel 58

A. 1,1 g 3,5-Dinitrobenzylchlorid, 0,85 g 4-Hydroxybiphenyl und 0,7 g fein gemahlenes Kaliumcarbonat wurden in 10 ml Acetonitril unter Argon während 2,5 Stunden bei 50°C und anschliessend während weiteren 8 Std. unter Rückfluss erwärmt. Anschliessend wurde filtriert, und das Filtrat wurde eingeengt. Der so erhaltene Rückstand wurde mit Methylenchlorid/Hexan (9:1) an Kieselgel chromatographiert. Dabei erhielt man 4-(3,5-Dinitro-benzyloxy)-biphenyl als gelblichen Festkörper; Elementaranalyse berechnet für $C_{19}H_{14}N_2O_5$: C=65,14%, H = 4,03%, N = 8,00%; gefunden: C = 64,84%, H = 4,04%, N = 7,79%.

B. 2,65 g 4-(3,5-Dinitro-benzyloxy)-biphenyl wurden in 30 mi Essigester unter Zusatz von 200 mg Platinoxid in einer Wasserstoffatmosphäre erschöpfend hydriert. Anschliessend wurde das Reaktionsgemisch über Dicalit abfiltriert und eingeengt. Der Rückstand wurde aus Ether/Hexan umkristallisert; dabei wurde 5-(Biphenyl-4-yloxymethyl)-benzol-1,3-diamin in Form farbloser Kristalle erhalten, MS: *m/z* 290 ($[M]^+$).

C. Eine Lösung von 1,0 g 5-(Biphenyl-4-yloxymethyl)-benzol-1,3-diamin, in 25 ml Tetrahydrofuran wurde unter Argon und unter Eiskühlung mit 0,97 ml Triethylamin und 690 mg Triphosgen versetzt und anschliessend während 4 Stunden bei Raumtemperatur gerührt. Hierauf wurde filtriert. Der Rückstand wurde eingeengt, in 20 ml Dimethylformamid gelöst, mit 1,3 g D-Glukamin versetzt und 18 Stunden bei Raumtemperatur gerührt. Anschliessend wurde erneut eingeengt, mit 50 ml Pyridin und 50 ml Acetanhydrid versetzt und weitere 18 Stunden gerührt. Hierauf wurde das Reaktionsgemisch erneut eingeengt, mit Eiswasser und 1 N Salzsäure versetzt und mit Methylenchlorid extrahiert. Die Methylenchoridphasen wurden über Magnesiumsulfat getrocknet und eingeengt. Der Rückstand wurde mit Essigester an Kieselgel chromatographiert. Die Produktfraktion wurde anschliessend in 25 ml Methanol und 10 ml Tetrahydrofuran gelöst, mit 1 mi 1 molarer Natriummethylatlösung in Methanol versetzt und weitere 18 Stunden bei Raumtemperatur gerührt. Dann wurde der ausgefallene Niederschlag abfiltriert und zur weiteren Reinigung erneut mit 25 ml Pyridin und 25 ml Acetanhydrid acetyliert. Das Rohprodukt wurde mit Methylenchlorid/Isopropanol (91:9) an Kieselgel chromatographiert. Dabei resultierte 3,3'-Bis-(2,3,4,5,6-penta-O-acetyl-D-glucit-1-yl)-1,1'-(5-biphenyl-4-yloxymethyl-benzol-1,3-diyl)-diharnstoff als farbloser Schaum, MS: *m/z* 1142,6 ($[M+NH_4]^+$).

D. Eine Lösung von 0,68 g 3,3'-Bis-(2,3,4,5,6-penta-O-acetyl-D-glucit-1-yl)-1,1'-(5-biphenyl-4-yloxymethyl-benzol-1,3-diyl)-diharnstoff in 10 ml Methanol und 4 ml Tetrahydrofuran wurde mit 0,5 ml 1 molarer Natriummethylatlösung in Methanol versetzt und 2 Stunden bei Raumtemperatur gerührt. Dann wurde der ausgefallene Niederschlag abfiltriert; dabei resultierte 3,3'-Di-D-glucit-1-yl-1,1'-(5-biphenyl-4-yloxymethyl-benzol-1,3-diyl)-diharnstoff als farbloser Festkörper, MS: *m/z* 705,4 ($[M+H]^+$).

E. Der oben erhaltene 3,3'-Di-D-glucit-1-yl-1,1'-(5-biphenyl-4-yloxymethyl-benzol-1,3-diyl)-diharnstoff wurde analog Beispiel 1.B. zu 3,3'-Bis-(2,3,4,5,6-penta-O-sulfo-D-glucit-1-yl)-1,1'-(5-biphenyl-4-yloxymethyl-benzol-1,3-diyl)-diharnstoff Decanatriumsalz umgesetzt, $[\alpha]_D^{20}$ -0,86° (*c* 0.7, Wasser), MS: *m/z* 1726,0 (rekonstruiertes M).

Beispiel 59

A. 3,00 g 2,3:4,5-Di-O-isopropyliden-1-O-(4-methyl-phenylsulfonyl)-D-arabinitol, 0,94 g 9,9-Dimethyl-9H-xanthen-3,6-diol und 5,38 g Kaliumcarbonat wurden in 130 ml Dimethylformamid während 3 Stunden bei Rückfluss gerührt. Das Reaktionsgemisch wurde mit Eiswasser versetzt und mit Methylenchlorid extrahiert. Die vereinigten Methylenchloridphasen wurden über Magnesiumsulfat getrocknet und eingeengt. Hierauf wurde das Rohprodukt an Kieselgel mit Hexan, Methylenchlorid und Methanol chromatographiert. Man erhielt 2,3:2',3':4,5:4'5'-Tetra-O-isopropyliden-1,1'-O-(9,9-dimethyl-9H-xanthen-3,6-diyl)-di-D-arabinitol, MS: *m/z* 655 ($[M-CH_3]^-$).

B. 1,80 g 2,3:2',3':4,5:4'5'-Tetra-O-isopropyliden-1,1'-O-(9,9-dimethyl-9H-xanthen-3,6-diyl)-di-D-arabinitol wurden in 70 ml Dioxan mit 12 ml Trifluoressigsäure (50 % in Wasser) während 3 Stunden bei Rückfluss gerührt. Nach dem Abkühlen auf Raumtemperatur wurde eingeengt, der Rückstand zweimal mit 50 ml Toluol versetzt und erneut eingeengt; der so erhaltene Rückstand wurde in 50 ml Wasser aufgenommen, 1 Stunde bei Raumtemperatur gerührt, dann auf 10 °C gekühlt und abfiltriert. Nach dem Trocknen über Phosphorpentoxid bei 50°C erhielt man 1,1'-O-(9,9-Dimethyl-9H-xanthen-3,6-diyl)-di-D-arabinitol; IR (KBr, $cm^{-1}$): 3394, 1613, 1502 , 1262, 1180, 1041, 833.

C. 1,02 g 1,1'-O-(9,9-Dimethyl-9H-xanthen-3,6-diyl)-di-D-arabinitol wurden in Analogie zu Beispiel 1.B. mit Schwefeltrioxidtrimethylaminkomplex umgesetzt. Dabei wurde 2,2',3,3',4,4',5,5'-Octa-O-sulfo-1,1'-O-(9,9-dimethyl-9H-xanthen-3,6-diyl)-di-D-arabinitol Octanatriumsalz erhalten, MS: *m/z* 1327,5 (rekonstruiertes M).

Beispiel 60

A. Eine Lösung von 0,47 g 3,6,9-Trioxaundecandisäure in 10 ml Acetonitril wurde unter Feuchtigkeitsausschluss bei 0 - 5 °C mit 0,44 ml 4-Methyl-morpholin versetzt und während 10 Minuten intensiv gerührt. Hierauf wurden 0,70 g 2-Chlor-4,6-dimethoxy-1,3,5-triazin fest hinzugefügt und weitere 2 Stunden bei 0 - 5 °C gerührt. Anschliessend wurde eine Lösung von 1,29 g 1-O-(4-Amino-phenyl)-2,3:4,5-di-O-isopropyliden-D-arabinitol in 30 ml Acetonitril/Dimethylformamid (2:1) zugefügt, und es wurde weitere 18 Stunden bei Raumtemperatur gerührt. Nach dem Abdestillieren der Lösungsmittel im Hochvakuum wurde der Rückstand an Kieselgel mit Methylenchlorid und Methanol chromatographiert. Man erhielt 3,6,9-Trioxaundecandisäure bis-[4-(2,3:4,5-di-O-isopropyliden-D-arabinit-1-yloxy)-phenylamid], MS: *m/z* 833 ($[M+H]^+$).

B. 1,12 g 3,6,9-Trioxaundecandisäure bis-[4-(2,3:4,5-di-O-isopropyliden-D-arabinit-1-yloxy)-phenylamid] wurde in Analogie zu Beispiel 12.D. umgesetzt und aufgearbeitet. Man erhielt 3,6,9-Trioxaundecandisäure bis-(4-D-arabinit-1-yloxy-phenylamid); IR (KBr, $cm^{-1}$): 3410, 3264, 1661, 1514, 1236, 1112, 1043, 825.

C. 3,6,9-Trioxaundecandisäure bis-(4-D-arabinit-1-yloxy-phenylamid) wurde in Analogie zu Beispiel 1.B. mit Schwefeltrioxidtrimethylaminkomplex umgesetzt. Dabei wurde 3,6,9-Trioxaundecandisäure bis-[4-(2,3,4,5-tetra-O-sulfo-D-arabinit-1-yloxy)-phenylamid] Octanatriumsalz erhalten, MS: *m/z* 1489,5 (rekonstruiertes M).

Beispiel 61

A. 4,4'-Dihydroxy-biphenyl und 2,3:4,5-Di-O-isopropyliden-1-O-(4-methyl-phenylsulfonyl)-D-arabinitol wurden in Analogie zu Beispiel 59.A. umgesetzt und aufgearbeitet. Das Rohprodukt wurde an Kieselgel mit Methylenchlorid und Methanol chromatographiert. Man erhielt 2,3:2',3':4,5:4',5'-Tetra-O-isopropyliden-1,1'-O-biphenyl-4,4'-diyl-di-D-arabinitol, MS: *m/z* 614 ($[M]^-$).

B. 0,98 g 2,3:2',3':4,5:4',5'-Tetra-O-isopropyliden-1,1'-O-biphenyl-4,4'-diyl-di-D-arabinitol wurden in Analogie zu Beispiel 12.D. mit 6 ml Trifluoressigsäure (70% in Wasser) und 15 ml Dioxan während 6 Stunden bei Rückfluss gerührt. Nach dem Abkühlen des Reaktionsgemisches auf Raumtemperatur wurde der Ansatz filtriert; der Rückstand wurde zweimal mit je 5 ml Essigester gewaschen und am Hochvakuum bei 50 °C über Phosphorpentoxid getrocknet. Man erhielt 1,1'-O-Biphenyl-4,4'-diyl-di-D-arabinitol, MS: *m/z* 455 ($[M]^-$).

G. 0,55 g 1,1'-O-Biphenyl-4,4'-diyl-di-D-arabinitol wurden in Analogie zu Beispiel 1.B. mit Schwefeltrioxidtrimethylaminkomplex umgesetzt. Dabei wurde 2,2',3,3',4,4',5,5'-Octa-O-sulfo-1.1'-O-biphenyl-4,4'-diyl-di-D-arabinitol Octanatriumsalz erhalten, MS: *m/z* 1271,0 (rekonstruiertes M).

Beispiel 62

A. 2,3:4,5-Di-O-isopropyliden-1-O-(4-methyl-phenylsulfonyl)-D-arabinitol und 4,4'-Ethindiyl-diphenol wurden in Analogie zu Beispiel 59.A. umgesetzt und aufgearbeitet. Das Rohprodukt wurde an Kieselgel mit Hexan, Methylenchlorid und Acetonitril chromatographiert. Man erhielt 2,3:2',3':4,5:4',5'-Tetra-O-isopropyliden-1,1'-O-(4,4'-ethindiyl-diphenylen)-di-D-arabinitol, MS: *m/z* 638 ($[M]^-$).

B. 1,57 g 2,3:2',3':4,5:4',5'-Tetra-O-isopropyliden-1,1'-O-(4,4'-ethindiyl-diphenylen)-di-D-arabinitol wurde in Analogie zu Beispiel 59.B. mit 25 ml Trifluoressigsäure (60% in Wasser) in 50 ml Dioxan umgesetzt und aufgearbeitet. Dabei wurde 1,1'-O-(4,4'-Ethindiyl-diphenylen)-di-D-arabinitol erhalten, Elementaranalyse berechnet für $C_{24}H_{30}O_{10}$: C =60,24%, H = 6,32%; gefunden: C = 59,98%, H = 6,35%.

C. 1,00 g 1,1'-O-(4,4'-Ethindiyl-diphenylen)-di-D-arabinitol wurde in Analogie zu Beispiel 1.B. mit Schwefeltrioxidtrimethylaminkomplex umgesetzt. Dabei wurde 2,2',3,3',4,4',5,5'-Octa-O-sulfo-1,1'-O-(4,4'-ethindiyl-diphenylen)-di-D-arabinitol Octanatriumsalz erhalten, MS: *m/z* 1294,5 (rekonstruiertes M).

Beispiel 63

0,40 g 2,2',3,3',4,4',5,5'-Octa-O-sulfo-1,1'-O-(4,4'-ethindiyl-diphenylen)-di-D-arabinitol Octanatriumsalz wurden mit 0,16 g Lindlar-Katalysator unter Zusatz von 1,5 ml Pyridin in 12 ml Wasser hydriert. Nach dem Abfiltrieren des Katalysators wurde das Filtrat lyophilisiert und ergab (Z)-2,2',3,3',4,4',5,5'-Octa-O-sulfo-1,1'-O-(4,4'-ethylen-diphenylen)-di-D-arabinitol Octanatriumsalz, MS: *m/z* 1296,5 (rekonstruiertes M).

Beispiel 64

0,42 g 2,2',3,3',4,4',5,5'-Octa-O-sulfo-1,1'-O-(4,4'-ethindiyl-diphenylen)-di-D-arabinitol Octanatriumsalz wurden durch katalytische Hydrierung mit 0,17 g Palladium auf Kohle (10%) in 10 ml Wasser reduziert. Nach dem Abfiltrieren des Katalysators wurde das Filtrat lyophilisiert. Dabei wurde 2,2',3,3',4,4',5,5'-Octa-O-sulfo- 1,1'-O-(4,4'ethan-1,2-diyl-diphenylen)-di-D-arabinitol Octanatriumsalz erhalten, MS: *m/z* 1299,0 (rekonstruiertes M).

Beispiel 65

A. 24,50 g 1,2;3,4-Di-O-isopropyliden-alpha-D-galactopyranose wurde mit 19,70 g p-Toluolsulfonylchlorid unter Zusatz von 0,12 g 4-Dimethylamino-pyridin in 200 ml Pyridin während 18 Stunden bei Raumtemperatur umgesetzt. Das Reaktionsgemisch wurde extrahierend mit Eiswasser und Methylenchlorid aufgearbeitet. Die organische Phase wurde zweimal mit Wasser gewaschen, über Magnesiumsulfat getrocknet, filtriert und eingeengt. Das Rohprodukt wurde an Kieselgel mit Methylenchlorid und Methanol chromatographiert. Man erhielt 1,2;3,4-Di-O-isopropyliden-6-O-(p-tolylsulfonyl)-alpha-D-galactopyranose, welche aus n-Hexan umkristallisiert wurde, Smp.: 91-92 °C.

B. 18,64 g 1,2;3,4-Di-O-isopropyliden-6-O-(p-tolylsulfonyl)-alpha-D-galactopyranose und 6,85 g 4-Nitrophenol wurden in Analogie zu Beispiel 59.A. umgesetzt. Das so erhaltene Rohprodukt wurde an Kieselgel mit n-Hexan und Methylenchlorid chromatographiert. Man erhielt 1,2:3,4-Di-O-isopropyliden-6-O-(4-nitro-phenyl)-alpha-D-galacto-pyranose, MS: *m/z* 381 ($[M]^-$).

C. 13,60 g 1,2:3,4-Di-O-isopropyliden-6-O-(4-nitro-phenyl)-alpha-D-galacto-pyranose wurden mit 3,0 g Palladium auf Kohle in 150 ml Methanol hydriert. Nach dem Abfiltrieren des Katalysators wurde zweimal mit Methanol gewaschen. Das Filtrat wurde eingeengt und ergab 1,2:3,4-Di-O-isopropyliden-6-O-(4-amino-phenyl)-alpha-D-galacto-pyranose, MS: *m/z* 351 ($[M]^-$).

D. 4,35 g 1,2:3,4-Di-O-isopropyliden-6-O-(4-amino-phenyl)-alpha-D-galacto-pyranose wurde mit 3,72 g Dibenzyldicarbonat und 6,20 ml Triethylamin in 150 ml Dioxan/Wasser 2:1 während 16 Stunden bei Raumtemperatur umgesetzt. Das Reaktionsgemisch wurde extrahierend mit Eiswasser und Methylenchlorid aufgearbeitet. Die organische Phase wurde mit Wasser gewaschen, über Magnesiumsulfat getrocknet, filtriert und eingeengt. Das so erhaltene Rohprodukt wurde an Kieselgel mit n-Hexan, Methylenchlorid und Acetonitril chromatographiert. Dabei erhielt man 6-O-(4-Benzyloxy-carbonyl-amino-phenyl)-1,2:3,4-di-O-isopropyliden-alpha-D-galactopyranose, MS:

*m/z* 485 ($[M]^-$).

E. 1,49 g 6-O-(4-Benzyloxycarbonylamino-phenyl)-1,2:3,4-di-O-iso-propyliden-alpha-D-galactopyranose wurde in Analogie zu Beispiel 12. D. mit 15 ml Trifluoressigsäure (35 % in Wasser) und 20 ml Dioxan während 3 Stunden bei Rückfluss gerührt. Nach dem Abdestillieren des Lösungsmittels im Hochvakuum wurde der Rückstand aus Methanol und Ether umkristallisiert. Dabei wurde 6-O-(4-Benzyloxycarbonylamino-phenyl)-D-galactose erhalten, MS: *m/z* 406 ($[M+H]^+$).

F. 3,60 g 6-O-(4-Benzyloxycarbonylamino-phenyl)-D-galactose wurde mit 1,40 g Natriumborhydrid in 125 ml Methanol während 65 Stunden bei Raumtemperatur gerührt. Nach Hydrolyse und Neutralisation des Reaktionsgemisches wurde dieses eingeengt. Der Rückstand wurde am Hochvakuum über Phosphorpentoxid während 4 Stunden getrocknet. Zur Reinigung wurde der Rückstand mit Acetanhydrid in Pyridin in das entsprechende Pentaacetylderivat übergeführt und dieses an Kieselgel mit Methylenchlorid chromatographiert. Dabei erhielt man 1,2,3,4,5-Penta-O-acetyl-6-O-(4-benzyloxycarbonylamino-phenyl)-D-galactitol, welches direkt in die nächste Stufe eingesetzt wurde.

G. 5,20 g 1,2,3,4,5-Penta-O-acetyl-6-O-(4-benzyloxycarbonylamino-phenyl)-D-galactitol wurde mit 1,0 g Palladium auf Kohle (10%) in 150 ml Tetrahydrofuran hydriert. Nach dem Abfiltrieren des Katalysators wurde das Lösungsmittel abdestilliert. Der Rückstand wurde aus Methylenchlorid und n-Hexan umkristallisiert. So erhielt man 1,2,3,4,5-Penta-O-acetyl-6-O-(4-amino-phenyl)-D-galactitol, welches direkt in die nächste Stufe eingesetzt wurde.

H. 3,37 g 1,2,3,4,5-Penta-O-acetyl-6-O-(4-amino-phenyl)-D-galactitol wurde in 250 ml Methanol mit 5,30 mi Natriummethylatlösung (5,4 molar in Methanol) während 18 Stunden bei Raumtemperatur gerührt. Nach der Neutralisation des Reaktionsgemisches mit 2N Salzsäure wurde das Lösungsmittel abdestilliert. Das Rohprodukt wurde in 50 ml Wasser aufgenommen, 15 Minuten bei Raumtemperatur gerührt, danach filtriert und zweimal mit je 10 ml Wasser gewaschen. Der Rückstand wurde in 100 ml 2N Salzsäure während 3 Stunden bei Rückfluss gerührt und danach eingeengt. Dieser Rückstand wurde am Hochvakuum bei Raumtemperatur während 6 Stunden über Phosphorpentoxid getrocknet. Man erhielt 6-O-(4-Amino-phenyl)-D-galactitol-hydrochlorid, MS: *m/z* 273 ($[M]^-$).

I. 1,24 g 6-O-(4-Amino-phenyl)-D-galactitol-hydrochlorid wurde in 40 ml Dimethylformamid mit 0,32 g 1,1'-Carbonyldiimidazol unter Zusatz von 1,12 ml Triethylamin während 20 Stunden bei Raumtemperatur gerührt. Durch Zugabe von Wasser wurde das Produkt ausgefällt, filtriert, zweimal mit Wasser gewaschen und im Hochvakuum während 5 Stunden über Phosphorpentoxid bei 50 °C getrocknet. Man erhielt 1,3-Bis-(4-D-galactit-6-yloxy-phenyl)-harnstoff, MS: *m/z* 595 ($[M+Na]^+$).

K. 0,73 g 1,3-Bis-(4-D-galactit-6-yloxy-phenyl)-harnstoff wurde in Analogie zu Beispiel 1.B. mit Schwefeltrioxidtrimethylaminkomplex umgesetzt. Dabei erhielt man 1,3-Bis-[4-(2,3,4,5,6-penta-O-sulfo-D-galactit-6-yloxy)-phenyl]-harnstoff Decanatriumsalz, MS: *m/z* 1593,0 (rekonstruiertes M).

Beispiel 66

A. 2,79 g 2,3:4,5-Di-O-isopropyliden-D-arabinitol wurden mit 0,98 ml Propargylbromid unter Zusatz von 0,78 g Kaliumhydroxid in 30 ml Tetrahydrofuran während 18 Stunden bei Raumtemperatur gerührt. Nach dem Abdestillieren des Lösungsmittels wurde das Rohprodukt an Kieselgel mit Methylenchlorid chromatographiert. So erhielt man 2,3:4,5-Di-O-isopropyliden-1-O-prop-2-inyl-D-arabinitol, MS: *m/z* 255 ($[M-CH_3]^-$).

B. 2,06 g 2,3:4,5-Di-O-isopropyliden-1-O-prop-2-inyl-D-arabinitol wurden in 55 ml 1,2-Dichlorbenzol mit 1,11 ml N,N,N',N'-Tetramethylethylendiamin, 0,07 g Kupfer(I)chlorid und 11,1 g Molekularsieb 4Å unter Durchströmen von Sauerstoff bei 80 °C während 2 Stunden gerührt. Nach dem Abdestillieren des Lösungsmittels wurde der Rückstand an Kieselgel mit Methylenchlorid und Methanol chromatographiert. Dabei erhielt man 2,3:2',3':4,5:4',5'-Tetra-O-isopropyliden-1,1'-O-hexa-2,4-diin-1,6-diyl-di-D-arabinitol, MS: *m/z* 523 ($[M-CH_3]^-$).

C. 1,10 g 2,3:2',3':4,5:4',5'-Tetra-O-isopropyliden-1,1'-O-hexa-2,4-diin-1,6-diyl-di-D-arabinitol wurden in Analogie zu Beispiel 12.D. mit 6,6 ml Trifluoressigsäure (70% in Wasser) in 35 ml Dioxan umgesetzt. Man erhielt 1,1'-O-Hexa-2,4-diin-1,6-diyl-di-D-arabinitol, welches direkt in die nächste Stufe eingesetzt wurde.

D. 0,75 g 1,1'-O-Hexa-2,4-diin-1,6-diyl-di-D-arabinitol wurde in Analogie zu Beispiel 1.B. mit Schwefeltrioxidtrimethylaminkomplex umgesetzt. So erhielt man 2,2',3,3',4,4',5,5'-Octa-O-sulfo-1,1'-O-hexa-2,4-diyn-1,6-diyl-di-D-arabinitol Octanatriumsalz, MS: *m/z* 1194,5 (rekonstruiertes M).

Beispiel 67

A. Eine Lösung von 3,44 g 4,4'-Difluor-3,3'-dinitrodiphenylsulfon in 50 ml absolutem Dimethylformamid und 3 ml Triethylamin wurde mit 3,7 g D-Glucamin versetzt und 5 Stunden bei Raumtemperatur gerührt. Dann wurden 50 ml Methanol zugetropft, das ausgefallene Produkt wurde abgenutscht, mit Methanol nachgewaschen und getrocknet. Man erhielt N,N'-Di-D-glucit-1-yl-2,2'-dinitro-4,4'-sulfonyldianilin als gelbes Pulver, $[\alpha]_D^{20}$ +12,2° (*c* 0,5; DMSO), MS: *m /z* 689,7 ([M+Na]$^+$).

B. Umsetzung von N,N'-Di-D-glucit-1-yl-2,2'-dinitro-4,4'-sulfonyldianilin wie unter 2.B. beschrieben (wobei Schwefeltrioxid-triethylaminkomplex anstelle des Schwefeltrioxidtrimethylaminkomplexes verwendet wurde) ergab N,N'-Bis-(2,3,4,5,6-penta-O-sulfo-D-glucit-1-yl)-2,2'-dinitro-4,4'-sulfonyldianilin Decanatriumsalz als oranges Pulver, $[\alpha]_D^{20}$ -2,0° (*c* 0,5; Wasser), MS: *m/z* 1687,0 (rekonstruiertes M).

Beispiel 68

A. Eine Lösung von 7,4 g 4-Fluor-3-nitrobenzoesäure in 100 ml Dimethylformamid wurde mit 16,0 g D-Glucamin versetzt und 4 Stunden bei Raumtemperatur gerührt. Nach Zugabe von 6 ml Triethylamin wurde 16 Stunden bei 40°C weitergerührt. Die Reaktionslösung wurde eingedampft. Der Rückstand wurde mit 400 ml Pyridin und 200 ml Essigsäureanhydrid 5 Stunden bei Raumtemperatur gerührt. Nach Einengen wurde der erhaltene Rückstand mit Wasser versetzt und mit 5 %iger Salzsäurelösung auf pH = 2-3 angesäuert und mit Essigester extrahiert. Die organischen Extrakte wurden mit Eiswasser und gesättigter Kochsalzlösung gewaschen, über Magnesiumsulfat getrocknet und eingedampft. Der Rückstand wurde mit Essigester über Kieselgel chromatographiert. Die Produktfraktionen wurden eingeengt, aus Ether kristallisiert und ergaben 4-(2,3,4,5,6-Penta-O-acetyl-D-glucit-1-ylamin)-3-nitrobenzoesäure als gelbe Kristalle, $[\alpha]_D^{20}$ -23,0° (*c* 0,5; DMSO), MS: *m/z* 579,7 ([M+Na]$^+$).

B. Eine Lösung von 1,12 g 4-(2,3,4,5,6-Penta-O-acetyl-D-glucit-1-ylamin)-3-nitrobenzoesäure in 6 ml Dimethylformamid und 0,23 ml N-Methylmorpholin wurde bei 0°C mit 385 mg 2-Chlor-4,6-dimethoxy-1,3,5-triazin versetzt und 2 Stunden gerührt. Nach Zugabe von 198 mg 4,4'-Diamino-diphenylmethan wurde 18 Stunden bei Raumtemperatur gerührt und eingeengt. Der Rückstand wurde mit Eiswasser versetzt und mit Essigester ausgezogen. Die Extrakte wurden mit Eiswasser und gesättigter Kochsalzlösung gewaschen, über Magnesiumsulfat getrocknet und eingedampft. Der Rückstand wurde mit Methylenchlorid/ Isopropanol über Kieselgel chromatographiert und ergab 4,4'-Bis-(2,3,4,5,6-penta-O-acetyl-D-glucit-1-ylamino)-3,3'-dinitro-N,N'-(4,4'-methylen-diphenyl)-dibenzamid als gelbes Pulver, $[\alpha]_D^{20}$ -6,8° (*c* 0,5; DMSO), MS: *m/z* 1298,3 ([M+Na]$^+$).

C. Eine Lösung von 1,3 g 4,4'-Bis-(2,3,4,5,6-penta-O-acetyl-D-glucit-1-ylamino)-3,3'-dinitro-N,N'-(4,4'-methylen-diphenyl)-dibenzamid in 13 ml Tetrahydrofuran und 15 mi Methanol wurde mit 1,3 ml 2 %iger methanolischer Natriummethanolatlösung 5 Stunden bei Raumtemperatur gerührt. Der Niederschlag wurde abgenutscht, mit Methanol gewaschen, getrocknet und ergab 4,4'-Bis-D-glucit-1-ylamin-3,3'-dinitro-N,N'-(4,4'-methylen-diphenyl)-dibenzamid als gelb-oranges Pulver, $[\alpha]_D^{20}$ +24,0° (*c* 0,5; DMSO), MS: *m/z* 877,7 ([M+Na]$^+$).

D. Umsetzung von 4,4'-Bis-D-glucit-1-ylamin-3,3'-dinitro-N.N'-(4,4'-methylen-diphenyl)-dibenzamid wie unter 67.B. beschrieben ergab 4,4'-Bis-(2,3,4,5,6-penta-O-sulfo-D-glucit-1-ylamino)-3,3'-dinitro-N.N'-(4,4'-methylen-diphenyl)-dibenzamid Decanatriumsalz als gelb-oranges Pulver, $[\alpha]_D^{20}$ +8,2° (*c* 0,5; Wasser), MS: *m/z* 1875,0 (rekonstruiertes M).

Beispiel 69

A. Umsetzung von 4-(2,3,4,5,6-Penta-O-acetyl-D-glucit-1-ylamino)-3-nitrobenzoesäure mit 1,3-Diaminobenzol wie unter 68.B. beschrieben ergab 4,4'-Bis-(2,3,4,5,6-penta-O-acetyl-D-glucit-1-ylamino)-3,3'-dinitro-N,N'-benzen-1,3-diyl-dibenzamid, $[\alpha]_D^{20}$ -6,6° (*c* 0,5; DMSO), MS: *m/z* 1207,6 ([M+Na]$^+$).

B. Umsetzung von 4,4'-Bis-(2,3,4,5,6-penta-O-acetyl-D-glucit-1-ylamino)-3,3'-dinitro-N,N'-benzen-1,3-diyl-dibenzamid wie unter 68.C. beschrieben ergab 4,4'-Bis-D-glucit-1-ylamino-3,3'-dinitro-N,N'-benzen-1,3-diyl-dibenzamid, $[\alpha]_D^{20}$ +27,4° (*c* 0,5; DMSO), MS: *m/z* 787,5 ([M+Na]$^+$).

C. Umsetzung von 4,4'-Bis-D-glucit-1-ylamino-3,3'-dinitro-N,N'-benzen-1,3-diyl-dibenzamid wie unter 67.B. beschrieben ergab 4,4'-Bis-(2,3,4,5,6-penta-O-sulfo-D-glucit-1-ylamino)-3,3'-dinitro-N,N'-benzen-1,3-diyl-dibenzamid Decanatriumsalz als gelb-oranges Pulver, $[\alpha]_D^{20}$ +9,8° (*c* 0,5; Wasser), MS: *m/z* 1785,0 (rekonstruiertes M).

Beispiel 70

A. Umsetzung von 4-(2,3,4,5,6-Penta-O-acetyl-D-glucit-1-ylamino)-3-nitrobenzoesäure mit 1,3-Diamino-propan-2-ol wie unter 68.B. beschrieben ergab 4,4'-Bis-(2,3,4,5,6-penta-O-acetyl-D-glucit-1-ylamino)-3,3'-dinitro-N,N'-(2-hydroxy-propan-1,3-diyl)-dibenzamid, $[\alpha]_D^{20}$ -3,8° (*c* 0,5; DMSO), MS: *m/z* 1189,3 ($[M+Na]^+$).

B. Umsetzung von 4,4'-Bis-(2,3,4,5,6-penta-O-acetyl-D-glucit-1-ylamino)-3,3'-dinitro-N,N'-(2-hydroxy-propan-1,3-diyl)-dibenzamid wie unter 68.C. beschrieben ergab 4,4'-Bis-D-glucit-1-ylamino-3,3'-dinitro-N,N'-(2-hydroxy-propan-1,3-diyl)-dibenzamid, $[\alpha]_D^{20}$ +27,2° (*c* 0,5; DMSO), MS: *m/z* 769 ($[M+Na]^+$).

C. Umsetzung von 4,4'-Bis-D-glucit-1-ylamino-3,3'-dinitro-N,N'-(2-hydroxypropan-1,3-diyl)-dibenzamid wie unter 67.B. beschrieben ergab 4,4'-Bis-(2,3,4,5,6-penta-O-sulfo-D-glucit-1-ylamino)-3,3'-dinitro-N,N'-(2-hydroxysulfonyloxy-propan-1,3-diyl)-dibenzamid Undecanatriumsalz als gelb-oranges Pulver, $[\alpha]_D^{20}$ +14,4° (*c* 0,5; Wasser), MS: *m/z* 1869,0 (rekonstruiertes M).

Beispiel 71

A. Eine Lösung von 2,0 g (Z)-Stilben-4,4'-dicarbonsäure-bis-(2,3,4,5,6-penta-O-sulfo-D-glucit-1-ylamid) Decanatriumsalz (vgl. Bsp. 3) in 25 ml Methylenchlorid wurde mit 1,0 g m-Chlorbenzoesäure versetzt und 5 Stunden bei Raumtemperatur gerührt. Die Reaktionslösung wurde auf Eiswasser gegossen und mit Methylenchlorid extrahiert. Die Extrakte wurden mit Wasser und Natriumbicarbonatlösung gewaschen, getrocknet und eingeengt. Der Rückstand wurde mit Methylenchlorid/Isopropanol auf Kieselgel chromatographiert und ergab cis-4,4'-Oxiran-2,3-diyl-dibenzoesäure-bis-(2,3,4,5,6-penta-O-acetyl-D-glucit-1-ylamid), $[\alpha]_D^{20}$ +18,8° (*c* 0,5; Chloroform), MS: *m/z* 1054,5 ($[M+Na]^+$).

B. Eine Suspension von 1,6 g cis-4,4'-Oxiran-2,3-diyl-dibenzoesäure-bis-(2,3,4,5,6-penta-O-acetyl-D-glucit-1-ylamid) in 25 ml Methanol wurde mit 480 mg Kaliumcarbonat versetzt und 3 Stunden bei Raumtemperatur gerührt. Das Reaktionsgemisch wurde eingedampft und Wasser/Acetonitril über eine 'reversed phase'-Säule (RP 18) gereinigt. Lyophilisierte Produktfraktionen ergaben reines cis-4,4'-Oxiran-2,3-diyl-dibenzoesäure-bis-D-glucit-1-ylamid, $[\alpha]_D^{20}$ -7,0° (*c* 0,5; DMSO), MS: *m/z* 611,4 ($[M+H]^+$).

C. Sulfatierung von cis-4,4'-Oxiran-2,3-diyl-dibenzoesäure-bis-D-glucit-1-ylamid wie unter Beispiel 67.B. beschrieben ergab cis-4,4'-Oxiran-2,3-diyl-dibenzoesäure-bis-(2,3,4,5,6-penta-O-sulfo-D-glucit-1-ylamid) Decanatriumsalz, $[\alpha]_D^{20}$ -4,0° (*c* 0,9; Wasser), MS: *m/z* 1632,0 (rekonstruiertes M).

Beispiel 72

A. Eine Lösung von 38,0 g literaturbekanntem (3-Brom-benzyl)-triphenylphosphoniumbromid in 250 ml Tetrahydrofuran wurde bei -10°C mit 8,75 g Kalium-tert.-butylat versetzt und 3 Minuten gerührt. Zu der gelborangen Suspension wurde innert 15 Minuten eine Lösung von 14,8 g 4-Brombenzaldehyd zugetropft. Nach 60 Minuten Rühren wurde das Reaktionsgemisch eingeengt. Der Rückstand wurde mit Eiswasser versetzt und mit Essigester/Ether ausgezogen. Die organischen Phasen wurden mit mit Wasser und gesättigter wässriger Natriumchioridlösung gewaschen, über Magnesiumsulfat getrocknet und eingedampft. Der Rückstand wurde mit Hexan und Hexan/Ether 99:1 über Kieselgel chromatographiert und ergab (E)-3,4'-Dibromstilben als farblose Kristalle, MS: *m/z* 338 ($M^+$), sowie (Z)-3,4'-Dibromstilben als farbloses Öl, MS: *m/z* 338 ($M^+$).

B. Eine Lösung von 3,38 g (E)-3,4'-Dibromstilben in 200 ml absolutem Ether wurde bei -10°C während 5-10 Minuten mit 20 ml einer 1,6 N Butyllithiumlösung in Hexan versetzt. Nach 3 Stunden Rühren wurde die Reaktionslösung auf Trockeneis/Ether gegossen und langsam auf 10°C erwärmt. Die Etherlösung wurde mehrfach mit Wasser extrahiert. Nach dem Ansäuern der wäßrigen Lösung mit 2 N Salzsäure wurde der ausgefallene Niederschlag abgenutscht, mit Wasser gewaschen und bei 60°C im Trockenschrank über Nacht getrocknet. Das Rohprodukt wurde in 50 ml Dimethoxyethan gelöst, mit 30 ml Diazomethanlösung versetzt und 1 Stunde bei Raumtemperatur gerührt. Ein Überschuß an Diazomethan wurde durch Zugabe von Essigsäure vernichtet. Das Reaktionsgemisch wurde eingeengt und an Kieselgel mit Essigester/Hexan/Methylenchlorid chromatographiert. Man erhielt (E)-Stilben-3,4'-dicarbonsäure-dimethylester, MS: *m/z* 296 ($M^+$).

C. Eine Lösung von 2,2 g (E)-Stilben-3,4'-dicarbonsäuredimethylester in 25 ml Dimethoxyethan und 12 ml Methanol wurde mit 10 ml 2 m Natriumhydroxidlösung versetzt und 18 Stunden unter Rückfluß erhitzt. Die Reaktionslösung wurde eingeengt. Der wäßrige Rückstand wurde weiter mit Wasser verdünnt und mit 2 n Salzsäure auf pH 1 angesäuert. Der Niederschlag wurde abgenutscht, mit Eiswasser nachgewaschen und im Trockenschrank bei 60°C über Nacht getrocknet. Man erhielt (E)-Stilben-3,4'-dicarbonsäure als leicht gefärbtes Pulver, MS: *m/z* 268 ($M^+$).

D. Umsetzung von (E)-Stilben-3,4'-dicarbonsäure und D-Glucamin wie unter Beispiel 3.A. beschrieben ergab (E)-Stilben-3,4'-dicarbonsäure-bis-D-glucit-1-ylamid, $[\alpha]_D^{20}$ -1,6° (*c* 0,5; DMSO), MS: *m/z* 595,4 ($[M+H]^+$).

E. Sulfatierung von (E)-Stilben-3,4'-dicarbonsäure-bis-D-glucit-1-ylamid wie unter 2.B. beschrieben ergab (E)-Stilben-3,4'-dicarbonsäure-bis-(2,3,4,5,6-penta-O-sulfo-D-glucit-1-ylamid) Decanatriumsalz, $[\alpha]_D^{20}$ -6,6° (*c* 0,5; Wasser), MS: *m/z* 1615,0 (rekonstruiertes M).

<u>Beispiel 73</u>

A. Umsetzung von 3-Brombenzaldehyd mit (3-Brom-benzyl)-triphenyl-phosphoniumbromid wie unter 72.A. beschrieben ergab (E)-3,3'-Dibromstilben als farblose Kristalle, MS: *m/z* 338 ($M^+$), sowie (Z)-3,3'-Dibromstilben als farbloses Öl, MS: *m/z* 338 ($M^+$).

B. Umsetzung von (E)-3,3'-Dibromstilben wie unter 72.B. beschrieben ergab (E)-Stilben-3,3'-dicarbonsäuredimethylester, MS: *m/z* 296 ($M^+$).

C. Umsetzung von (E)-Stilben-3,3'-dicarbonsäuredimethylester wie unter 72.C. beschrieben ergab (E)-Stilben-3,3'-dicarbonsäure, MS: *m/z* 268 ($M^+$).

D. Umsetzung von (E)-Stilben-3,3'-dicarbonsäure und D-Glucamin wie unter Beispiel 3.A. beschrieben ergab (E)-Stilben-3,3'-dicarbonsäure-bis-D-glucit-1-ylamid, $[\alpha]_D^{20}$ -3,2° (*c* 0,5; DMSO), MS: *m/z* 595,5 ($[M+H]^+$).

E. Sulfatierung von (E)-Stilben-3,3'-dicarbonsäure-bis-D-glucit-1-ylamid wie unter 2.B. beschrieben ergab (E)-Stilben-3,3'-dicarbonsäure-bis-(2,3,4,5,6-penta-O-sulfo-D-glucit-1-ylamid) Decanatriumsalz, $[\alpha]_D^{20}$ -7,8°(*c* 0,5; Wasser), MS: *m/z* 1616,0 (rekonstruiertes M).

<u>Beispiel 74</u>

A. Umsetzung von 4-Brombenzaldehyd mit (2-Brom-benzyl)-triphenyl-phosphoniumbromid wie unter 72.A. beschrieben ergab (E)-2,4'-Dibromstilben als farblose Kristalle, MS: *m/z* 338 ($M^+$), sowie (Z)-2,4'-Dibromstilben als farbloses Öl, MS: *m/z* 338 ($M^+$).

B. Umsetzung von (E)-2,4'-Dibromstilben wie unter 72.B. beschrieben ergab (E)-Stilben-2,4'-dicarbonsäuredimethylester, MS: *m/z* 296 ($M^+$).

C. Umsetzung von (E)-Stilben-2,4'-dicarbonsäuredimethylester wie unter 72.C. beschrieben ergab (E)-Stilben-2,4'-dicarbonsäure, MS: *m/z* 268 ($M^+$).

D. Umsetzung von (E)-Stilben-2,4'-dicarbonsäure und D-Glucamin wie unter Beispiel 3.A. beschrieben ergab (E)-Stilben-2,4'-dicarbonsäure-bis-D-glucit-1-ylamid, $[\alpha]_D^{20}$ -11,8°(*c* 0,5; DMSO), MS: *m/z* 595,4 ($[M+H]^+$).

E. Sulfatierung von (E)-Stilben-2,4'-dicarbonsäure-bis-D-glucit-1-ylamid wie unter 2.B. beschrieben ergab (E)-Stilben-2,4'-dicarbonsäure-bis-(2,3,4,5,6-penta-O-sulfo-D-glucit-1-ylamid) Decanatriumsalz, $[\alpha]_D^{20}$ -7,4°(*c* 0,5; Wasser), MS: *m/z* 1615,0 (rekonstruiertes M).

<u>Beispiel 75</u>

A. Umsetzung von 3-Brombenzaldehyd mit (2-Brom-benzyl)-triphenyl-phosphoniumbromid wie unter 72.A. beschrieben ergab (E)-2,3'-Dibromstilben als farblose Kristalle, MS: *m/z* 338 ($M^+$), sowie (Z)-2,3'-Dibromstilben als farbloses Öl, MS: *m/z* 338 ($M^+$).

B. Umsetzung von (E)-2,3'-Dibromstilben wie unter 72.B. beschrieben ergab (E)-Stilben-2,3'-dicarbonsäuredimethylester, MS: *m/z* 296 ($M^+$).

C. Umsetzung von (E)-Stilben-2,3'-dicarbonsäuredimethylester wie unter 72.C. beschrieben ergab (E)-Stilben-2,3'-dicarbonsäure, MS: *m/z* 268 ($M^+$).

D. Umsetzung von (E)-Stilben-2,3'-dicarbonsäure und D-Glucamin wie unter Beispiel 3.A. beschrieben ergab (E)-Stilben-2,3'-dicarbonsäure-bis-D-glucit-1-ylamid, $[\alpha]_D^{20}$ -9,4° (*c* 0,5; DMSO), MS: *m/z* 595,4 ($[M+H]^+$)

E. Sulfatierung von (E)-Stilben-2,3'-dicarbonsäure-bis-D-glucit-1-ylamid wie unter 2.B. beschrieben ergab (E)-Stilben-2,3'-dicarbonsäure-bis-(2,3,4,5,6-penta-O-sulfo-D-glucit-1-ylamid) Decanatriumsalz, $[\alpha]_D^{20}$ -9,6° (*c* 0,5; Wasser), MS: *m/z* 1616,0 (rekonstruiertes M).

Beispiel 76

A. Umsetzung von 2-Brombenzaldehyd mit (2-Brom-benzyl)-triphenylphosphoniumbromid wie unter 72.A. beschrieben ergab (Z)-2,2'-Dibromstilben als farbloses Öl, MS: *m/z* 338 ($M^+$).

B. Umsetzung von (Z)-2,2'-Dibromstilben wie unter 72.B. beschrieben ergab (Z)-Stilben-2,2'-dicarbonsäuredimethylester, MS: *m/z* 296 ($M^+$).

C. Umsetzung von (Z)-Stilben-2,2'-dicarbonsäuredimethylester wie unter 72.C. beschrieben ergab (Z)-Stilben-2,2'-dicarbonsäure, MS: *m/z* 268 ($M^+$).

D. Umsetzung von (Z)-Stilben-2,2'-dicarbonsäure und D-Glucamin wie unter Beispiel 3.A. beschrieben ergab (Z)-Stilben-2,2'-dicarbonsäure-bis-D-glucit-1-ylamid, $[\alpha]_D^{20}$ D -8,2° (*c* 0,5; DMSO), MS: *m/z* 595,5 ($[M+H]^+$).

E. Sulfatierung von (Z)-Stilben-2,2'-dicarbonsäure-bis-D-glucit-1-ylamid wie unter 2.B. beschrieben ergab (Z)-Stilben-2,2'-dicarbonsäure-bis-(2,3,4,5,6-penta-O-sulfo-D-glucit-1-ylamid) Decanatriumsalz, $[\alpha]_D^{20}$ -15,4° (*c* 0,5; Wasser), MS: *m/z* 1615,0 (rekonstruiertes M).

Beispiel 77

A. Umsetzung von (Z)-3,4'-Dibromstilben wie unter 72.B. beschrieben ergab (Z)-Stilben-3,4'-dicarbonsäuredimethylester, MS: *m/z* 296 ($M^+$).

B. Umsetzung von (Z)-Stilben-3,4'-dicarbonsäuredimethylester wie unter 72.C. beschrieben ergab (Z)-Stilben-3,4'-dicarbonsäure, MS: *m/z* 268 ($M^+$).

C. Umsetzung von (Z)-Stilben-3,4'-dicarbonsäure und D-Glucamin wie unter Beispiel 3.A. beschrieben ergab (Z)-Stilben-3,4'-dicarbonsäure-bis-D-glucit-1-ylamid, $[\alpha]_D^{20}$ -8,4° (*c* 0,5; DMSO), MS: *m/z* 595,5 ($[M+H]^+$).

D. Sulfatierung von (Z)-Stilben-3,4'-dicarbonsäure-bis-D-glucit-1-ylamid wie unter 2.B. beschrieben ergab (Z)-Stilben-3,4'-dicarbonsäure-bis-(2,3,4,5,6-penta-O-sulfo-D-glucit-1-ylamid) Decanatriumsalz, $[\alpha]_D^{20}$ -6,2° (*c* 0,5; Wasser), MS: *m/z* 1616,0 (rekonstruiertes M).

Beispiel 78

A. Umsetzung von (Z)-3,3'-Dibromstilben wie unter 72.B. beschrieben ergab (Z)-Stilben-3,3'-dicarbonsäuredimethylester, MS: *m/z* 296 ($M^+$).

B. Umsetzung von (Z)-Stilben-3,3'-dicarbonsäuredimethylester wie unter 72.C. beschrieben ergab (Z)-Stilben-3,3'-dicarbonsäure, MS: *m/z* 268 ($M^+$).

C. Umsetzung von (Z)-Stilben-3,3'-dicarbonsäure und D-Glucamin wie unter Beispiel 3.A. beschrieben ergab (Z)-Stilben-3,3'-dicarbonsäure-bis-D-glucit-1-ylamid, $[\alpha]_D^{20}$ -9,4° (*c* 0,5; DMSO), MS: *m/z* 595,4 ($[M+H]^+$).

D. Sulfatierung von (Z)-Stilben-3,3'-dicarbonsäure-bis-D-glucit-1-ylamid wie unter 2.B. beschrieben ergab (Z)-Stilben-3,3'-dicarbonsäure-bis-(2,3,4,5,6-penta-O-sulfo-D-glucit-1-ylamid) Decanatriumsalz, $[\alpha]_D^{20}$ -8,6° (*c* 0,5; Wasser), MS: *m/z* 1615,0 (rekonstruiertes M).

Beispiel 79

A. Umsetzung von (Z)-2,3'-Dibromstilben wie unter 72.B. beschrieben ergab (Z)-Stilben-2,3'-dicarbonsäuredimethylester, MS: *m/z* 296 ($M^+$).

B. Umsetzung von (Z)-Stilben-2,3'-dicarbonsäuredimethylester wie unter 72.C. beschrieben ergab (Z)-Stilben-2,3'-dicarbonsäure, MS: *m/z* 268 ($M^+$).

C. Umsetzung von (Z)-Stilben-2,3'-dicarbonsäure und D-Glucamin wie unter Beispiel 3.A. beschrieben ergab (Z)-Stilben-2,3'-dicarbonsäure-bis-D-glucit-1-ylamid, $[\alpha]_D^{20}$ -6,0° (*c* 0,5; DMSO), MS: *m/z* 595,4 ($[M+H]^+$).

D. Sulfatierung von (Z)-Stilben-2,3'-dicarbonsäure-bis-D-glucit-1-ylamid wie unter 2.B. beschrieben ergab (Z)-Stilben-2,3'-dicarbonsäure-bis-(2,3,4,5,6-penta-O-sulfo-D-glucit-1-ylamid) Decanatriumsalz, $[\alpha]_D^{20}$ -11,0° (*c* 0,5; Wasser), MS: *m/z* 1615,0 (rekonstruiertes M).

Beispiel 80

Hydrierung von (E)-Stilben-3,4'-dicarbonsäure-bis-(2,3,4,5,6-penta-O-sulfo-D-glucit-1-ylamid) Decanatriumsalz (vgl. Bsp. 72) wie unter 10. beschrieben ergab 3,4'-Ethylendibenzoesäure-bis-(2,3,4,5,6-penta-O-sulfo-D-glucit-1-ylamid) Decanatriumsalz, $[\alpha]_D^{20}$ -6,0° (*c* 0,5; Wasser), MS: *m/z* 1617,5 (rekonstruiertes M).

Beispiel 81

Hydrierung von (E)-Stilben-2,4'-dicarbonsäure-bis-(2,3,4,5,6-penta-O-sulfo-D-glucit-1-ylamid) Decanatriumsalz (vgl. Bsp. 74) wie unter 10. beschrieben ergab 2,4'-Ethylendibenzoesäure-bis-(2,3,4,5,6-penta-O-sulfo-D-glucit-1-ylamid) Decanatriumsalz, $[\alpha]_D^{20}$ -8,4° (*c* 0,5; Wasser), MS: *m/z* 1616,8 (rekonstruiertes M).

Beispiel 82

Hydrierung von (E)-Stilben-2,3'-dicarbonsäure-bis-(2,3,4,5,6-penta-O-sulfo-D-glucit-1-ylamid) Decanatriumsalz (vgl. Bsp. 75) wie unter 10. beschrieben ergab 2,3'-Ethylendibenzoesäure-bis-(2,3,4,5,6-penta-O-sulfo-D-glucit-1-ylamid) Decanatriumsalz, $[\alpha]_D^{20}$ -10,0° (*c* 0,5; Wasser), MS: *m/z* 1617,2 (rekonstruiertes M).

Beispiel 83

Hydrierung von (Z)-Stilben-2,2'-dicarbonsäure-bis-(2,3,4,5,6-penta-O-sulfo-D-glucit-1-ylamid) Decanatriumsalz (vgl. Bsp. 76) wie unter 10. beschrieben ergab 2,2'-Ethylendibenzoesäure-bis-(2,3,4,5,6-penta-O-sulfo-D-glucit-1-ylamid) Decanatriumsalz, $[\alpha]_D^{20}$ -11,0° (*c* 0,5; Wasser), MS: *m/z* 1617,2 (rekonstruiertes M).

Beispiel 84

Hydrierung von (E)-Stilben-3,3'-dicarbonsäure-bis-(2,3,4,5,6-penta-O-sulfo-D-glucit-1-ylamid) Decanatriumsalz (vgl. Bsp. 73) wie unter 10. beschrieben ergab 3,3'-Ethylendibenzoesäure-bis-(2,3,4,5,6-penta-O-sulfo-D-glucit-1-ylamid) Decanatriumsalz, $[\alpha]_D^{20}$ -4,2° (*c* 0,5; Wasser), MS: *m/z* 1617,6 (rekonstruiertes M).

Beispiel 85

A. Eine Lösung von 55,3 g 3-Chlor-4-methyl-benzoesäuremethylester in 1 l Tetrachlorkohlenstoff wurde mit 53,2 g N-Bromsuccinimid und 0,1 g Dibenzoylperoxîd versetzt und unter Belichtung mit einer 150 W Birne 2 Stunden am Rückfluß erhitzt. Ausgefallenes Succinimid wurde von der abgekühlten Reaktionslösung abfiltriert. Das Filtrat wurde eingeengt und mit Tetractuorkohlenstoff/Ether über Kieselgel filtriert. Das Eluat wurde eingedampft und im Vakuum getrocknet. Eine Lösung dieses Rohproduktes in 750 ml Benzol wurde mit 83 g Triphenylphosphin versetzt und 4 Stunden unter Rückfluß erhitzt, wobei das Produkt ausfiel. Die Suspension wurde auf ca. 15°C abgekühlt und genutscht. Das Produkt wurde mit Benzol und Ether gewaschen und bei 50°C im Vakuum getrocknet. Man erhielt (2-Chlor-4-methoxycarbonylbenzyl)-triphenylphosphoniumbromid, MS: *m/z* 445,3 ($M^+$).

B. Eine Suspension von 52,6 g (2-Chlor-4-methoxycarbonylbenzyl)-triphenylphosphoniumbromid in 500 ml Tetrahydrofuran wurde bei 0°C tropfenweise mit 115 ml 2 %iger methanolischer Natriummethylatlösung versetzt. Dann wurde innerhalb 10 Minuten eine Lösung von 16,4 g 4-Formylbenzoesäuremethylester zugetropft und 1 Stunde bei Raumtemperatur gerührt. Die Suspension wurde über Dicalit filtriert, und das Filtrat wurde eingedampft. Der Rückstand wurde mit Essigester/ Hexan/ Methylenchlorid chromatographiert und ergab (Z)-2-Chlorstilben-4,4'-dicarbonsäuredimethylester, MS: *m/z* 330 ($M^+$), und (E)-2-Chlorstilben-4,4'-dicarbonsäuredimethylester als farblose Kristalle, Smp. 134-135°C, MS: *m/z* 330 ($M^+$).

C. Umsetzung von (E)-2-Chlorstilben-4,4'-dicarbonsäuredimethylester wie unter 72.C. beschrieben ergab (E)-2-Chlorstilben-4,4'-dicarbonsäure, MS: *m/z* 302 ($M^+$).

D. Umsetzung von (E)-2-Chlorstilben-4,4'-dicarbonsäure und D-Glucamin wie unter Beispiel 3.A. beschrieben ergab (E)-2-Chlorstilben-4,4'-dicarbonsäure-bis-D-glucit-1-ylamid, $[\alpha]_D^{20}$ -2,0°(*c* 0,5; DMSO), MS: *m/z* 630,5 ($[M+H]^+$).

E. Sulfatierung von (E)-2-Chlorstilben-4,4'-dicarbonsäure-bis-D-glucit-1-ylamid wie unter 2.B. beschrieben ergab (E)-2-Chlorstilben-4,4'-dicarbonsäure-bis-(2,3,4,5,6-penta-O-sulfo-D-glucit-1-ylamid) Decanatriumsalz, $[\alpha]_D^{20}$ -5,2°(*c* 0,5; Wasser), MS: *m/z* 1650,0 (rekonstruiertes M).

Beispiel 86

A. Umsetzung von (Z)-2-Chlorstilben-4,4'-dicarbonsäuredimethylester wie unter 72.C. beschrieben ergab (Z)-2-Chlorstilben-4,4'-dicarbonsäure, MS: *m/z* 302 ($M^+$).

B. Umsetzung von (Z)-2-Chlorstilben-4,4'-dicarbonsäure und D-Glucamin wie unter Beispiel 3.A. beschrieben ergab (Z)-2-Chlorstilben-4,4'-dicarbonsäure-bis-D-glucit-1-ylamid, $[\alpha]_D^{20}$ -4,4°(*c* 0,5; DMSO), MS: *m/z* 629,6 ($[M+H]^+$).

C. Sulfatierung von (Z)-2-Chlorstilben-4,4'-dicarbonsäure-bis-D-glucit-1-ylamid wie unter 2.B. beschrieben ergab (Z)-2-Chlorstilben-4,4'-dicarbonsäure-bis-(2,3,4,5,6-penta-O-sulfo-D-glucit-1-ylamid) Decanatriumsalz, $[\alpha]_D^{20}$ -4,2°(*c* 0,5; Wasser), MS: *m/z* 1659,5 (rekonstruiertes M).

Beispiel 87

A. Umsetzung von (2-Brom-4-methoxycarbonylbenzyl)-triphenyl-phosphoniumbromid mit 4-Formylbenzoesäuremethylester wie in 85.B. beschrieben ergab kristalline (Z)-2-Bromstilben-4,4'-dicarbonsäure-dimethylester, MS: *m/z* 374 ($M^+$), und (E)-2-Bromstilben-4,4'-dicarbonsäure-dimethylester, MS: *m/z* 374 ($M^+$).

B. Umsetzung von (E)-2-Bromstilben-4,4'-dicarbonsäuredimethylester wie unter 72.C. beschrieben ergab (E)-2-Bromstilben-4,4'-dicarbonsäure, MS: Z/z 346 ($M^+$).

C. Umsetzung von (E)-2-Bromstilben-4,4'-dicarbonsäure und D-Glucamin wie unter Beispiel 3.A. beschrieben ergab (E)-2-Bromstilben-4,4'-dicarbonsäure-bis-D-glucit-1-ylamid, $[\alpha]_D^{20}$ -2,4°(*c* 0,5; DMSO), MS: *m/z* 695,5 ($[M+H]^+$).

D. Sulfatierung von (E)-2-Bromstilben-4,4'-dicarbonsäure-bis-D-glucit-1-ylamid wie unter 2.B. beschrieben ergab (E)-2-Bromstilben-4,4'-dicarbonsäure-bis-(2,3,4,5,6-penta-O-sulfo-D-glucit-1-ylamid) Decanatriumsalz, $[\alpha]_D^{20}$ -4,2° (*c* 0,5; Wasser), MS: *m/z* 1694,0 (rekonstruiertes M).

Beispiel 88

A. Umsetzung von (Z)-2-Bromstilben-4,4'-dicarbonsäuredimethylester wie unter 72.C. beschrieben ergab (Z)-2-Bromstilben-4,4'-dicarbonsäure, MS: *m /z* 346 ($M^+$).

B. Umsetzung von (Z)-2-Bromstilben-4,4'-dicarbonsäure und D-Glucamin wie unter Beispiel 3.A. beschrieben ergab (Z)-2-Bromstilben-4,4'-dicarbonsäure-bis-D-glucit-1-ylamid, $[\alpha]_D^{20}$ -2,4° (*c* 0,5; DMSO), MS: *m/z* 673,6 ($[M+H]^+$).

C. Sulfatierung von (Z)-2-Bromstilben-4,4'-dicarbonsäure-bis-D-glucit-1-ylamid wie unter 2.B. beschrieben ergab (Z)-2-Bromstilben-4,4'-dicarbonsäure-bis-(2,3,4,5,6-penta-O-sulfo-D-glucit-1-ylamid) Decanatriumsalz, $[\alpha]_D^{20}$ -2,8°(*c* 0,5; Wasser), MS: *m/z* 1694,0 (rekonstruiertes M).

Beispiel 89

A. Umsetzung von 4-(2,3,4,5,6-Penta-O-acetyl-D-glucit-1-ylamin)-3-nitrobenzoesäure mit (E)-4,4'-Diaminostilben wie unter 68.B. beschrieben ergab (E)-4,4'-Bis-(2,3,4,5,6-penta-O-acetyl-D-glucit-1-ylamino)-3,3'-dinitro-N,N'-stilben-4,4'-diyl-dibenzamid, $[\alpha]_D^{20}$ -10,4°(*c* 0,5; DMSO), MS: *m/z* 1287,4 ($[M+Na]^+$).

B. Umsetzung von (E)-4,4'-Bis-(2,3,4,5,6-penta-O-acetyl-D-glucit-1-ylamino)-3,3'-dinitro-N,N'-stilben-4,4'-diyl-dibenzamid wie unter 68.C. beschrieben ergab (E)-4,4'-Bis-D-glucit-1-ylamino-3,3'-dinitro-N,N'-stilben-4,4'-diyl-dibenzamid, $[\alpha]_D^{20}$ +25,0°(*c* 0,5; DMSO), MS: *m/z* 867,4 ($(M+Na]^+$).

C. Umsetzung von (E)-4,4'-Bis-D-glucit- 1-ylamino-3,3'-dinitro-N,N'-stilben-4,4'-diyl-dibenzamid wie unter 67.B. beschrieben ergab (E)-4,4'-Bis-(2,3,4,5,6-penta-O-sulfo-D-glucit-1-ylamino)-3,3'-dinitro-N,N'-stilben-4,4'-diyl- dibenzamid Decanatriumsalz als gelb-oranges Pulver, $[\alpha]_D^{20}$ +5,8° (*c* 0,5; Wasser), MS: *m/z* 1887,0 (rekonstruiertes M).

Beispiel 90

A. Eine Suspension von 1,69 g (Z)-Stilben-4,4'-dicarbonsäure in 40 ml Tetrahydrofuran, 40 ml Acetonitril und 1,43 ml N-Methylmorpholin wurde mit 2,22 g 2-Chlor-4,6-dimethoxy-1,3,5-triazin versetzt und 2 Stunden bei Raumtemperatur gerührt. Nach Zugabe von 2,3 g 1-Amino-1-desoxy-D-mannitol wurde 18 Stunden bei Raumtemperatur gerührt. Ausgefallene Kristalle wurden abfiltriert und mit Acetanhydrid in Pyridin acetyliert. Das Reaktionsgemisch wurde eingeengt und über Kieselgel chromatographiert. Das gereinigte Produkt wurde wie unter 1.A. beschrieben deacetyliert und ergab (Z)-Stilben-4,4'-dicarbonsäure bis-D-mannit-1-ylamid, $[\alpha]_D^{20}$ +3,50 (*c* 0,2; DMF), MS: *m/z* 595,5 ($[M+H]^+$).

B. Umsetzung von (Z)-Stilben-4,4'-dicarbonsäure bis-D-mannit-1-ylamid wie unter 2.B. beschrieben ergab (Z)-Stilben-4,4'-dicarbonsäure bis-(2,3,4,5,6-penta-O-sulfo-D-mannit-1-ylamid) Decanatriumsalz, $[\alpha]_D^{20}$ +19,5°(*c* 0,2; Wasser), MS: *m/z* 1614 (rekonstruiertes M).

Beispiel 91

A. Umsetzung von (Z)-Stilben-4,4'-dicarbonsäure mit 1-Amino-1-desoxy-D-galactitol wie unter 90.A. beschrieben ergab (Z)-Stilben-4,4'-dicarbonsäure bis-D-galactit-1-ylamid, MS: *m/z* 595,4 ($[M+H]^+$).

B. Umsetzung von (Z)-Stilben-4,4'-dicarbonsäure bis-D-galactit-1-ylamid wie unter 2.B. beschrieben ergab (Z)-Stilben-4,4'-dicarbonsäure bis-(2,3,4,5,6-penta-O-sulfo-D-galactit-1-ylamid) Decanatriumsalz, $[\alpha]_D^{20}$ -14,0°(*c* 0,2; Wasser), MS: *m/z* 1615 (rekonstruiertes M).

Beispiel 92

A. Umsetzung von (Z)-Stilben-4,4'-dicarbonsäure mit 1-Amino-1-desoxy-D-arabinitol wie unter 90.A. beschrieben ergab (Z)-Stilben-4,4'-dicarbonsäure bis-D-arabinit-1-ylamid, MS: *m/z* 557,4 ($[M+Na]^+$).

B. Umsetzung von (Z)-Stilben-4,4'-dicarbonsäure bis-D-arabinit-1-ylamid wie unter 2.B. beschrieben ergab (Z)-Stilben-4,4'-dicarbonsäure bis-(2,3,4,5,6-penta-O-sulfo-D-arabinit-1-ylamid) Octanatriumsalz, $[\alpha]_D^{20}$ +25,5°(*c* 0,2; Wasser), MS: *m/z* 1350,0 (rekonstruiertes M).

Beispiel 93

A. Umsetzung von (Z)-Stilben-4,4'-dicarbonsäure mit 1-Amino-1-desoxy-L-rhamnitol wie unter 90.A. beschrieben ergab (Z)-Stilben-4,4'-dicarbonsäure bis-L-rhamnit-1-ylamid, MS: *m/z* 563,7 ($[M+H]^+$).

B. Umsetzung von (Z)-Stilben-4,4'-dicarbonsäure bis-L-rhamnit-1-ylamid wie unter 2.B. beschrieben ergab (Z)-Stilben-4,4'-dicarbonsäure bis-(2,3,4,5,6-penta-O-sulfo-L-rhamnit-1-ylamid) Octanatriumsalz, $[\alpha]_D^{20}$ -21,0°(*c* 0,2;

Wasser), MS: *m/z* 1379,0 (rekonstruiertes M).

Beispiel 94

A. Eine Suspension von 2,0 g 1-Amino-1-desoxy-D-mannitol in 20 ml Dimethylformamid wurde mit Toluylen-2,6-diisocyanat versetzt und 6 Stunden bei Raumtemperatur gerührt. Durch Zugabe von Acetanhydrid und Pyridin wurde direkt acetyliert. Das Reaktionsgemisch wurde eingeengt und über Kieselgel chromatographiert. Reine Produktfraktionen wurden eingeengt, in Methanol/Dioxan gelöst und mit 2 %iger methanolischer Natriummethylatlösung versetzt. Nach 18 Stunden bei Raumtemperatur wurden entstandene Kristalle abgenutscht, mit Methanol gewaschen und getrocknet. Es resultierte 3,3'-Di-D-mannit-1-yl-1,1'-(toluen-2,6-diyl)-diharnstoff, MS: *m/z* 537,6 ($[M+H]^+$).

B. Umsetzung von 3,3'-Di-D-mannit-1-yl-1,1'-(toluen-2,6-diyl)-diharnstoff wie unter 2.B. beschrieben ergab 3,3'-Bis-(2,3,4,5,6-penta-O-sulfo-D-mannit-1-yl)-1,1'-(toluen-2,6-diyl)-diharnstoff Decanatriumsalz, $[\alpha]^{20}_{D}$ +16,0° (*c* 0,2; Wasser), MS: *m/z* 1556,0 (rekonstruiertes M).

Beispiel 95

A. Umsetzung von 1-Amino-1-desoxy-D-galactitol mit Toluylen-2,6-diisocyanat wie unter 94.A. beschrieben ergab 3,3'-Di-D-galactit-1-yl-1,1'-(toluen-2,6-diyl)-diharnstoff, MS: *m/z* 537,5 ($[M+H]^+$).

B. Umsetzung von 3,3'-Di-D-galactit-1-yl-1,1'-(toluen-2,6-diyl)-diharnstoff wie unter 2.B. beschrieben ergab 3,3'-Bis-(2,3,4,5,6-penta-O-sulfo-D-galactit-1-yl)-1,1'-(toluen-2,6-diyl)-diharnstoff Decanatriumsalz, $[\alpha]^{20}_{D}$ -3,0° (*c* 0,2; Wasser), MS: *m/z* 1556,0 (rekonstruiertes M).

Beispiel 96

A. Umsetzung von 1-Amino-1-desoxy-D-arabinitol mit Toluylen-2,6-diisocyanat wie unter 94.A. beschrieben ergab 3,3'-Di-D-arabinit-1-yl-1,1' (toluen-2,6-diyl)-diharnstoff, MS: *m/z* 499,5 ($[M+Na]^+$).

B. Umsetzung von 3,3'-Di-D-arabinit-1-yl-1,1'-(toluen-2,6-diyl)-diharnstoff wie unter 2.B. beschrieben ergab 3,3'-Bis-(2,3,4,5,6-penta-O-sulfo-D-arabinit-1-yl)-1,1'-(toluen-2,6-diyl)-diharnstoff Octanatriumsalz, $[\alpha]^{20}_{D}$ +14,0° (*c* 0,2; Wasser), MS: *m/z* 1293,0 (rekonstruiertes M).

Beispiel 97

A. Umsetzung von 1-Amino-1-desoxy-L-rhamnitol mit Toluylen-2,6-diisocyanat wie unter 94.A. beschrieben ergab 3,3'-Di-L-rhamnit-1-yl-1,1'-(toluen-2,6-diyl)-diharnstoff MS: *m/z* 527 ($[M+Na]^+$).

B. Umsetzung von 3,3'-Di-L-rhamnit-1-yl-1,1'-(toluen-2,6-diyl)-diharnstoff wie unter 2.B. beschrieben ergab 3,3'-Bis-(2,3,4,5,6-penta-O-sulfo-L-rhamnit-1-yl)-1,1'-(toluen-2,6-diyl)-diharnstoff Octanatriumsalz, $[\alpha]^{20}_{D}$ -15,0° (*c* 0,2; Wasser), MS: *m/z* 1321,0 (rekonstruiertes M).

Beispiel A

| Tablette: | | |
|---|---|---|
| 1 | Verbindung der Formel I | 500 mg |
| 2 | Laktose, wasserfrei | 150 mg |
| 3 | Mikrokristalline Cellulose | 150 mg |
| 4 | Polyvinylpyrrolidon | 40 mg |
| 5 | Talk | 50 mg |
| 6 | Magnesiumstearat | 10 mg |
| | Tablettengewicht | 900 mg |

Die Bestandteile 1-4 werden gesiebt und gemischt. Diese Mischung wird mit demineralisiertem Wasser granuliert

und das getrocknete Granulat mit den Bestandteilen 5 und 6 vermischt. Man verpresst zu Tabletten geeigneter Form.

Beispiel B

| Pellets: | | |
|---|---|---|
| 1 | Verbindung der Formel I | 500 mg |
| 2 | Mikrokristalline Cellulose | 200 mg |
| 3 | PRIMOJEL | 70 mg |
| 4 | Aromapulver | 10 mg |
| 5 | Talk | 20 mg |

Die gemischten und gesiebten Bestandteile 1-3 werden mit demineralisiertem Wasser ausreichend befeuchtet und mittels Extruder durch eine geeignete Lochscheibe gepresst. Das Extrudat wird auf einen Pelletierteller überführt, zu Kügelchen ausgerundet und anschliessend getrocknet. Man versetzt mit den gesiebten Bestandteilen 4 und 5 und füllt in Papiersachets (oder ähnliches) ab.

Beispiel C

Injektionslösung:

Zur Herstellung einer Injektionslösung werden 50 mg Verbindung der Formel I sowie 0,5 mg Tris-Puffer in Wasser für Injektionszwecke ad 1 ml gelöst und der pH-Wert auf 7,4 eingestellt. Die Lösung wird sterilfiltriert und nach Abfüllen in Ampullen autoklaviert.

**Patentansprüche**

1. Verbindungen der allgemeinen Formel

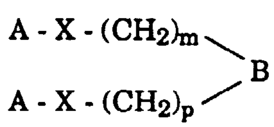

(I)

worin
A ein um die 1-Hydroxygruppe verminderter Rest eines Zuckeralkohols oder eines Derivats davon, oder der tris-(Hydroxymethyl)methylrest ist, wobei mindestens eine Hydroxygruppe des Restes A mit Schwefelsäure verestert ist;
X $-NR^1CO-$; -NHCONH-; -NHCSNH-; $-NHSO_2-$, $-NR^1-$ oder -O-;
m und p unabhängig voneinander 0 oder 1;
$R^1$ Wasserstoff, nieder-Alkyl oder Hydroxy-1-6C-alkyl ist; und
B ein System von konjugierten Mehrfachbindungen ist;
und Salze davon.

2. Verbindungen nach Anspruch 1, worin X $-NR^1CO-$; -NHCONH-; -NHCSNH-; $-NHSO_2-$ oder -O- und B ein System von konjugierten Doppelbindungen ist und Salze davon.

3. Verbindungen nach Anspruch 1 oder 2, worin A ein Rest $-(CHR^2)_nCH_2R^2$ und n eine ganze Zahl von 1-5; $R^2$ H, $-OSO_3H$ oder OZ ist; Z eine Schutzgruppe; und mindestens ein Rest $R^2$ $-OSO_3H$ ist.

4. Verbindungen nach einem der Ansprüche 1-3, worin das System von konjugierten Mehrfachbindungen B ein Polyen- oder Polyin-Kohienwasserstoffrest mit 2-8 konjugierten Mehrfachbindungen ist.

5. Verbindungen nach einem der Ansprüche 1-4, worin das System von konjugierten Mehrfachbindungen B ein System von konjugierten Doppelbindungen ist.

6. Verbindungen nach Anspruch 5, worin das System von konjugierten Doppelbindungen ein aromatisches Ringsystem ist.

7. Verbindungen nach Anspruch 6, worin das aromatische Ringsystem Phenylen; durch 1-6C-Alkyl substituierten Phenylen; Naphthylen, Fluorenylen; oder ein Rest einer der Formeln (a) bis (s) ist

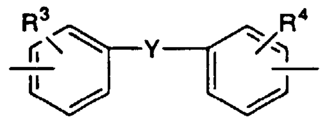
(a)

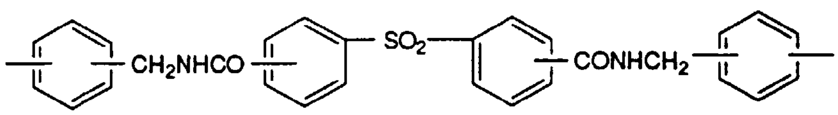
(b)

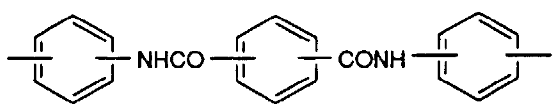
(c)

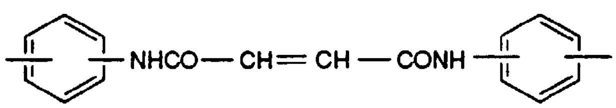
(d)

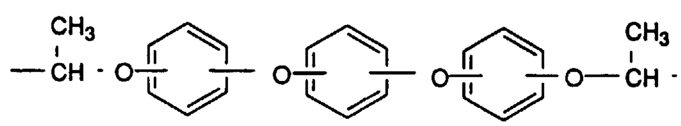
(e)

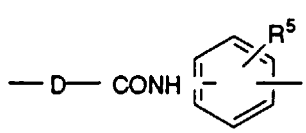
(f)

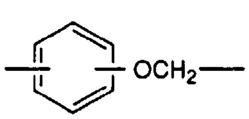
(g)

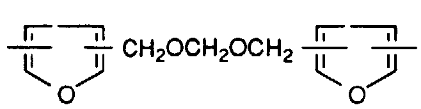
(h)

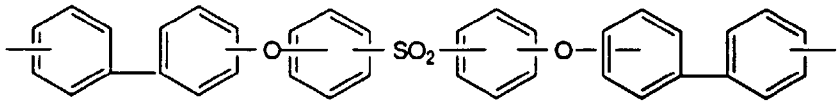

(i)

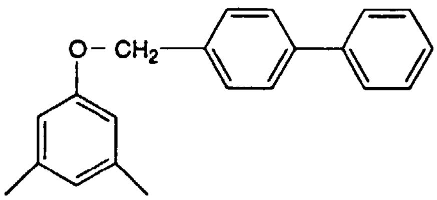

(j)

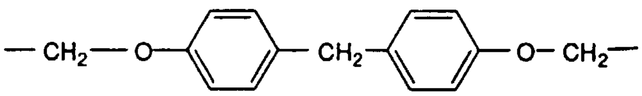

(k)

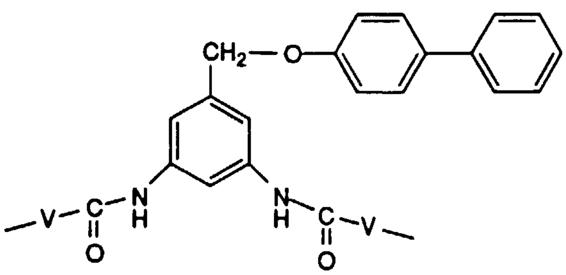

(l)

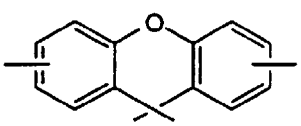

(m)

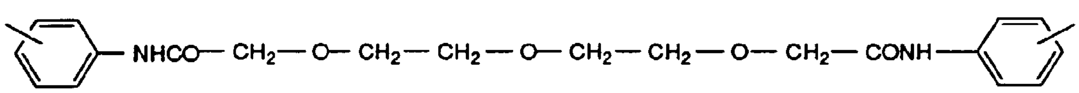

(n)

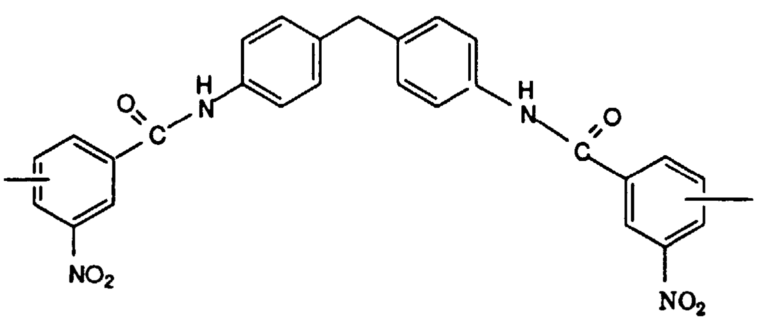

(o)

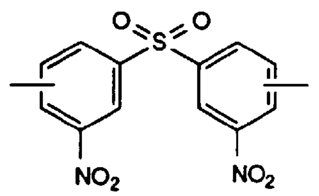

(p)

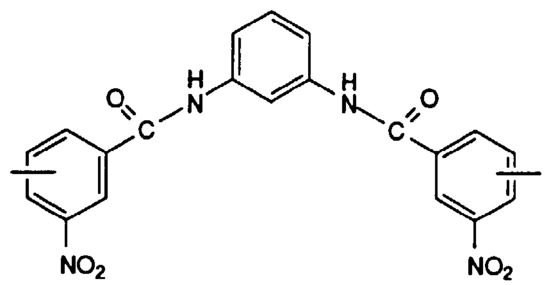

(q)

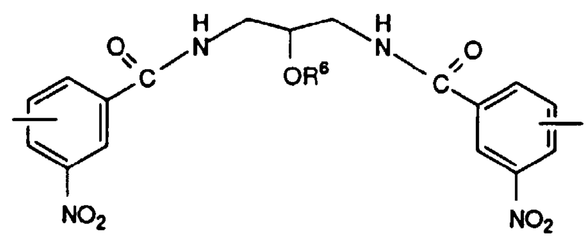

(r)

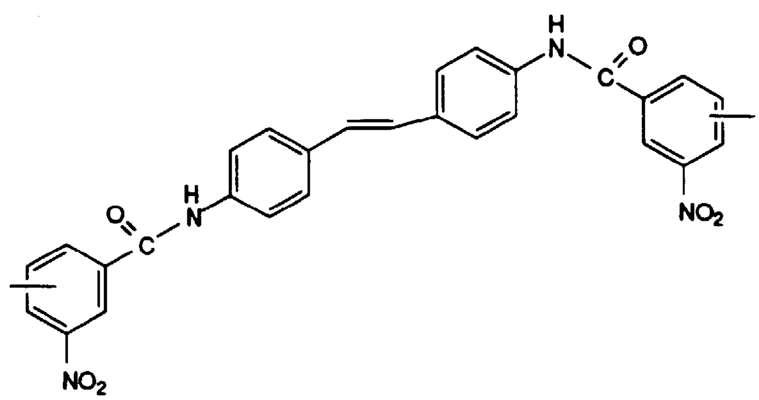

(s)

worin Y eine Kohlenstoff-Kohlenstoff-Bindung; -O-,-CO, $-CH_2-$, $-CH_2CH_2-$, -CH=CH-, -C≡C-, -CH(O)CH-,$-SO_2-$, $-C(CF_3)_2-$, $-CONR^1-$, -NHCONH-, -NHCOCONH-, Phenylen oder Phenylendioxy ist; $R^1$ Wasserstoff, 1-6C-Alkyl oder Hydroxy-1-6C-Alkyl; $R^3$ und $R^4$ Wasserstoff, 1-6C-Alkyl, 1-6C-Alkoxy, Halogen oder $-SO_3H$; $R^5$ 1-6C-Alkoxy-1-6C-alkoxy oder Phenoxy, oder durch Halogen substituiertes Phenoxy; $R^6$ Wasserstoff oder $SO_3H$ und D $-CH_2CH_2-$ oder -CH=CH- sind und worin V -CH=CH-, $-CH_2CH_2-$ oder eine Einfachbindung bedeutet.

**8.** (Z)-Butendisäure (Z)-[3-biphenyl-4-yloxymethyl-5-[3-(2,3,4,5,6-penta-O-sulfo-D-glucit-1-ylcarbamoyl)-acryloylamino]-phenylamid]-(2,3,4,5,6-penta-O-sulfo-D-glucit-1-yl)-amid Decanatriumsalz,

(E)-2-Chlorstilben-4,4'-dicarbonsäure-bis-(2,3,4,5,6-penta-O-sulfo-D-glucit-1-ylamid) Decanatriumsalz,

(Z)-Stilben-4,4'-dicarbonsäure-bis-(2,3,4,5,6-penta-O-sulfo-D-glucit-1-ylamid) Decanatriumsalz,

(2E,4E,6E,8E,10E,12E,14E)-2,6,11,15-Tetramethyl-hexadeca-2,4,6,8,10,12,14-heptaendicarbonsäure bis-(2,3,4,5,6-penta-O-sulfo-D-glucit-1-ylamid) Decanatriumsalz,

4,4'-Ethylen-dibenzoesäure-bis-(2,3,4,5,6-penta-O-sulfo-D-glucit-1-ylamid) Decanatriumsalz,

3,3'-Bis-(2,3,4,5,6-penta-O-sulfo-D-glucit-1-yl)-1,1'-(toluol-2,6-diyl)-diharnstoff Decanatriumsalz,
4,4'-Bis-(2,3,4,5,6-penta-O-sulfo-D-glucit-1-ylamino)-3,3'-dinitro-N,N'-(4,4'-methylen-diphenyl)-dibenzamid Decanatriumsalz,
N,N'-Bis-(2,3,4,5,6-penta-O-sulfo-D-glucit-1-yl)-2,2'-dinitro-4,4'-sulfonyldianilin Decanatriumsalz,
(Z)-Stilben-4,4'-dicarbonsäure bis-(2,3,4,5,6-penta-O-sulfo-L-rhamnit-1-ylamid) Octanatriumsalz,
Naphtalin-1,5-disulfonsäure-bis-(2,3,4,5,6-penta-O-sulfo-D-glucit-1-ylamid) Decanatriumsalz,
N,N'-Bis-[4-(2,3,4,5-tetra-O-sulfo-D-arabinit-1-yloxy)-phenyl]-isophthalamid Octanatriumsalz,
1,3-Bis-[4-(2,3,4,5,6-penta-O-sulfo-D-galactit-6-yloxy)-phenyl]-harnstoff Decanatriumsalz,
N,N'-Bis-[4-[3-(2,3,4,5,6-penta-O-sulfo-D-glucit-1-yl)-ureido]-phenyl]-isophthalamid Decanatriumsalz,
9-Oxo-9H-fluoren-2,7-dicarbonsäure bis-[methyl-(2,3,4,5,6-penta-O-sulfo-D-glucit-1-yl)-amid] Decanatriumsalz,
4,4'-Methylen-dibenzoesäure-bis-(2,3,4,5,6-penta-O-sulfo-D-glucit-1-ylamid) Decanatriumsalz,
Naphthalin-2,6-dicarbonsäure-bis-(2,3,4,5,6-penta-O-sulfo-D-glucit-1-ylamid) Decanatriumsalz,
N,N'-Bis-(2,3,4,5,6-penta-O-sulfo-D-glucit-1-yl)-isophthalamid Decanatriumsalz,
N,N'-Bis-[4-[3-(2,3,4,5,6-penta-O-sulfo-D-glucit-1-yl)-ureido]-phenyl]-fumaramid Decanatriumsalz,
4,4'-Sulfonyl-dibenzoesäure-bis-(2,3,4,5,6-penta-O-sulfo-D-glucit-1-ylamid) Decanatriumsalz.

**9.** Verbindungen nach einem der Ansprüche 1-8 zur Anwendung als therapeutische Wirkstoffe, insbesondere zur Behandlung und/oder Prophylaxe arteriosklerotischer Gefässwandveränderungen.

**10.** Arzneimittel, insbesondere zur Behandlung und/oder Prophylaxe arteriosklerotischer Gefässwandveränderungen enthaltend eine Verbindung nach einem der Ansprüche 1-8 und einen therapeutisch inerten Träger.

**11.** Verfahren zur Herstellung von Verbindungen nach einem der Amsprüche 1-8, dadurch gekennzeichnet, dass man eine entsprechende, nicht sulfatierte Verbindung mit einem Sulfatierungsmittel umsetzt.

**12.** Verwendung von Verbindungen nach einem der Ansprüche 1-8 zur Behandlung und/oder Prophylaxe arteriosklerotischer Gefässwandveränderungen bzw. zur Herstellung entsprechender Arzneimittel.

## Claims

**1.** Compounds of the general formula

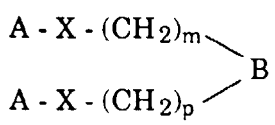

(I)

wherein

A is the residue of a sugar alcohol or of a derivative thereof devoid of the 1-hydroxy group, or the tris-(hydroxymethyl)methyl residue, with at least one hydroxy group of the residue A being esterified with sulphuric acid;

X is $-NR^1CO-$; -NHCONH-; -NHCSNH-; $-NHSO_2-$; $-NR^1-$ or -O-;

m and p are each independently 0 or 1;

$R^1$ is hydrogen, lower-alkyl or hydroxy-1-6C-alkyl; and

B is a system of conjugated multiple bonds;

and salts thereof.

**2.** Compounds according to claim 1, wherein X is $-NR^1CO-$; -NHCONH-; -NHCSNH-; $-NHSO_2-$ or -O- and B is a

system of conjugated double bonds, and salts thereof.

3. Compounds according to claim 1 or 2, wherein A is a residue $-(CHR^2)_nCH_2R^2$ and n is a whole number of 1-5; $R^2$ is H, $-OSO_3H$ or OZ; Z is a protecting group; and at least one residue $R^2$ is $-OSO_3H$.

4. Compounds according to any one of claims 1-3, wherein the system of conjugated multiple bonds B is a polyene or polyyne hydrocarbon residue having 2-8 conjugated multiple bonds.

5. Compounds according to any one of claims 1-4, wherein the system of conjugated multiple bonds B is a system of conjugated double bonds.

6. Compounds according to claim 5, wherein the system of conjugated double bonds is an aromatic ring system.

7. Compounds according to claim 6, wherein the aromatic ring system is phenylene; phenylene substituted by 1-6C-alkyl; naphthylene, fluorenylene; or a residue of one of formulae (a) to (s)

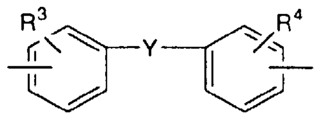

(a)

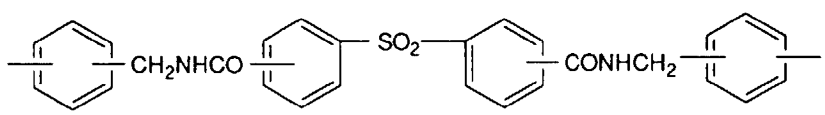

(b)

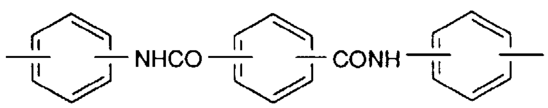

(c)

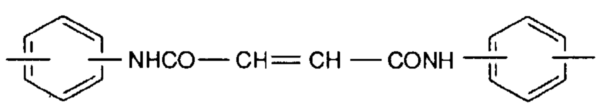

(d)

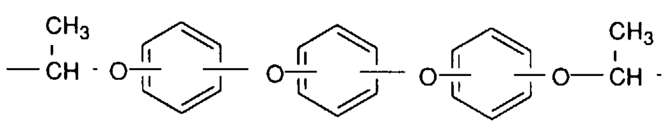

(e)

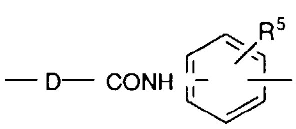

(f)

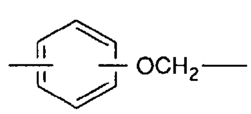

(g)

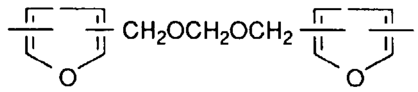

(h)

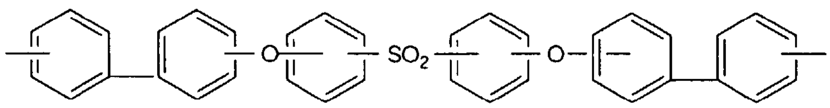

(i)

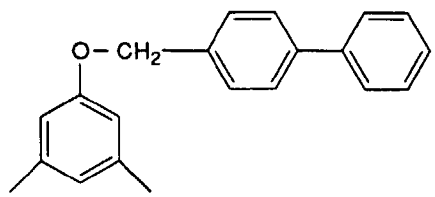

(j)

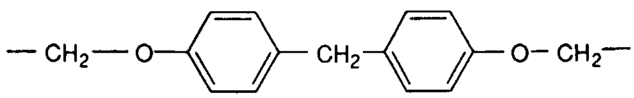

(k)

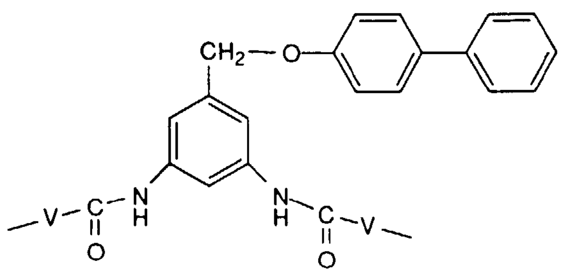

(l)

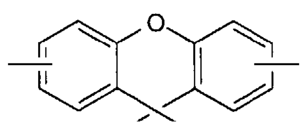

(m)

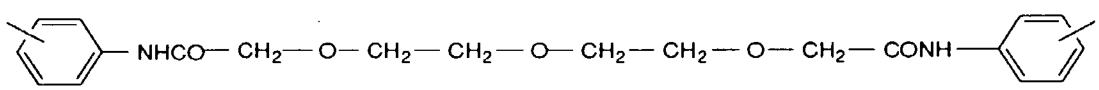

(n)

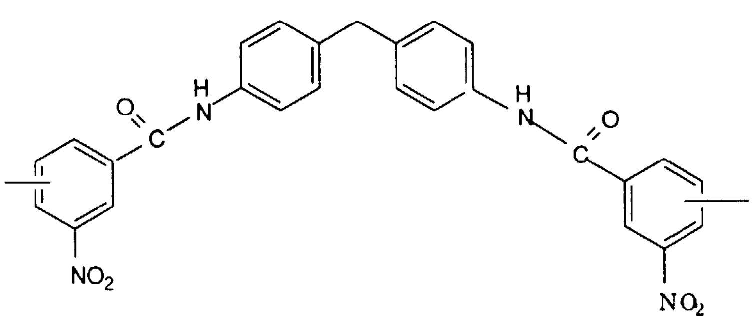

(o)

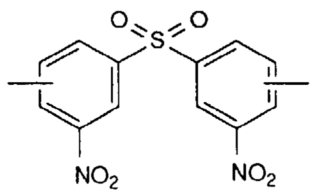

(p)

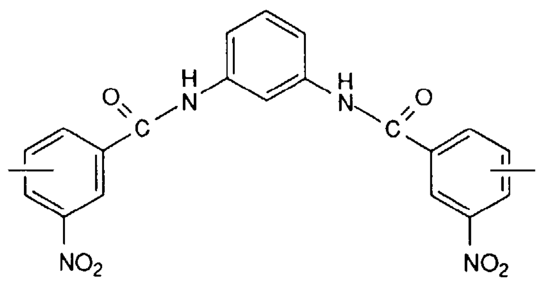

(q)

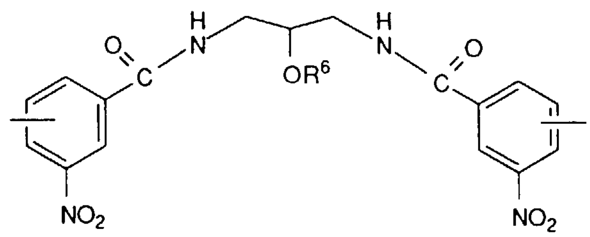

(r)

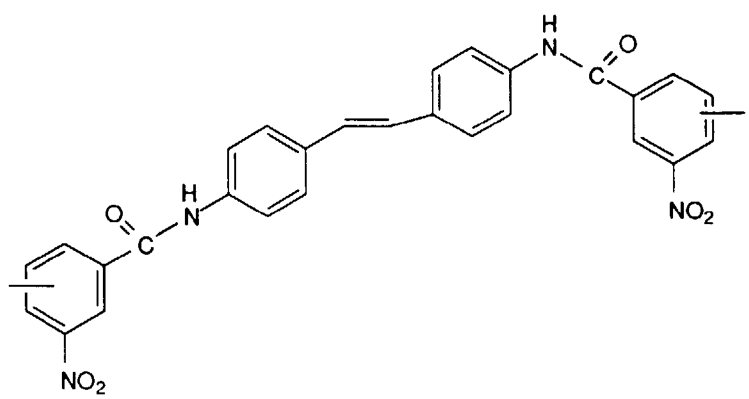

(s)

wherein Y is a carbon-carbon bond; -O-, -CO, $-CH_2-$, $-CH_2CH_2-$, -C≡C-, -CH(O)CH-, -CH=CH-, $-SO_2-$, $-C(CF_3)_2-$, $-CONR^1-$, -NHCONH-, -NHCOCONH-, phenylene or phenylenedioxy; $R^1$ is hydrogen, 1-6C-alkyl or hydroxy-1-6C-alkyl; $R^3$ and $R^4$ are hydrogen, 1-6C-alkyl, 1-6C-alkoxy, halogen or $-SO_3H$; $R^5$ is 1-6C-alkoxy-1-6C-alkoxy, phenoxy or phenoxy substituted by halogen; $R^6$ is hydrogen or $SO_3H$, D is $-CH_2CH_2-$ or -CH=CH- and V signifies -CH=CH-, $-CH_2CH_2-$ or a single bond.

**8.** (Z)-Butenedioic acid (Z)-[3-biphenyl-4-yloxymethyl-5-[3-(2,3,4,5,6-penta-O-sulpho-D-glucit-1-ylcarbamoyl) acryloylamino]-phenylamide]-(2,3,4,5,6-penta-O-sulpho-D-glucit-1-yl)-amide decasodium salt,
(E)-2-chlorostilbene-4,4'-dicarboxylic acid bis-(2,3,4,5,6-penta-O-sulpho-D-glucit-1-ylamide) decasodium salt,
(Z)-stilbene-4,4'-dicarboxylic acid bis-(2,3,4,5,6-penta-O-sulpho-D-glucit-1-ylamide) decasodium salt,
(2E,4E,6E,8E,10E,12E,14E)-2,6,11,15-tetramethyl-hexadeca-2,4,6,8,10,12,14-heptaenedicarboxylic acid bis-(2,3,4,5,6-penta-O-sulpho-D-glucit-1-ylamide) decasodium salt,
4,4'-ethylene-dibenzoic acid bis-(2,3,4,5,6-penta-O-sulpho-D-glucit-1-ylamide) decasodium salt,

3,3'-bis-(2,3,4,5,6-penta-O-sulpho-D-glucit-1-yl)-1,1'-(toluene-2,6-diyl)-diurea decasodium salt,
4,4'-bis-(2,3,4,5,6-penta-O-sulpho-D-glucit-1-ylamino)-3,3'-dinitro-N,N'-(4,4'-methylene-diphenyl)-dibenzamide decasodium salt,
N,N'-bis-(2,3,4,5,6-penta-O-sulpho-D-glucit-1-yl)-2,2'-dinitro-4,4'-sulphonyldianiline decasodium salt,
(Z)-stilbene-4,4'-dicarboxylic acid bis-(2,3,4,5,6-penta-O-sulpho-L-rhamnit-1-ylamide) octasodium salt,
naphthalene-1,5-disulphonic acid bis-(2,3,4,5,6-penta-O-sulpho-D-glucit-1-ylamide) decasodium salt,
N,N'-bis-[4-(2,3,4,5-tetra-O-sulpho-D-arabinit-1-yloxy)-phenyl]-isophthalamide octasodium salt,
1,3-bis-[4-(2,3,4,5,6-penta-O-sulpho-D-galactit-6-yloxy)-phenyl]-urea decasodium salt,
N,N'-bis-[4-[3-(2,3,4,5,6-penta-O-sulpho-D-glucit-1-yl)-ureido]-phenyl]-isophthalamide decasodium salt,
9-oxo-9H-fluorene-2,7-dicarboxylic acid bis-[methyl-(2,3,4,5,6-penta-O-sulpho-D-glucit-1-yl)-amide] decasodium salt,
4,4'-methylene-dibenzoic acid bis-(2,3,4,5,6-penta-O-sulpho-D-glucit-1-ylamide) decasodium salt,
naphthalene-2,6-dicarboxylic acid bis-(2,3,4,5,6-penta-O-sulpho-D-glucit-1-ylamide) decasodium salt,
N,N'-bis-(2,3,4,5,6-penta-O-sulpho-D-glucit-1-yl)-isophthalamide decasodium salt,
N,N'-bis-[4-[3-(2,3,4,5,6-penta-O-sulpho-D-glucit-1-yl)-ureido]-phenyl]-fumaramide decasodium salt,
4,4'-sulphonyl-dibenzoic acid bis-(2,3,4,5,6-penta-O-sulpho-D-glucit-1-ylamide) decasodium salt.

**9.** Compounds according to any one of claims 1-8 for use as therapeutically active substances, especially for the treatment and/or prophylaxis of arteriosclerotic changes in the vascular wall.

**10.** A medicament, especially for the treatment and/or prophylaxis of arteriosclerotic changes in the vascular wall, containing a compound according to any one of claims 1-8 and a therapeutically inert carrier.

**11.** A process for the manufacture of compounds according to any one of claims 1-8, which process comprises reacting a corresponding non-sulphated compound with a sulphating agent.

**12.** The use of compounds according to any one of claims 1-8 for the treatment and/or prophylaxis of arteriosclerotic changes in the vascular wall or for the production of corresponding medicaments.

## Revendications

**1.** Composés de formule générale

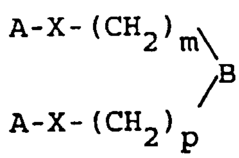

(I)

dans laquelle

A est un reste, diminué du groupe 1-hydroxy, d'un alcool glucidique ou d'un dérivé de celui-ci, ou le radical tris(hydroxyméthyl)méthyle, au moins un groupe hydroxy du radical A étant estérifié par l'acide sulfurique;
X est $-NR^1CO-$, -NHCONH-, -NHCSNH-, $-NHSO_2-$, $-NR^1-$ ou -O-;
m et p sont, indépendamment l'un de l'autre, 0 ou 1;
$R^1$ est un atome d'hydrogène ou un groupe alkyle inférieur ou hydroxyalkyle en $C_{1-6}$; et
B est un système de liaisons multiples conjuguées;

et leurs sels.

**2.** Composés selon la revendication 1, dans lesquels X est $-NR^1CO-$, -NHCONH-, -NHCSNH-, $-NHSO_2-$ ou -O-, et B est un système de doubles liaisons conjuguées, et leurs sels.

**3.** Composés selon la revendication 1 ou 2, dans lesquels A est un radical $-(CHR^2)_nCH_2R^2$ et n est un nombre entier allant de 1 à 5, $R^2$ est H, $-OSO_3H$ ou OZ, Z est un groupe protecteur, et au moins un radical $R^2$ est $-OSO_3H$.

4. Composés selon l'une des revendications 1 à 3, dans lesquels le système de liaisons multiples conjuguées B est un radical hydrocarboné polyène ou poly-yne comportant 2-8 liaisons multiples conjuguées.

5. Composés selon l'une des revendications 1 à 4, dans lesquels le système de liaisons multiples conjuguées B est un système de doubles liaisons conjuguées.

6. Composés selon la revendication 5, dans lesquels le système de doubles liaisons conjuguées est un système cyclique aromatique.

7. Composés selon la revendication 6, dans lesquels le système cyclique aromatique est le radical phénylène, un radical phénylène substitué par un groupe alkyle en $C_{1-6}$, le radical naphtylène, fluoroénylène, ou un radical correspondant à l'une des formules (a) à (s)

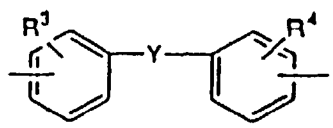
(a)

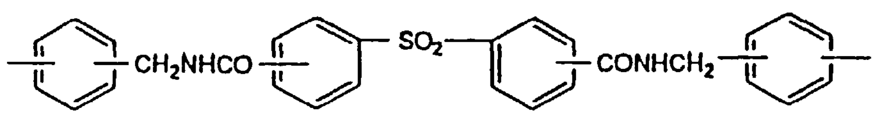
(b)

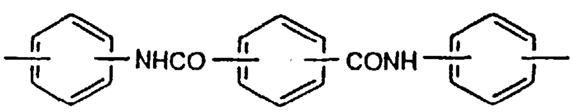
(c)

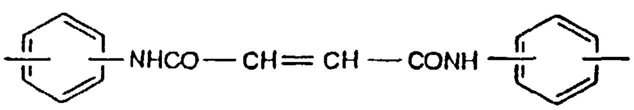
(d)

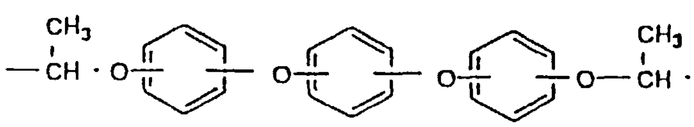
(e)

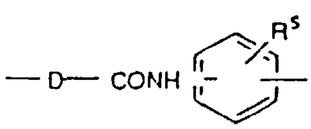
(f)

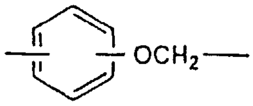

(g)

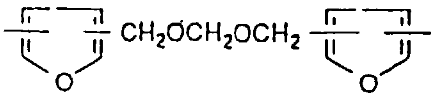

(h)

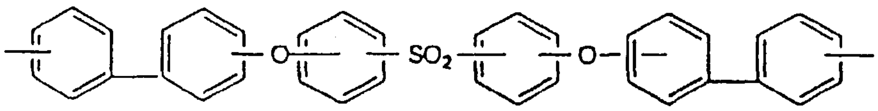

(i)

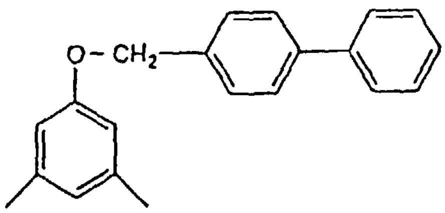

(j)

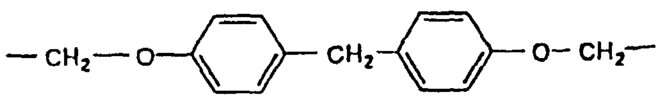

(k)

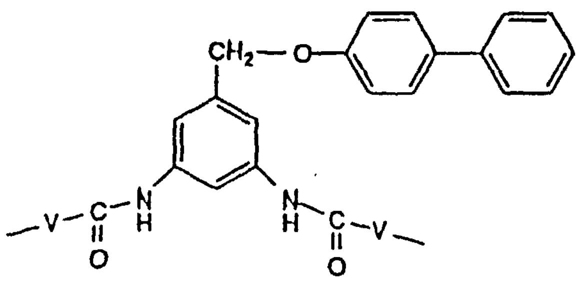

(l)

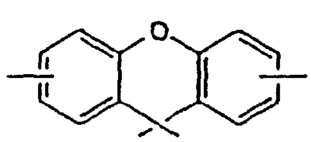

(m)

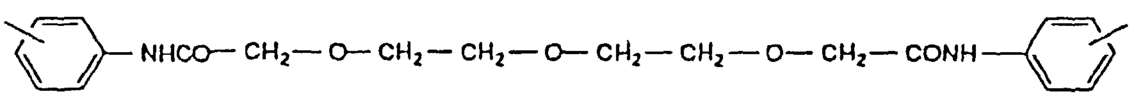

(n)

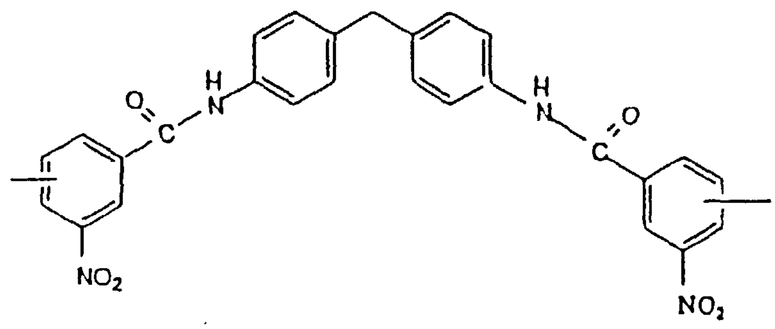

(o)

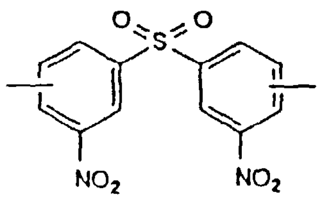

(p)

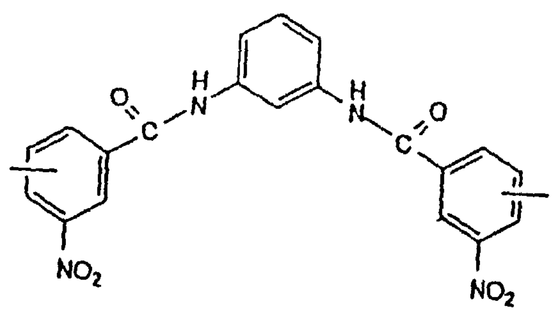

(q)

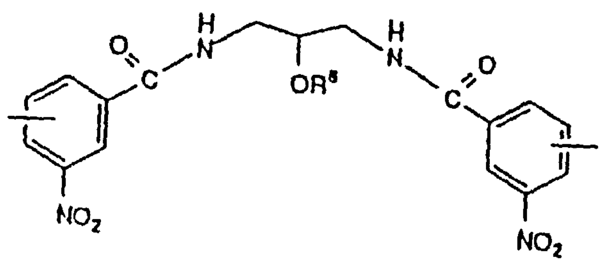

(r)

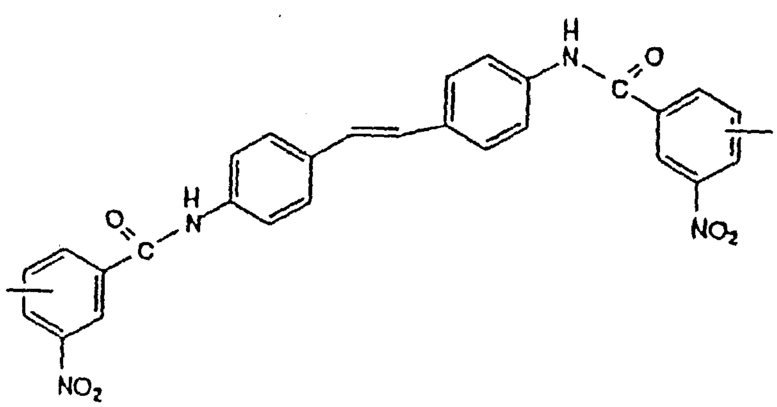

(s)

dans lesquelles Y est une liaison carbone-carbone, -O-, -CO-, $-CH_2-$, $-CH_2CH_2-$, -CH=CH-, -C≡C-, -CH(O)CH-, $-SO_2-$, $-C(CF_3)_2-$, $-CONR^1-$, -NHCONH-, -NHCOCONH-, le radical phénylène ou phénylènedioxy; $R^1$ représente

un atome d'hydrogène ou un groupe alkyle en $C_{1-6}$ ou hydroxyalkyle en $C_{1-6}$; $R^3$ et $R^4$ représentent un atome d'hydrogène ou d'halogène ou un groupe alkyle en $C_{1-6}$, alcoxy en $C_{1-6}$ ou $-SO_3H$; $R^5$ est un groupe alcoxy($C_{1-6}$)-alcoxy($C_{1-6}$) ou phénoxy, ou un radical phénoxy substitué par un atome d'halogène; $R^6$ est un atome d'hydrogène ou $SO_3H$, et D est $-CH_2CH_2-$ ou -CH=CH-, et dans lesquelles V représente -CH=CH-, $-CH_2CH_2-$ ou une liaison simple.

**8.** (Z)-[3-biphényl-4-yloxyméthyl-5-[3-(2,3,4,5,6-penta-O-sulfo-D-glucit-1-ylcarbamoyl)acryloylamino]phénylamide]-(2,3,4,5,6-penta-O-sulfo-D-glucit-1-yl)amide d'acide (Z)-butènedioïque (sel décasodique),
bis(2,3,4,5,6-penta-o-sulfo-D-glucit-1-ylamide) d'acide (E)-2-chlorostilbène-4,4'-dicarboxylique (sel décasodique),
bis(2,3,4,5,6-penta-O-sulfo-D-glucit-1-ylamide) d'acide (Z)-stilbène-4,4'-dicarboxylique (sel décasodique),
bis(2,3,4,5,6-penta-O-sulfo-D-glucit-1-ylamide) d'acide (2E,4E,6E,8E,10E,12E,14E)-2,6,11,15-tétraméthylhexadéca-2,4,6,8,10,12,14-heptaènedicarboxylique (sel décasodique),
bis(2,3,4,5,6-penta-O-sulfo-D-glucit-1-ylamide) d'acide 4,4'-éthylène-dibenzoique (sel décasodique),
3,3'-bis(2,3,4,5,6-penta-O-sulfo-D-glucit-1-yl)-1,1'-(toluène-2,6-diyl)diurée (sel décasodique),
4,4'-bis(2,3,4,5,6-penta-O-sulfo-D-glucit-1-ylamino)-3,3'-dinitro-N,N'-(4,4'-méthylènediphényl)dibenzamide (sel décasodique),
N,N'-bis(2,3,4,5,6-penta-O-sulfo-D-glucit-1-yl)-2,2'-dinitro-4,4'-sulfonyldianiline (sel décasodique),
bis(2,3,4,5,6-penta-O-sulfo-L-rhamnit-1-ylamide) d'acide (Z)-stilbène-4,4'-dicarboxylique (sel octasodique),
bis(2,3,4,5,6-penta-O-sulfo-D-glucit-1-ylamide) d'acide naphtalène-1,5-disulfonique (sel décasodique),
N,N'-bis[4-(2,3,4,5-tetra-O-sulfo-D-arabinit-1-yl-oxy)phényl]isophtalamide (sel octasodique),
1,3-bis[4-(2,3,4,5,6-penta-O-sulfo-D-galactit-6-yl-oxy)phényl]urée (sel décasodique),
N,N'-bis[4-[3-(2,3,4,5,6-penta-O-sulfo-D-glucit-1-yl)uréido]phényl]isophtalamide (sel décasodique),
bis[méthyl-(2,3,4,5,6-penta-O-sulfo-D-glucit-1-yl)-amide] d'acide 9-oxo-9H-fluorène-2,7-dicarboxylique (sel décasodique),
bis(2,3,4,5,6-penta-O-sulfo-D-glucit-1-ylamide) d'acide 4,4'-méthylènedibenzoïque (sel décasodique),
bis(2,3,4,5,6-penta-O-sulfo-D-glucit-1-ylamide) d'acide naphtalène-2,6-dicarboxylique (sel décasodique),
N,N'-bis(2,3,4,5,6-penta-O-sulfo-D-glucit-1-yl)isophtalamide (sel décasodique),
N,N'-bis[4-[3-(2,3,4,5,6-penta-O-sulfo-D-glucit-1-yl)-uréido]phényl]fumaramide (sel décasodique),
bis(2,3,4,5,6-penta-O-sulfo-D-glucit-1-ylamide) d'acide 4,4'-sulfonyldibenzoique (sel décasodique).

**9.** Composés selon l'une des revendications 1 à 8, pour utilisation en tant que substances actives thérapeutiques, en particulier pour le traitement et/ou la prophylaxie d'altérations artérioscléreuses de la paroi vasculaire.

**10.** Médicaments, destinés en particulier au traitement et/ou à la prophylaxie d'altérations artérioscléreuses de la paroi vasculaire, contenant un composé selon l'une des revendications 1 à 8 et un véhicule thérapeutiquement inerte.

**11.** Procédé pour la préparation des composés selon l'une des revendications 1 à 8, caractérisé en ce que l'on fait réagir un composé non sulfaté correspondant avec un agent de sulfatation.

**12.** Utilisation des composés selon l'une des revendications 1 à 8, pour le traitement et/ou la prophylaxie d'altérations artérioscléreuses de la paroi vasculaire, ou pour la fabrication de médicaments correspondants.